# EUROPEAN PATENT SPECIFICATION

(11) **EP 3 977 121 B1**
(45) Date of publication and mention of the grant of the patent: **19.03.2025**
(21) Application number: 20747238.2
(22) Date of filing: 03.06.2020
(51) Int. Cl.: G01N 33/50, C12N 9/10

(54) **A CHEMOENZYMATIC METHOD FOR THE DETECTION OF CELL-CELL PROXIMITY INTERACTION AND ISOLATION OF TUMOR-SPECIFIC ANTIGEN REACTIVE T CELLS FOR IMMUNE THERAPY**
CHEMOENZYMATISCHES VERFAHREN ZUM NACHWEIS VON ZELL-ZELLEN-NÄHRUNGWECHSELWIRKUNG UND ISOLIERUNG VON TUMORSPEZIFISCHEN ANTIGEN-REAKTIVEN T-ZELLEN ZUR IMMUNTHERAPIE
PROCÉDÉ CHIMIO-ENZYMATIQUE POUR LA DÉTECTION D'INTERACTION DE PROXIMITÉ CELLULE-CELLULE ET L'ISOLEMENT DE LYMPHOCYTES T RÉACTIFS À UN ANTIGÈNE SPÉCIFIQUE D'UNE TUMEUR POUR UNE THÉRAPIE IMMUNITAIRE

(30) Priority: 03.06.2019 US 201962856551 P; 16.03.2020 US 202062990383 P
(43) Date of publication of application: 06.04.2022
(73) Proprietor: The Scripps Research Institute, La Jolla, CA 92037 (US)
(72) Inventor: WU, Peng, La Jolla, California 92037 (US); LIU, Zilei, La Jolla, California 92037 (US); LI, Jie, La Jolla, California 92037 (US); TEIJARO, John, La Jolla, California 92037 (US)
(74) Representative: Hutter, Anton
(86) International application number: PCT/US2020/035940
(87) International publication number: WO 2020/247510

(56) References cited:
- WO-A1-2018/144769
- WO-A1-2019/080829
- CN-A- 109 797 194
- US-A1- 2018 346 899
- US-A1- 2019 002 515
- YUN GE ET AL: "Enzyme-Mediated Intercellular Proximity Labeling for Detecting Cell-Cell Interactions", JOURNAL OF THE AMERICAN CHEMICAL SOCIETY, vol. 141, no. 5, 24 January 2019 (2019-01-24), US, pages 1833 - 1837, XP055726676, ISSN: 0002-7863, DOI: 10.1021/jacs.8b10286
- ZILEI LIU ET AL: "Detecting Tumor Specific Antigen-Reactive T cells from Tumor Infiltrating Lymphocytes via Interaction Dependent Fucosyl-biotinylation", BIORXIV, 20 March 2020 (2020-03-20), XP055726704, Retrieved from the Internet <URL:https://www.biorxiv.org/content/10.1101/2020.03.18.996017v1.full.pdf> [retrieved on 20200902], DOI: 10.1101/2020.03.18.996017

## Description

### CROSS-REFERENCE TO RELATED APPLICATIONS

This application claims priority to U.S. Provisional Application No. 62/856,551, filed, June 03, 2019, and U.S. Provisional Application No. 62/990,383, filed March 16, 2020.

### STATEMENT REGARDING SEQUENCE LISTING

The Sequence Listing associated with this application is provided in text format in lieu of a paper copy. The name of the text file containing the Sequence Listing is TSRI_012_01WO_ST25.txt. The text file is 34 KB, created on June 1, 2020, and is being submitted electronically via EFS-Web.

### STATEMENT OF GOVERNMENT INTEREST

This invention was made with government support under grant number R01 AI143884 and R01 GM093282 awarded by the National Institute of Health. The government has certain rights in the invention.

### BACKGROUND

Cell-cell interactions are essential in numerous biological processes, including immune responses¹, embryonic development² and neuronal signaling³. A technique for monitoring the dynamics of cell-cell interactions is required for researchers to better understand these biological processes. To date, there are limited methods for monitoring cell-cell interaction in vitro and in vivo. For example, Parker et al. used intravital microscopy to detect the dynamics of immune cell interactions in vivo in 2008.⁴ Recently, Victoria reported a strategy involves sortagging (using sortase enzymes) to study immune partnerships in vitro and in vivo.⁵ However, these methods require either special equipment or complicated genetic modification of certain types of cells. A generalizable, robust and cost-effective method for detection of cell-cell interaction is required.

Molecules presented on the cell surface determine how cells interact with their partners and their environment. For this reason, methods for engineering the cell-surface landscape are instrumental for the study of cell-cell communications⁵⁻⁶ and the downstream signaling⁷. As the most practiced cell-engineering approach, genetic engineering is limited by technical complications and safety concerns, such as the viral transduction resistance of primary cells, heterogeneous expression levels, and the potential for endogenous gene disruption⁸⁻¹⁰. To address these issues, engineering cell surfaces from "outside" using chemical biology tools has emerged as a complementary and generally-applicable approach¹¹⁻¹². In situ glycan editing via glycosylation enzymes is a single-step approach to modify glycocalyx. The most notable example of its application is ex vivo fucosylation of mesenchymal stem cells and regulatory T cells using the GDP-fucose (GDP-Fuc) donor and recombinant human α(1,3)-fucosyltransferase VI¹³⁻¹⁴. Currently undergoing several clinical trials, this procedure improves adhesion, homing, and engraftment of adoptively transferred cells. Furthermore, US 2018/346899 A1 discloses a sortase-mediated intercellular labeling method allowing for tracking ligand-receptor interaction both in vitro and in vivo; and uses thereof for tracking molecule interactions both in vitro and in vivo, identifying modulators of ligand-receptor interaction, identifying potential binding partners of a protein of interest, identifying B cells expressing high affinity B cell receptors to antigens, and identifying the antigen to which a T cell of interest binds.

Inspired by these studies, we aim to develop a method to functionalize cell surface with glycan editing enzymes. This enzyme functionalized cell will serve as a "detector" to transfer probe (e.g., biotin, tags, fluorescent molecules) to adjacent cells in an interaction-dependent manner. To fulfill this aim, we need (1) a simple and efficient method to transfer enzyme to cell surface; (2) the enzyme should exhibit excellent catalytic ability (high reaction rate, high selectivity and good safety to host cells); (3) cell-cell interaction serves as a kinetic switch: when no interaction occurs, no enzyme-catalyzed labeling takes place; when an interaction does occur, the labeling takes place rapidly.

The present disclosure provides, *inter alia,* 1) a one-pot process to conjugate H. pylori α1,3fucosyltransferase (FT) or other enzymes to the cell surface; (2) a protocol using FT functionalized cells to monitor immune cell-cell interactions *in vitro, ex vivo* and *in vivo*; and (3) a technique using FT functionalized immature dendritic cells (iDCs) to identify and isolate antigen specific T cells and, in particular, tumor specific T cells from tumor infiltrating lymphocytes (TILs) for cancer immune therapy or other immunotherapy applications.

### SUMMARY OF THE EMBODIMENTS

The scope of the claimed invention is defined by the independent claims. Preferred examples are set out in the dependent claims. The present disclosure provides, *inter alia*, compositions and methods for monitoring cell to cell ("cell-cell") interactions *in vitro, ex vivo,* and *in vivo.* In some examples, the present disclosure provides for the use of these compositions and methods (i) to identify and enrich for tumor-specific antigen (TSA) reactive T cells from tumor infiltrating lymphocytes (TILs) or circulating T cells; (ii) to identify and enrich for T cells that recognize autoantigens in a particular autoimmune disease, and/or (iii) to identify and enrich for antigen specific regulatory T cells that have the potential to be exploited to treat autoimmune disease.

In some embodiments, the present disclosure provides a method for identifying tumor-specific antigen (TSA) reactive T cells from tumor infiltrating lymphocytes (TILs) or circulating T cells comprising: (a) contacting a population of T cells with a modified dendritic cell in the presence of a donor sugar nucleotide that is conjugated to a label, (i) wherein the modified dendritic cell has been engineered to comprise on its cell surface an active glycosyltransferase that is capable of catalyzing the glycosylation of a cell surface glycan on the T cell using the donor sugar nucleotide; and (i) wherein the modified dendritic cell has been primed with one or more tumor antigen; and (b) analyzing the cell surface of the population of T cells, after the contacting, to determine whether any label is present; wherein, the presence of the label on the cell surface of the T cell indicates that the T cell is a TSA reactive T cell with specificity for at least one of the one or more of the tumor antigens. In some embodiments, the analyzing comprises enriching for cells comprising the label via Fluorescence-activated cell sorting (FACS). In some embodiments, the analyzing further comprises determining whether the cells comprising the label further comprise other markers indicative of TSA reactivity. In some embodiments, the method further comprises enriching for cells comprising the other markers indicative of TSA reactivity via FACS. In some embodiments, the other markers include one or more of PD-1 expression; CD134 expression, and CD137 expression. In some embodiments, the method comprises enriching for the tagged TSA reactive T cells based upon expression profiles for other markers, e.g. CXCR5 expression, and/or TIM3 expression. In some embodiments, the method comprises enriching for cells comprising both the label and PD-1 expression. In some embodiments, the method further comprises enriching for cells comprising CD8+ and/or CD4+ expression. In some examples, any cells comprising the label and exhibiting both CD8+ expression and PD-1+ expression are TSA reactive cytotoxic T cells. In some examples, any cells comprising the label and exhibiting both CD4+ expression and PD-1+ expression are TSA reactive helper T cells. In some examples, the magnitude of label present on the T cell is indicative of the binding affinity of a T cell receptor expressed on the surface of the T cell for the TSA. In some examples, the magnitude of label present on the T cell is indicative of the enriched T cell's ability to kill other cells expressing the TSA and/or of the enriched T cell's ability to become activated in the presence of the TSA. In some examples, the method further comprises sequencing a cell having label on its cell surface by single cell T cell receptor (TCR) sequencing to identify a TSA-specific TCR expressed by the cell. In some examples, the method further comprises expanding the enriched cells for patient specific immune cell therapy. In some examples, the modified dendritic cell has been primed with tumor lysate containing the tumor antigen. In some examples, the tumor lysate is from a tumor that produces neoantigens. In some examples, the TSA is from a tumor selected from a melanoma tumor; a breast cancer tumor; and a tumor selected from the group consisting of Pilocytic astrocytoma; AML; ALL; Thyroid; Kidney chromophobe; CLL; Medulloblastoma; Neuroblastoma; Glioma low grade; Glioblastoma; Prostate; Ovary; Myeloma; Pancreas; Kidney papillary; Lymphoma B-cell; Kidney clear cell; Head and neck; Liver; Cervix; Uterus; Bladder; Colorectum; Lung small cell; Esophagus; Stomach; Lung adeno; and Lung squamous. In some embodiments, the glycosyltransferase is a fucosyltransferase. In some embodiments, the glycosyltransferase is H. pylori α1,3fucosyltransferase. In some embodiments, the donor sugar nucleotide is GDP-fucose. In some embodiments, the glycosyltransferase is a sialyltransferase. In some embodiments, the donor sugar nucleotide is CMP-Sialic acid. In some embodiments, the label is selected from a small molecule, a polynucleotide, a polypeptide, an antibody, a chemical or biological marker and/or probe. In some embodiments, the chemical or biological moiety is biotin, a biotin probe, a fluorescent molecule, a probe comprising a fluorescent molecule, a dye, a probe comprising a dye, a dye-labeled single strand DNA, a FLAG tag, or a Strep tag. In some embodiments, the dye is FAM or a Cyanine dye. In some embodiments, the fluorescent molecule is Cy2, Cy3, Cy3B, Cy3.5, Cy5, Cy5.5, or Cy7. In some embodiments, the dye is an Alexa Fluor dye or a Janelia Fluor dye. In some examples, the cell surface glycan is selected from Gal, LacNAc, and sialyl LacNAc. In some examples, the glycosyltransferase is not native to the second bait cell. In some examples, the glycosyltransferase is conjugated to the cell surface of the second cell. In some examples, the glycosyltransferase is covalently bound to the cell surface of the second cell. In some examples, the glycosyltransferase is covalently bound to a second cell surface glycan present on the surface of the second cell. In some examples, the glycosyltransferase and the second cell surface glycan are covalently bound via a glycosylation reaction. In some examples, the glycosyltransferase is attached to the second donor sugar nucleotide via a linker moiety. In some examples, the glycosyltransferase is recombinantly expressed on the cell surface of the second bait cell. In some examples, the expression of the glycosyltransferase is driven by a conditionally activated promoter. In some examples, the conditionally activated promoter is activated in the presence of an exogenous compound. In some examples, the exogenous compound is a small molecule or polypeptide. In some examples, the method takes less than two weeks to complete. In some examples, the method does not comprise identifying TSA candidates prior to enriching for the TSA-reactive T cells. In some examples, the method comprises excluding bystander T cells from the enriched T cells.

In some examples, the present disclosure provides a method for tagging a first prey cell with a label comprising: contacting the first prey cell with a second bait cell in the presence of a donor sugar nucleotide that is conjugated to the label; wherein (i) the first prey cell comprises a first cell surface glycan; and wherein the second bait cell comprises on its cell surface an active glycosyltransferase that is capable of catalyzing the glycosylation of the first cell surface glycan using the donor sugar nucleotide; and (ii) wherein, upon contacting the first prey cell, the second bait cell catalyzes the glycosylation of the first cell surface glycan using the donor sugar nucleotide, thereby attaching the label to the first prey cell. In some examples, the present disclosure provides a method for detecting cell to cell interactions comprising: (a) contacting a first prey cell with a second bait cell in the presence of a donor sugar nucleotide that is conjugated to a label; wherein the first prey cell comprises a first cell surface glycan; and wherein the second bait cell comprises on its cell surface an active glycosyltransferase that is capable of catalyzing the glycosylation of the first cell surface glycan using the donor sugar nucleotide; and (b) analyzing the first prey cell, after the contacting, to determine whether the label is present on the cell surface of the first prey cell; wherein, the presence of the label on the cell surface of the first prey cell indicates that the first prey cell and the second bait have undergone a cell to cell interaction. In some embodiments, the glycosyltransferase is a fucosyltransferase. In some embodiments, the glycosyltransferase is H. pylori α1,3fucosyltransferase. In some embodiments, the donor sugar nucleotide is a GDP- fucose. In some embodiments, the glycosyltransferase is a sialyltransferase. In some embodiments, the donor sugar in CMP-Sialic acid. In some examples, the label is selected from a small molecule, a polynucleotide, a polypeptide, and an antibody. In some examples, the label is a chemical or biological marker and/or probe. In some examples, the chemical or biological moiety is a biotin probe, a fluorescent molecule, a probe comprising a fluorescent molecule, a dye, a probe comprising a dye, a dye-labeled single stranded DNA, a FLAG tag, or a Strep tag. In some embodiments, the dye is FAM or a cyanine dye. In some embodiments, the label is Cy2, Cy3, Cy3B, Cy3.5, Cy5, Cy5.5, or Cy7, an Alexa Fluor dye, or a Janelia Fluor dye. In some examples, the cell surface glycan is selected from Gal, LacNAc, and sialyl LacNAc. In some examples, the glycosyltransferase is not native to the second bait cell. In some examples, the glycosyltransferase is conjugated to the cell surface of the second cell. In some examples, the glycosyltransferase is covalently bound to the cell surface of the second cell. In some examples, the glycosyltransferase is covalently bound to a second cell surface glycan present on the surface of the second cell. In some examples, the glycosyltransferase and the second cell surface glycan are covalently bound via a glycosylation reaction In some examples, the glycosyltransferase is attached to the second donor sugar nucleotide via a linker moiety. In some examples, the glycosyltransferase is recombinantly expressed on the cell surface of the second bait cell. In some examples, the expression of the glycosyltransferase is driven by a conditionally activated promoter. In some examples, the conditionally activated promoter is activated in the presence of an exogenous compound. In some examples, the exogenous compound is a small molecule. In some examples, the first prey cell is a T cell. In some examples, the first prey cell is a CD4+ or a CD8+ T cell. In some examples, the first bait cell is an immature dendritic cell. In some examples, the first bait cell is a B cell.

In some examples, the present disclosure provides a TCR-engineered T cell comprising a TSA-specific TCR identified by a method disclosed herein, or an antigen binding portion of the TCR. In some examples, the present disclosure provides a method for treating cancer comprising administering to a patient in need thereof such a TCR-engineered T cell.

In some examples, the present disclosure provides a method for identifying a tumor-specific antigen (TSA)-specific T cell receptors (TCRs) comprising identifying a TSA reactive T cell by a method disclosed herein, and sequencing the TSA-specific T cell by single cell T cell receptor (TCR) sequencing to identify the TSA-specific TCR expressed by the cell.

In some examples, the present disclosure provides a method for treating cancer comprising, administering to a patient in need thereof a TSA reactive T cell identified by, or enriched for by, a method disclosed herein.

In some examples, the present disclosure provides a composition comprising GDP-Fuc-FT and one or more immature dendritic cell (iDC).

In some examples, the present disclosure provides a composition comprising a CMP-NeuAc-sialyltransferase and one or more immature dendritic cell (iDC).

In some examples, the present disclosure provides a composition comprising an immature dendritic cell engineered to have a fucosyltransferase or sialyltransferase conjugated to its cell surface. In some examples, the dendritic cell has been primed with an antigen. In some examples, the dendritic cell has been primed with tumor antigen. In some examples, the fucosyltransferase or sialyltransferase is conjugated to the cell surface of the immature dendritic cell by a method comprising contacting an immature dendritic cell with such a composition.

In some examples, the present disclosure provides a method for expanding (TSA) reactive T cells comprising performing a contact-induced interaction-dependent labeling method disclosed herein; FACS sorting the labeled cells to enrich for cells coexpressing the label and one or more PD-1, CD134 or CD137 markers; and expanding the cells by culturing them at low concentration in rapid expansion protocol (REP) conditions in the presence of irradiated allogeneic feeder cells, IL-2, and OKT3 antibody.

In some examples, the present disclosure provides a method for the construction of TCR-engineered T cells (TCR-T) comprising identifying a TSA reactive T cell by performing a contact-induced interaction-dependent labeling method disclosed herein; isolating the TSA reactive T cell; and contacting the TSA reactive T cell in vitro with one or more cytokine to promote the expansion of the TSA reactive T cells; wherein, optionally, the expansion is induced by utilizing rapid expansion protocol (REP) conditions; wherein sorted T cells are cultured in 96-well plates at 3 cells/well in the presence of irradiated allogeneic feeder cells, 3,000 IU/ml IL-2, and anti-CD3ε (OKT3). In some examples, the isolating is by FACS.

In some embodiments, the present disclosure provides a method for identifying antigen-reactive T cells from tissue infiltrating lymphocytes (TiILs) or circulating T cells comprising: (a) contacting a population of T cells obtained from TiILs or circulating T cells with a modified dendritic cell in the presence of a donor sugar nucleotide that is conjugated to a label, (i) wherein the modified dendritic cell has been engineered to comprise on its cell surface an active glycosyltransferase that is capable of catalyzing the glycosylation of a cell surface glycan on the T cell using the donor sugar nucleotide; and (ii) wherein the modified dendritic cell has been primed with one or more antigen; and (b) analyzing the cell surface of the population of T cells, after the contacting, to determine whether any label is present; wherein, the presence of the label on the cell surface of a T cell in the population of T cells indicates that the T cell is an antigen-reactive T cell with specificity for at least one of the one or more of the antigens used to prime the dendritic cell. In some embodiments, the antigen is from a pathogen. In some embodiments, the antigen is a viral antigen or a bacterial antigen.

In some examples, the present disclosure provides a method for determining the relative binding affinity for an antigen of a first and one or more second TCRs respectively expressed on a first and one or more second T cell comprising (i) performing a contact-induced interaction-dependent labeling method disclosed herein; (ii) quantifying the level of label transferred to the first and each second T cell; ranking the affinity of the cells by the level of label transferred; wherein the T cell with the most label is the T cell with the highest affinity for the antigen, the T cell with the least label is the T cell with the lowest affinity for the antigen, and any T cells with intermediate levels of label has corresponding intermediate levels of affinity for the antigen. In some examples, the label is biotin. In some examples, the relative quantity of label on each of the T cells corresponds with the efficacy of the T cell for killing cells expressing the antigen. In some examples, the relative quantity of label on each of the T cells corresponds with the efficacy of the T cell for producing IFNγ in response to exposure to the antigen.

In some examples, the present disclosure provides a method for identifying and enriching auto-reactive T cells present in a population of tissue infiltrating lymphocytes (TiILs) or circulating T cells, the method comprising providing a dendritic cell; incubating the dendritic cell with one or more autoantigens or a source of one or more autoantigens in order to prime the dendritic cell with one or more autoantigen; conjugating the dendritic cell on its cell surface with a suitable enzyme for catalyzing an interaction-dependent labeling reaction on a prey cell; and contacting the bait cell with a population of TiILs wherein the population comprises at least one auto-reactive T cell; wherein the contacting occurs in the presence of a tagged substrate for the enzyme. In some examples, the present disclosure provides a method for identifying and enriching auto-reactive T cells present in a population of tissue infiltrating lymphocytes (TiILs) or circulating T cells, the method comprising providing a dendritic cell conjugated on its cell surface with a suitable enzyme for catalyzing an interaction-dependent labeling reaction on a prey cell; incubating the dendritic cell with one or more autoantigens or a source of one or more autoantigens in order to prime the dendritic cell with one or more autoantigen; contacting the bait cell with a population of TiILs wherein the population comprises at least one auto-reactive T cell; and wherein the contacting occurs in the presence of a tagged substrate for the enzyme. In some examples, the source of one or more autoantigens is a cell lysate prepared from a diseased tissue biopsy from a subject having an autoimmune disease. In some examples, the T cells comprised in a population of cells are obtained from a diseased tissue biopsy from the subject having an autoimmune disease or from autologous PBMCs harvested from the patient. In some examples, the enzyme is selected from a fucosyltransferase and a sialyltransferase. In some examples, the enzyme is an α1,3fucosyltransferase. In some examples, the enzyme is H. pylori α1,3fucosyltransferase.

In some embodiments, the present disclosure provides a method for identifying antigen-reactive T cells present in a population of tissue infiltrating lymphocytes (TiILs) or circulating T cells, the method comprising (a) providing a dendritic cell; (b) incubating the dendritic cell with one or more antigens or a source of one or more antigens in order to prime the dendritic cell with one or more of the antigens; (c) conjugating the dendritic cell on its cell surface with a suitable enzyme for catalyzing an interaction-dependent labeling reaction on a prey cell; wherein the conjugating occurs before or after incubating step (b); (d) contacting the dendritic cell after conjugating step (C) with a population of TiILs or circulating T cells, wherein the population comprises at least one antigen-reactive T cell and wherein the contacting occurs in the presence of a substrate for the enzyme, said substrate comprising a detectable tag; (e) detecting any cells comprising the tag; thereby identifying the antigen-reactive T cells. In some embodiments, the method comprises enriching for the cells comprising the tag. In some embodiments, the method comprises isolating single cells comprising the tag. In some embodiments, the method comprises expanding cells comprising the tag. In some examples, the enzyme is a sortase and the substrate is a peptide comprising a sortase recognition sequence and a tag. In some examples, the enzyme is a sortase selected from sortase A: (5M) and mgSrtA. In some examples, the enzyme is sortase A and the substrate is a peptide comprising a sortase recognition sequence and a tag. In some examples, the sortase recognition sequence is selected from LPXTX (SEQ ID NO: 4), wherein each occurrence of X represents independently any amino acid residue; LPKTG (SEQ ID NO: 8); LPATG (SEQ ID NO: 9); LPNTG (SEQ ID NO: 10); LPETG (SEQ ID NO: 5); LPXAG (SEQ ID NO: 11), wherein X represents any amino acid; LPNAG (SEQ ID NO: 12; LPXTA (SEQ ID NO: 13), wherein X represents any amino acid; LPNTA (SEQ ID NO: 14), LGXTG (SEQ ID NO: 15), wherein X represents any amino acid; LGATG (SEQ ID NO: 16), IPXTG (SEQ ID NO: 17), wherein X represents any amino acid, IPNTG (SEQ ID NO: 18) and IPETG (SEQ ID NO: 19). In some examples, the enzyme is a promiscuous biotin ligase and the substrate is biotin. In some examples, the promiscuous biotin ligase is selected from TurboID, mini Turbo, BioID, and BioID2. In some embodiments, the enzyme is a glycosyltransferase and the substrate is a labeled donor sugar nucleotide. In some embodiments, the enzyme is a fucosyltransferase and the substrate is tagged GDP-fucose. In some embodiments, the fucosyltransferase is H. pylori α1,3fucosyltransferase. In some embodiments, the enzyme is a sialyltransferase and the substrate is tagged CMP-NeuAc. In some embodiments, the method further comprises determining whether the cells comprising the tag further comprise other markers indicative of TSA reactivity. In some embodiments, the method further comprises enriching for cells comprising the other markers, if present, that are indicative of TSA reactivity via FACS. In some embodiments, the other markers include one or more of PD-1 expression; CD134 expression, and CD137 expression. In some embodiments, the method comprises enriching for cells comprising both the tag and PD-1 expression. In some examples, the method further comprises enriching for cells comprising CD8+ and/or CD4+ expression. In some examples, the tag is selected from biotin, a fluorescent molecule, a dye, a FAM dye, a cyanine dye, Cy2, Cy3, Cy3B, Cy3.5, Cy5, Cy5.5, Cy7, an Alexa Fluor dye, and a Janelia Fluor dye.

In some examples, the present disclosure provides an engineered T cell receptor (TCR), or an antigen-binding fragment thereof, comprising a Vα and a Vβ derived from a wild type T cell receptor, wherein the Vα and Vβ each comprise a complementarity determining region 1 (CDR-1), a complementarity determining region 2 (CDR-2), and a complementarity determining region 3 (CDR-3), wherein the Vα CDR-3 comprises an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

In some examples, the present disclosure provides a T cell expressing the TCR or antigen binding fragment thereof, disclosed in the preceding paragraph. In some examples, the present disclosure provides a pharmaceutical composition comprising the T cell expressing the TCR or antigen binding fragment thereof, disclosed in the preceding paragraph. In some examples, the pharmaceutical composition further comprises one or more carrier, salt, vehicle, and/or excipient.

In some examples, the present disclosure provides a single chain T cell receptor (TCR) comprising a TSA-specific TCR comprising a Vα and a Vβ derived from a wild type T cell receptor, wherein the Vα and Vβ each comprise a complementarity determining region 1 (CDR-1), a complementarity determining region 2 (CDR-2), and a complementarity determining region 3 (CDR-3), wherein the Vα CDR-3 comprises an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

In some examples, the present disclosure provides a pharmaceutical composition comprising the single chain TCR disclosed in the preceding paragraph. In some examples, the pharmaceutical composition further comprises one or more carrier, salt, vehicle, and/or excipient.

In some examples, the present disclosure provides an engineered T cell receptor (TCR), or an antigen-binding fragment thereof, comprising a Vα and a Vβ derived from a wild type T cell receptor, wherein the Vα and Vβ each comprise a complementarity determining region 1 (CDR-1), a complementarity determining region 2 (CDR-2), and a complementarity determining region 3 (CDR-3), wherein the Vα CDR-3 comprises an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

In some examples, the present disclosure provides a bispecific TCR comprising a TSA-specific TCR comprising a Vα and a Vβ derived from a wild type T cell receptor, wherein the Vα and Vβ each comprise a complementarity determining region 1 (CDR-1), a complementarity determining region 2 (CDR-2), and a complementarity determining region 3 (CDR-3), wherein the Vα CDR-3 comprises an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

In some examples, the present disclosure provides a pharmaceutical composition comprising the bispecific disclosed in the preceding paragraph. In some examples, the pharmaceutical composition further comprises one or more carrier, salt, vehicle, and/or excipient.

In some examples, the present disclosure provides a composition comprising one or more tumor-specific antigen (TSA) reactive T cells comprising a label attached to its surface indirectly via a donor sugar nucleotide. In some embodiments, the donor sugar nucleotide is attached to the TSA reactive T cell via glycosylation. In some embodiments, the glycosylation was mediated by a glycosyltransferase. In some embodiments, the glycosyltransferase is a fucosyltransferase. In some embodiments, the glycosyltransferase is an α1,3fucosyltransferase. In some embodiments, the glycosyltransferase is H. pylori α1,3fucosyltransferase. In some embodiments, the the donor sugar nucleotide is GDP-fucose. In some embodiments, the glycosyltransferase is a sialyltransferase. In some embodiments, the donor sugar nucleotide is CMP-Sialic acid. In some embodiments, the label is selected from a small molecule, a polynucleotide, a polypeptide, an antibody, a chemical or biological marker and/or probe. In some embodiments, the chemical or biological moiety is biotin, a biotin probe, a fluorescent molecule, a probe comprising a fluorescent molecule, a dye, a probe comprising a dye, a dye-labeled single strand DNA, a FLAG tag, or a Strep tag. In some embodiments, the the dye is FAM or a Cyanine dye. In some embodiments, the fluorescent molecule is Cy2, Cy3, Cy3B, Cy3.5, Cy5, Cy5.5, or Cy7. In some embodiments, the the dye is an Alexa Fluor dye or a Janelia Fluor dye. In some embodiments, the label is attached to to a cell surface glycan on the TSA reactive T cell. In some embodiments, the cell surface glycan is selected from Gal, LacNAc, and sialyl LacNAc. In some embodiments, the present disclosure provides a composition comprising a population of TSA reactive T cells expanded *in vitro* from a composition of any such compositions comprising one or more tumor-specific antigen (TSA) reactive T cells comprising a label, as discussed above or herein. In some embodiments, the TSA reactive T cells were expanded *in vitro* by culturing the T cells in the presence of one or more cytokine. In some embodiments, the TSA reactive T cells are expanded *in vitro* by culturing the T cells in the presence of one or more cytokine. In some examples, the TSA reactive T cells were expanded in via a method comprising
a. contacting a population of T cells with a modified dendritic cell in the presence of a donor sugar nucleotide that is conjugated to a label,
   i. wherein the modified dendritic cell has been engineered to comprise on its cell surface an active glycosyltransferase that is capable of catalyzing the glycosylation of a cell surface glycan on the T cell using the donor sugar nucleotide; and
   ii. wherein the modified dendritic cell has been primed with one or more tumor antigen; and
b. analyzing by FACS the cell surface of the population of T cells, after the contacting, to determine whether any label is present;
c. sorting the labeled cells to enrich for cells coexpressing the label and one or more PD-1, CD134, CD137, CXCR5, or TIM3 markers; and
d. expanding the cells by culturing them at low concentration in rapid expansion protocol (REP) conditions in the presence of irradiated allogeneic feeder cells, IL-2, and OKT3 antibody.

In some examples, the TSA reactive T cells are expanded via a method comprising
e. contacting a population of T cells with a modified dendritic cell in the presence of a donor sugar nucleotide that is conjugated to a label,
   i. wherein the modified dendritic cell has been engineered to comprise on its cell surface an active glycosyltransferase that is capable of catalyzing the glycosylation of a cell surface glycan on the T cell using the donor sugar nucleotide; and
   ii. wherein the modified dendritic cell has been primed with one or more tumor antigen; and
f. analyzing the cell surface of the population of T cells, after the contacting, to determine whether any label is present;
wherein, the presence of the label on the cell surface of the T cell indicates that the T cell is a TSA reactive T cell with specificity for at least one of the one or more of the tumor antigens; isolating one or more of the TSA reactive T cells; and contacting the TSA reactive T cell *in vitro* with one or more cytokine to promote the expansion of the TSA reactive T cells; wherein, optionally, the expansion is induced by utilizing rapid expansion protocol (REP) conditions; wherein sorted T cells are cultured in 96-well plates at 3 cells/well in the presence of irradiated allogeneic feeder cells, 3,000 IU/ml IL-2, and anti-CD3ε (OKT3). In some examples, the isolating is by FACS. In some examples,the TSA reactive T cells or T cell compositions are for use in a patient specific immune cell therapy. In some examples,the TSA reactive T cells are for use in treating a cancer. In some examples, the cancer is selected from a melanoma; a breast cancer; and a Pilocytic astrocytoma; AML; ALL; Thyroid cancer; Kidney chromophobe; CLL; Medulloblastoma; Neuroblastoma; Glioma low grade; Glioblastoma; Prostate cancer; Ovariran cancer; Myeloma; Pancreaic cancer; Kidney papillary cancer; a Lymphoma, a B-cell cancer; a Kidney clear cell cancer; a Head and neck cancer; Liver cancer; Cervical cancer; Uterine caner; Bladder cancer; Colorectal cancer; aLung small cell cancer; Esophageal cancer; Stomach cancer; Lung adenocarcinoma; and Lung squamous cell cancer.

### BRIEF DESCRIPTION OF THE DRAWINGS

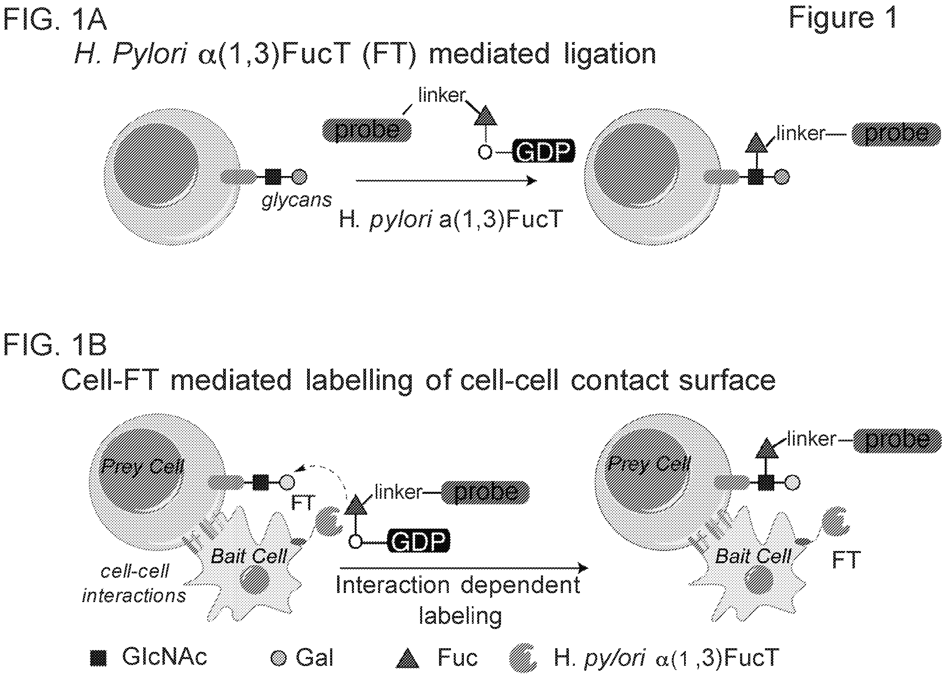

**Figure 1** shows application of glycosyltransferase-mediated cell-surface engineering as exemplified by fucosyltransferase (FT)-mediated glycan editing FIG. 1A shows cell surface glycan engineering using H. pylori α1,3fucosyltransferase. FIG. 1B shows strategy of cell-cell proximity labelling by FT functionalized cell A.

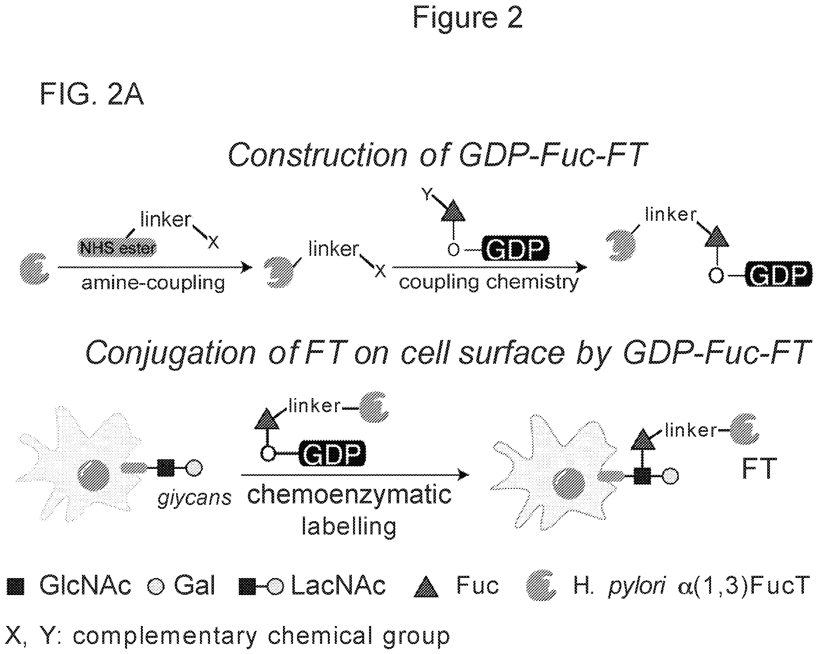

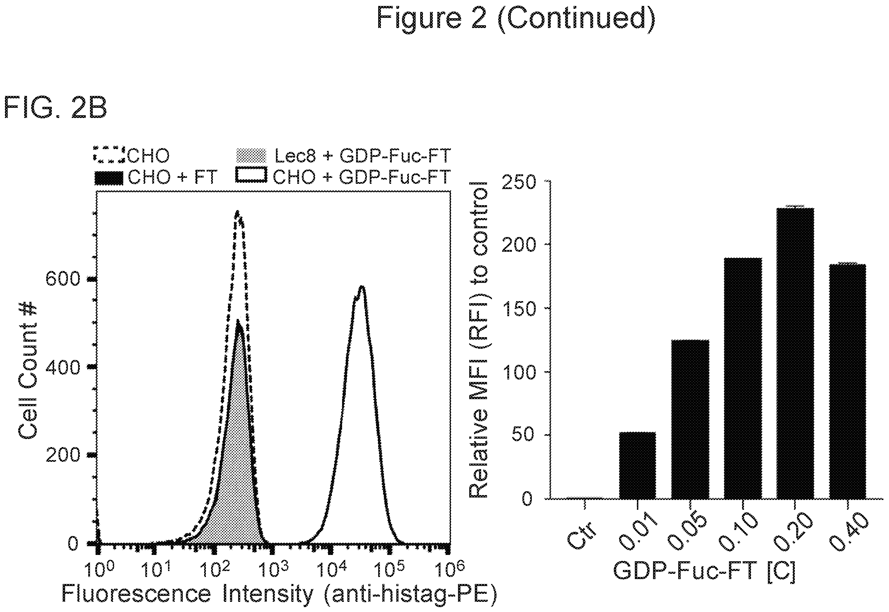

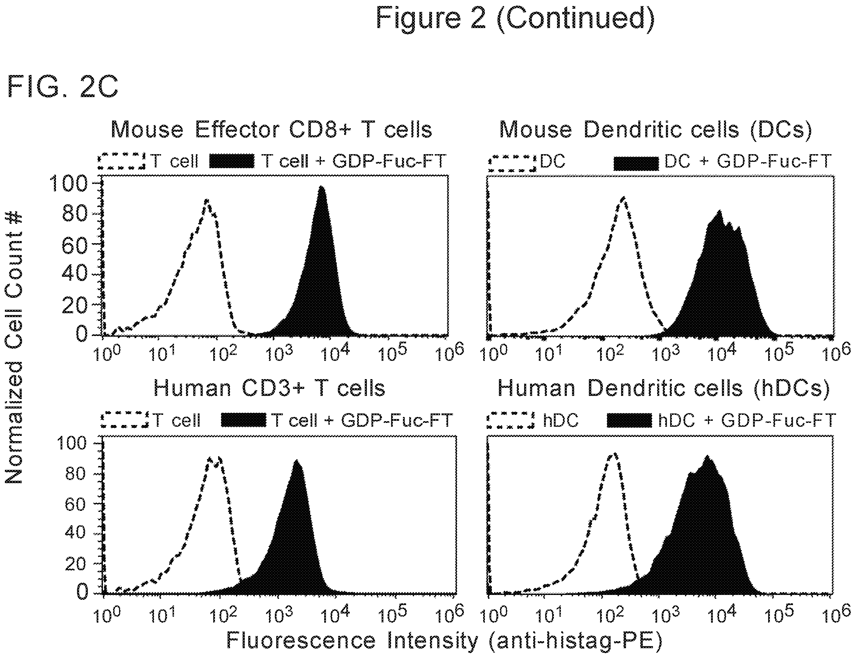

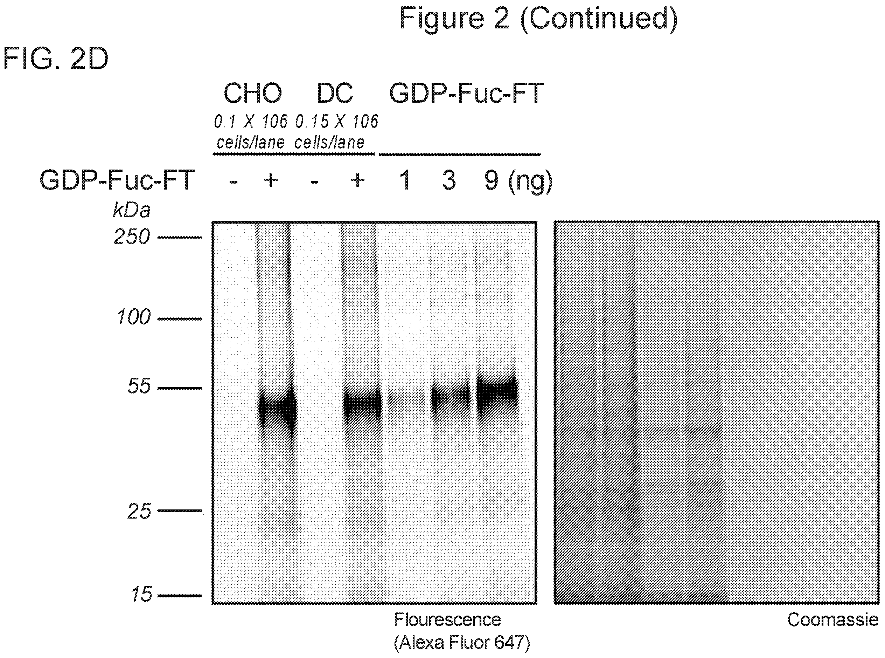

**Figure 2** shows conjugation of FT on cell surface. FIG. 2A shows a scheme for the conjugation of FT on cell surface of a cell via fucosylation. X and Y represent any two moieties that may be chemically conjugated together via coupling chemistry, e.g., via a covalent interaction (such as, *e.g.*, via click chemistry). FIG. 2B, left panel, shows flow cytometry confirmation that FT is present on the cell surface of CHO cells treated with GDP-Fuc-FT (0.01 mg/mL) for 30 min at room temperature. Cells were probed using an anti-His-PE antibody (FT has a His-tag). Controls include untreated CHO cells; CHO cells treated with free FT; and Lec8 cells (which do not express membrane LacNAc) treated with GDP-Fuc-FT, all of which result in only background signals. FIG. 2B, right panel, shows quantification of FT present on the CHO cell surface after treating CHO cells with various concentrations of GDP-Fuc-FT. 0.20 mg/mL was determined as the saturated concentration. FIG. 2C shows FT can be robustly conjugated on various cells including activated CD8+ T cells and dendritic cells (DCs). FIG. 2D, left panel, shows fluorescent SDS-PAGE gel analysis of FT labelled cells. GDP-Fuc-FT molecules modified with Alexa Fluor 647 were used in the experiments for quantifying the number of FT molecules conjugated to one cell surface. FIG. 2D, right panel, shows Coomassie blue staining of the gel from the left panel.

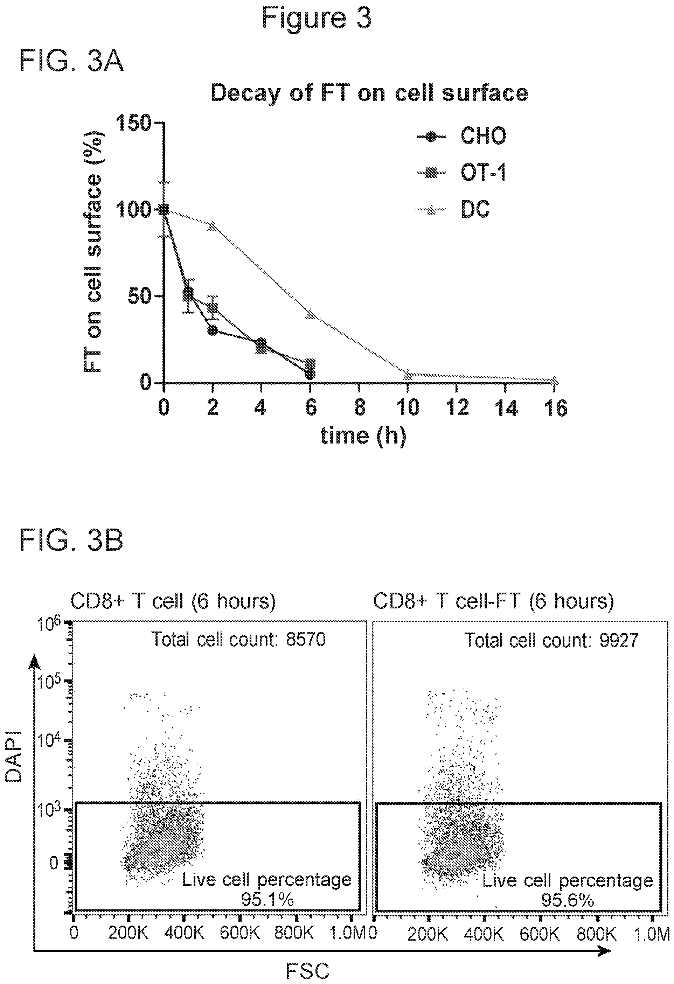

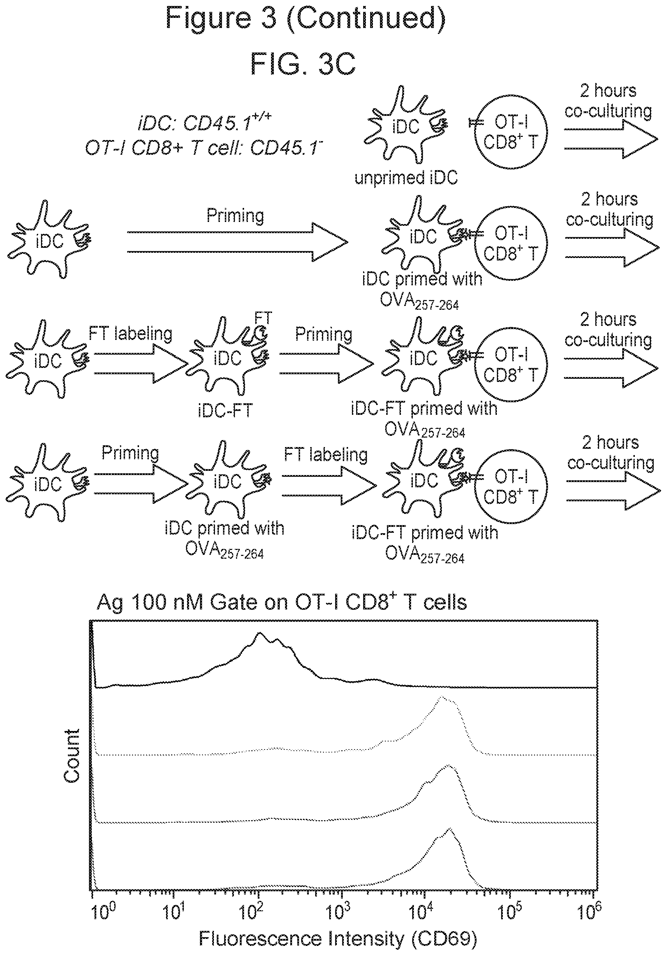

**Figure 3****.** FIG. 3A and 3B show the decay of FT on cell surface and that FT modification does not affect cell proliferation/viability. FIG. 3A shows the decay of FT on cell surface at various time points. FIG. 3B shows, by flow cytometry analysis, that the viability of modified CD8+ T-FT cells (right panel) is the same as the viability of unmodified CD8+ T cells (left panel). Activated CD8+ T cells and CD8+ T-FT conjugate were incubated for 6 hours, stained with DAPI, and analyzed by flow cytometry. Y axis: DAPI signal; X axis: Forward-scattered light (FSC) signal. FIG. 3C shows comparison of the antigen presentation abilities of immature DCs (iDCs) and FT-modified immature DCs (iDC-FT). cell mixtures were stained with anti-mCD45.1-FICT, anti-mCD8a-PB and anti-mCD69-PE for flow cytometry analysis, showing the FT functionalization of iDCs did not affect the iDC-mediated upregulation of CD69 in CD8+ T cells. Representative flow cytometry figures from three replicates are shown.

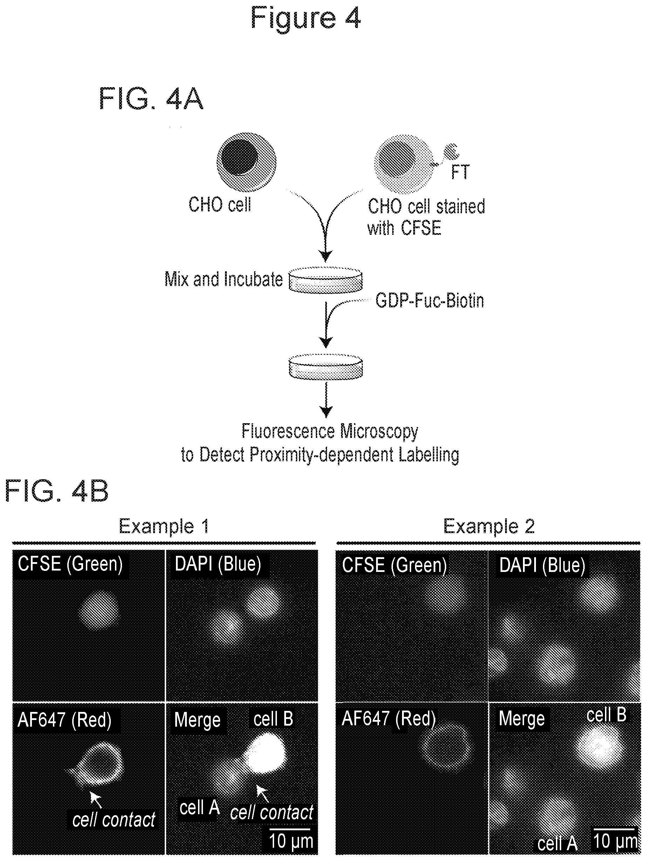

**Figure 4** shows FT functionalized CHO cells are capable of labelling the contact cells FIG. 4A shows an illustration of the experimental method. FIG. 4B: microscopy imaging clearly shows that (1) all Cell B (green, upper right cell in each of Examples 1 and 2) were robustly labelled on the membrane (red); (2) Cell A (lower left cell in each of Examples 1 and 2) not in contact with Cell B were not labelled (Example 2), but the Cell A contacting with Cell B was selectively labelled at the cell-cell contacting regions (Example 1). In contrast, the treatment of free FT resulted in the unselective labelling of all cells (data not shown).

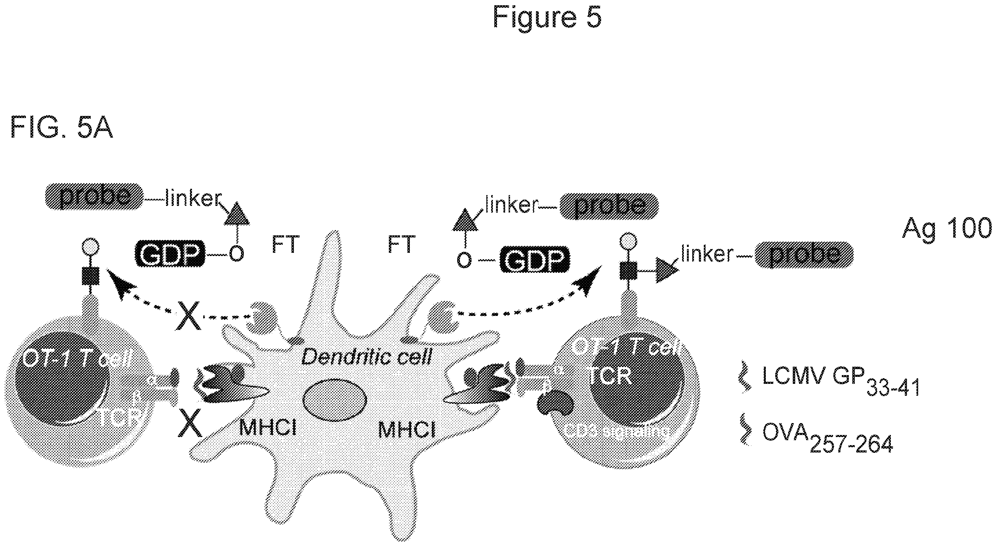

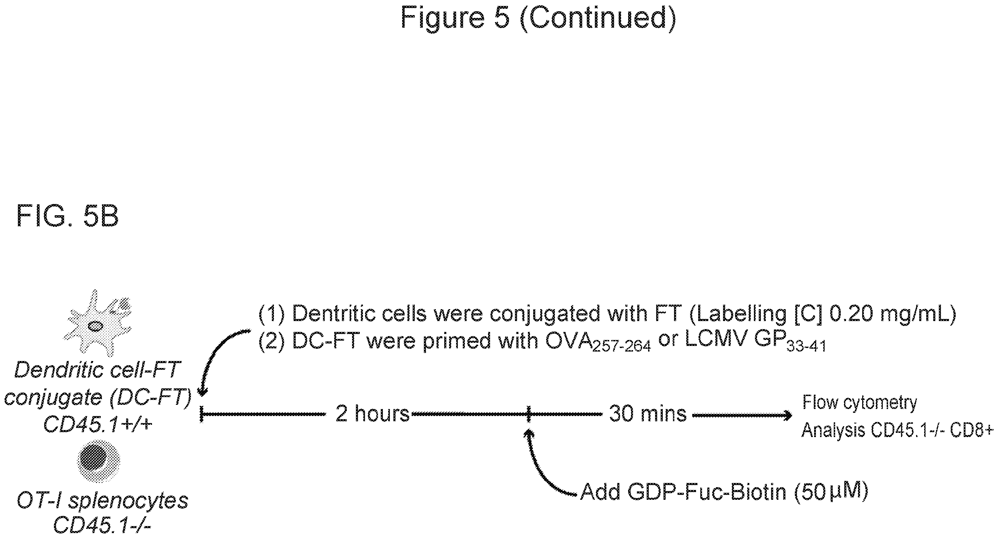

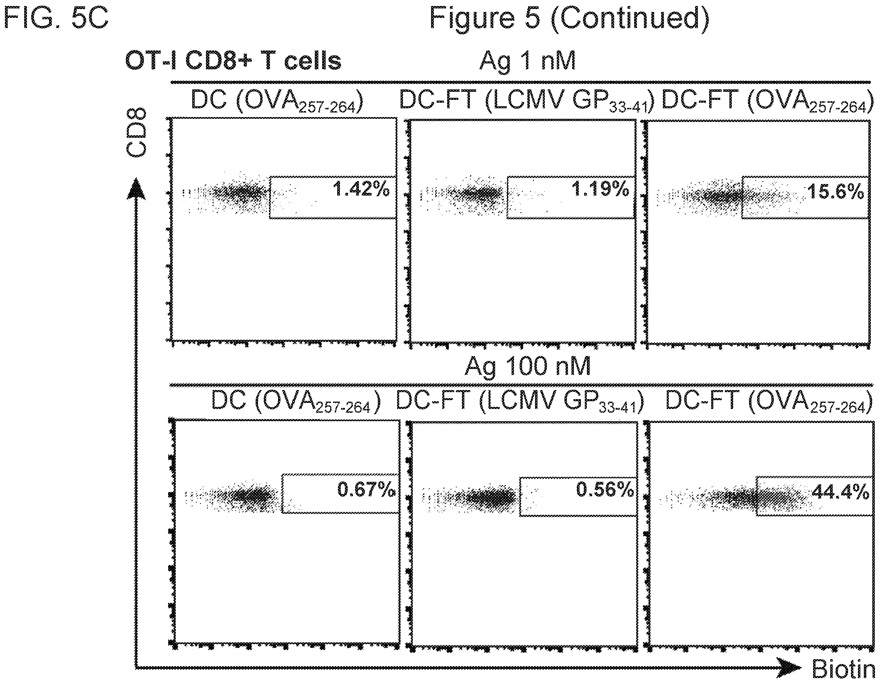

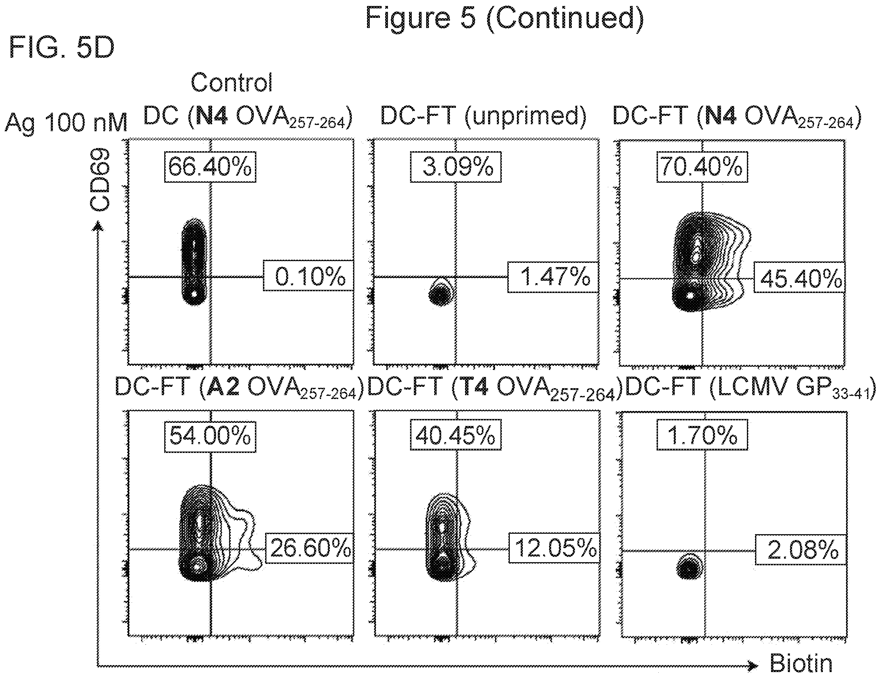

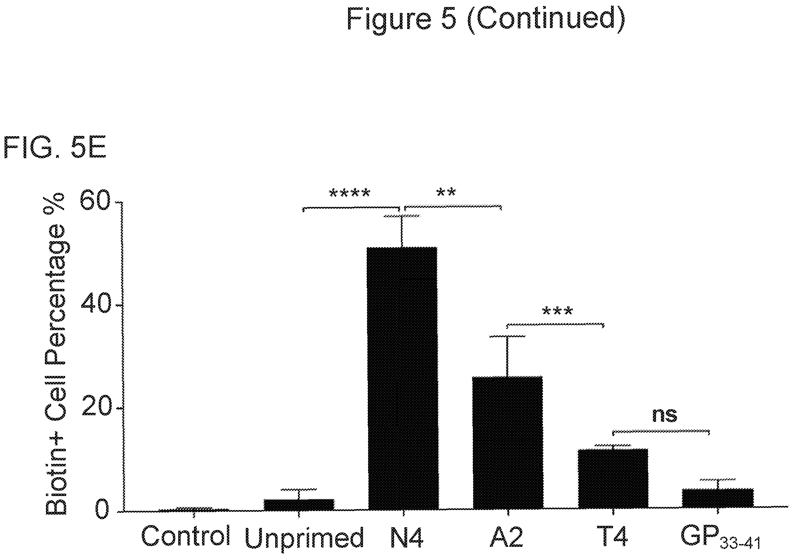

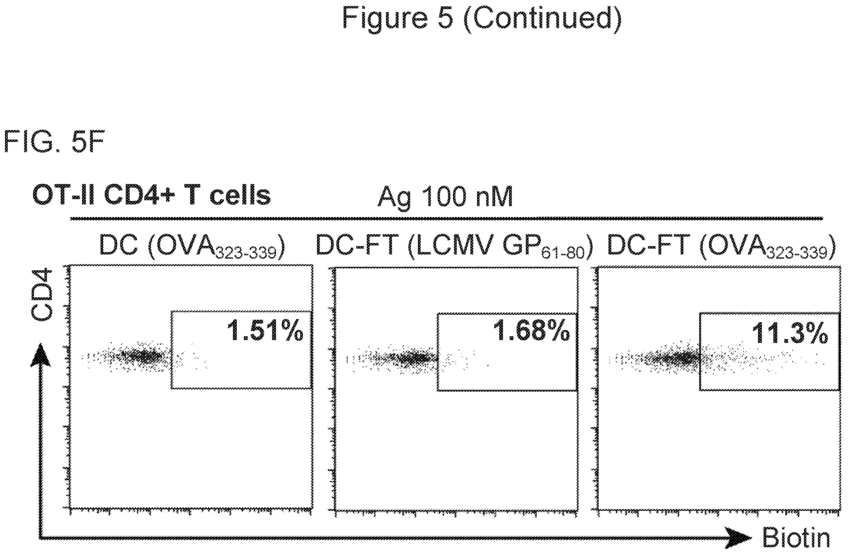

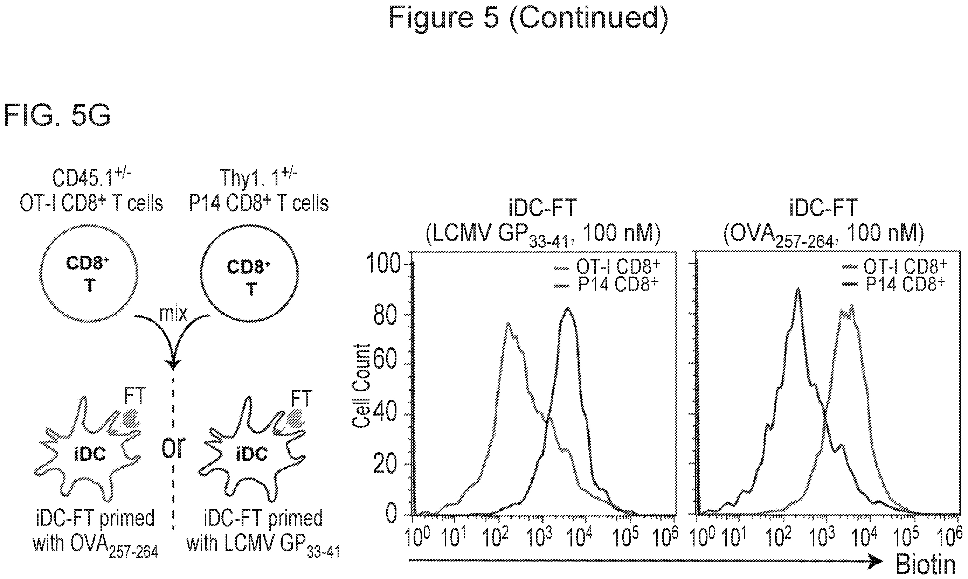

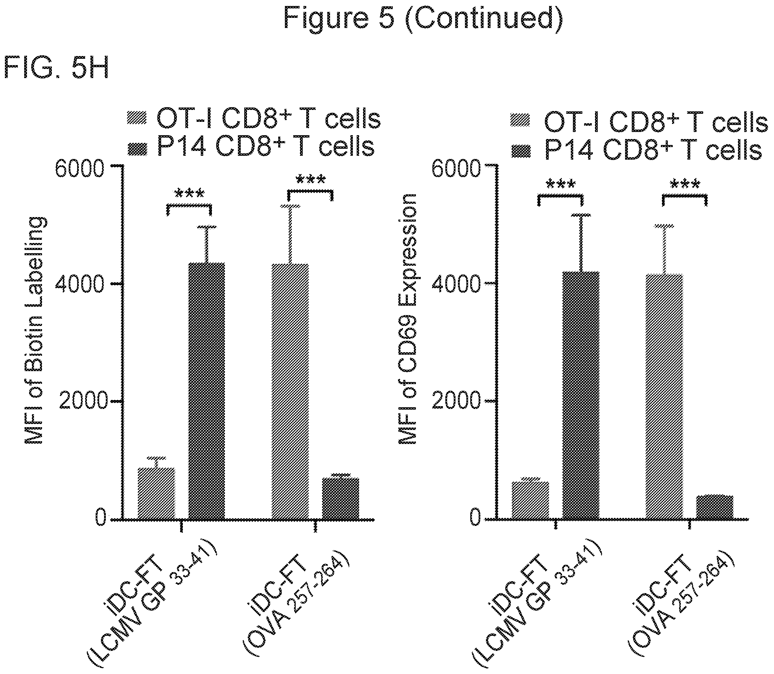

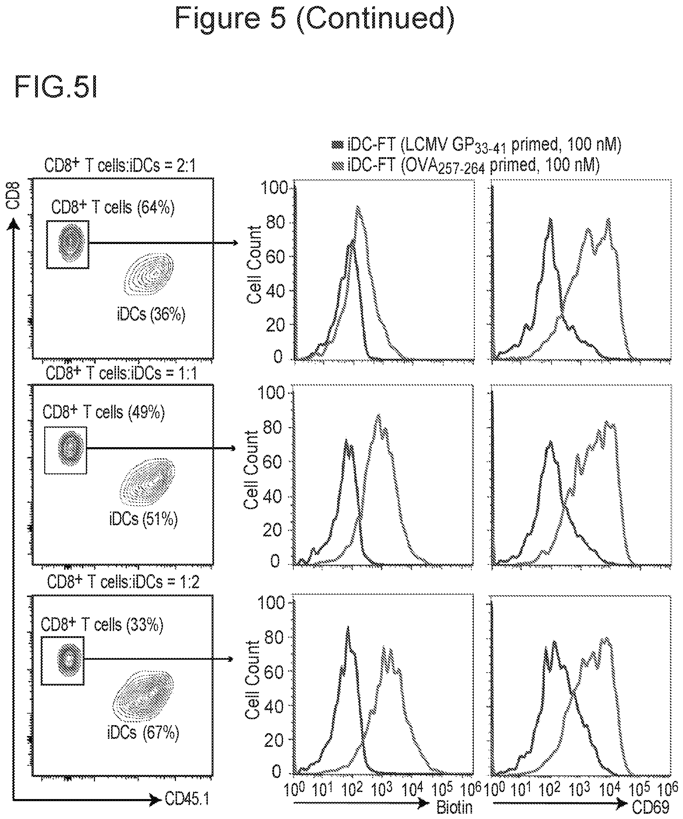

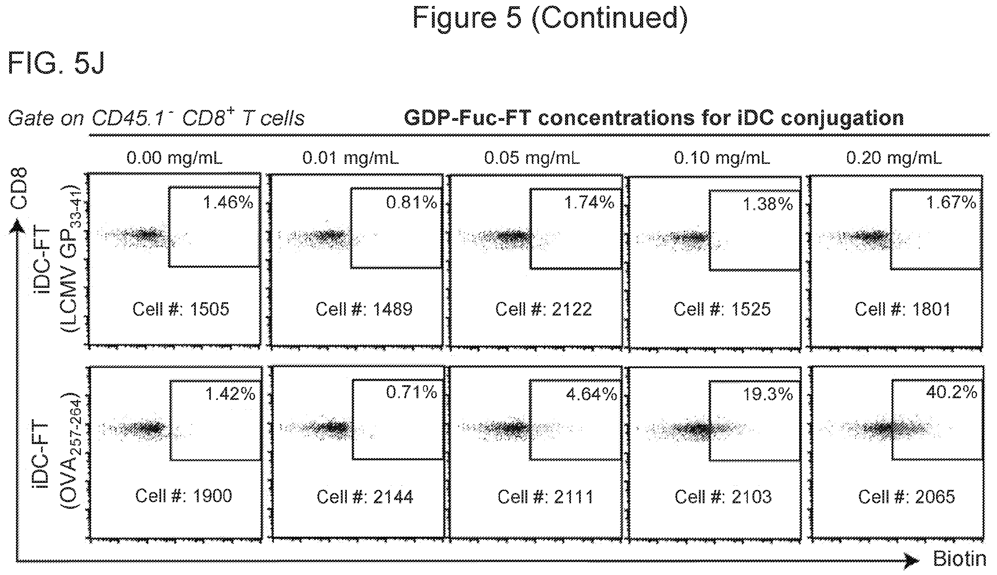

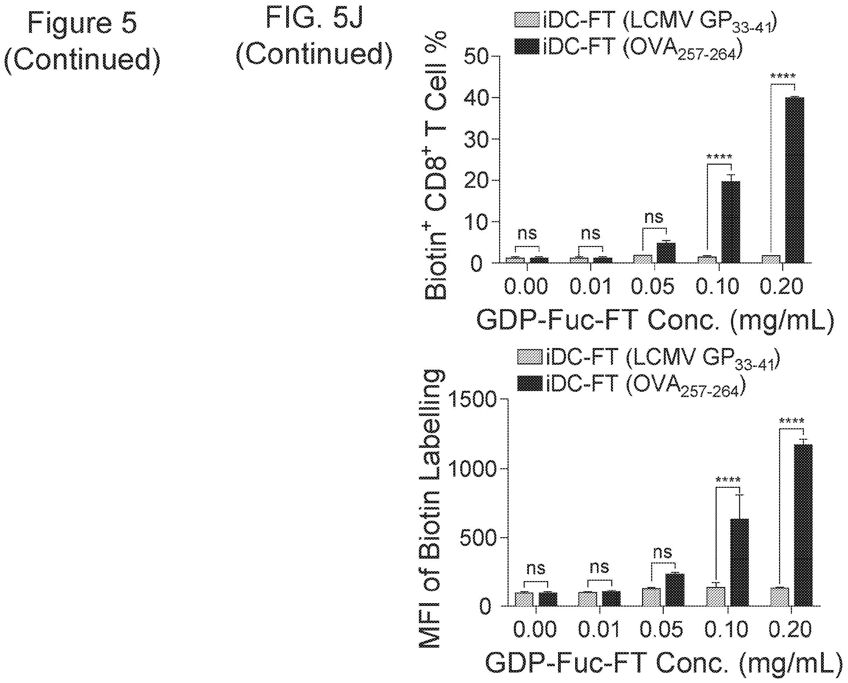

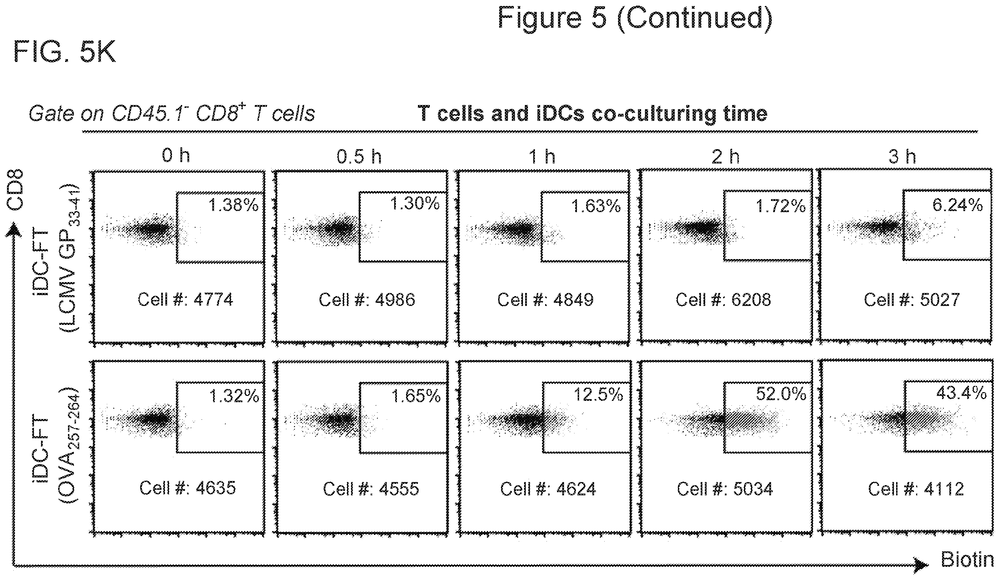

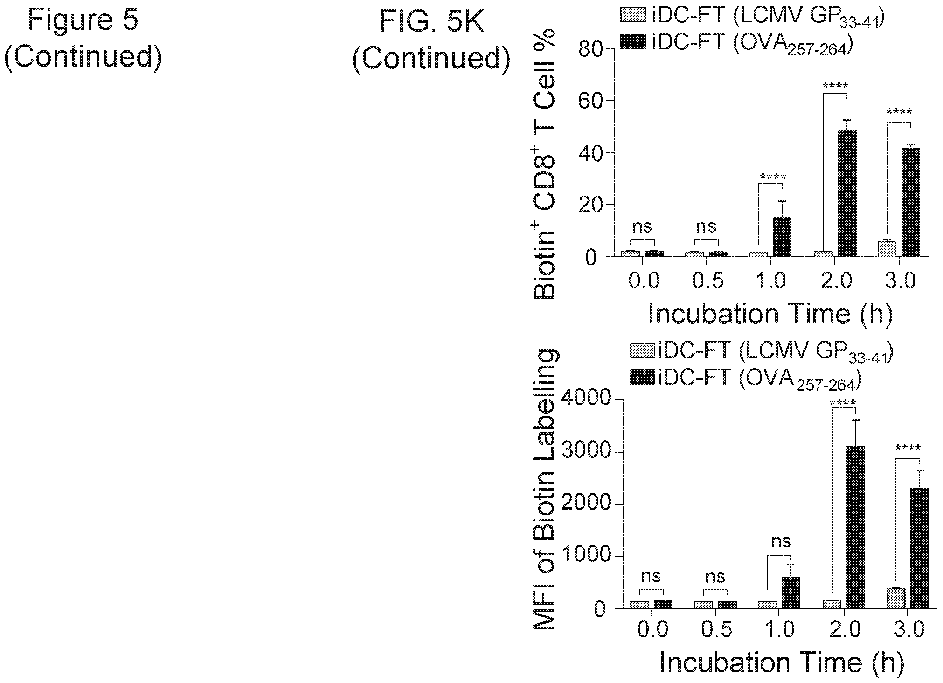

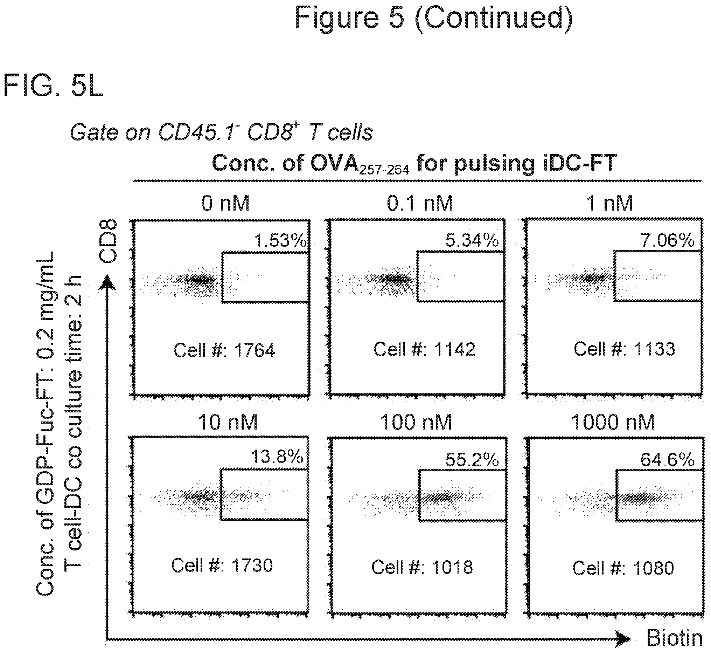

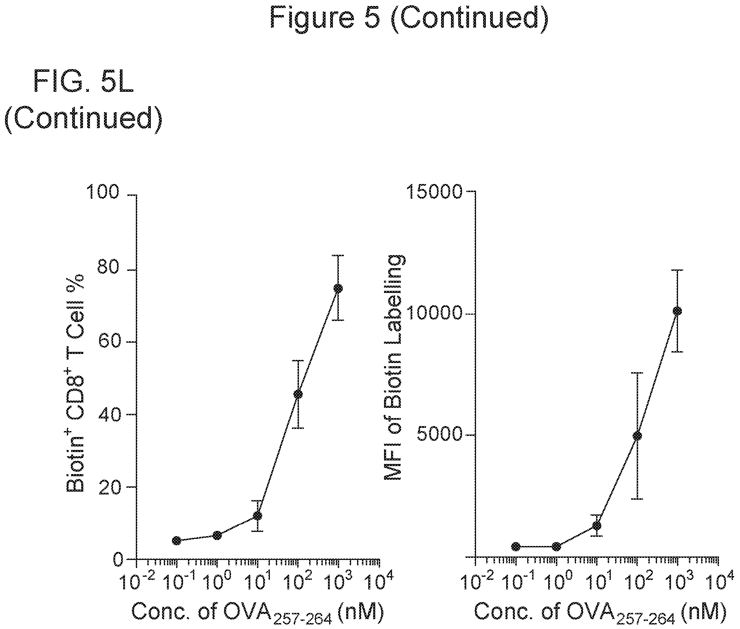

**Figure 5** shows a method for using FT-conjugated Dendritic Cells (DCs) as probes for the detection of DC-CD8+ T cell interactions *ex vivo.* FIG. 5A shows an illustration of how FT modified DCs pulsed with SIINFEKL N4 peptide (OVA₂₅₇₋₂₆₄) (SEQ ID NO: 69) can be used to specifically label naïve CD8+ T cells expressing a transgenic T cell receptor (TCR) specific for that antigen; whereas FT modified DCs pulsed with a different antigen (GP₃₃₋₄₁; peptide KAVYNFATM (SEQ ID NO: 75), derived from the lymphocytic choreomeningitis virus (LCMV) glycoprotein) do not label naïve CD8+ T cells expressing the TCR specific for the SIINFEKL N4 peptide (SEQ ID NO: 69). FIG. 5B shows the experimental method of detecting the DC-CD8+ T cell interaction using biotinylated GDP-fucose, the substrate for FT. FIG. 5C shows flow cytometry analysis of CD8+ T cells contacted, in the presence of biotinylated GDP-fucose, with the FT-conjugated DCs made according to the method described in FIGS. 5A and 5B. The T cells were stained with Alexa Fluor 647-streptavidin, which binds to any biotin-label present on the T cells. FIG. 5D shows flow cytometry analysis of a similar experiment as shown in FIG 5C, except in this case two additional altered peptide ligands (APLs), SAINFEKL(A2) (SEQ ID NO: 70) and SIITFEKL(T4) (SEQ ID NO: 71) derived from the original OT-I N4 ligand SIINFEKL (SEQ ID NO: 69) were also used for priming FT-conjugated DCs. These APLs bind equally well to MHC-I H-2Kb as N4, but differ in their potency for interacting with the transgenic TCR (binding strength: SIINFEKL(N4) (SEQ ID NO: 69) >SAINFEKL(A2) (SEQ ID NO: 70) >SIITFEKL(T4) (SEQ ID NO: 71)). FIG. 5E shows quantification of the flow cytometry results shown in FIG. 5D. FIG. 5F shows results of flow cytometry analysis of an experiment that is the same as shown in FIG 5B, except in this case OT-II mice splenocytes were used instead of the OT-I mice splenocytes that were used in FIG. 5B. CD4+ T cells from OT-II mice express a TCR specific for the OVA₃₂₃₋₃₃₉ peptide, but not for LCMV Gp61-80 peptide *GLNGPDIYKGVYQFKSVEFD* (SEQ ID NO: 72). FIG. 5G shows flow cytometric analysis of antigen-specific Fuc-biotinylation in the mixture of OT-I and P14 CD8+ T cells when incubated with iDC-FT primed with LCMV GP33-41 or OVA257-264. In all figures, mean ± SD (n=3); ns, P > 0.05; *P < 0.05; **P < 0.01; ***P < 0.001; ****P < 0.0001; one-way ANOVA followed by Tukey's multiple comparisons test; two-way ANOVA followed by Sidak's multiple comparisons test. FIG. 5H shows quantification of the results from FIG. 5G. FIG. 5I shows flow cytometric analysis showing antigen specific Fuc-biotinylation of OT-I CD8⁺ T cells at different iDC/T cell ratios. Representative figures from three independent experiments. FIG. 5J-5L show optimization of FucoID condition in the iDC-OT-I CD8+ T co-culturing system. FIG. 5J shows representative flow cytometric analysis of antigen specific fucosyl-biotinylation of OT-I CD8⁺ T cells by iDCs anchored with different amount of FT, as well as bar graph quantification of the flow cytometric analysis. Cells were stained with anti-mCD45.1-FITC, anti-mCD8a-PB and streptavidin-APC for flow cytometry analysis. FIG. 5K shows representative flow cytometric analysis of antigen specific biotinylation of OT-I CD8⁺ T cells by iDC-FT with different co-culturing time before adding GDP-Fuc-biotin, as well as bar graph quantification of the flow cytometric analysis. Cells were stained with anti-mCD45.1-FITC, anti-mCD8a-PB and streptavidin-APC for flow cytometry analysis. FIG. 5L shows flow cytometry analysis of antigen dose dependent curve of the biotinylation of OT-I CD8+ T cells by iDC-FT, as well as a line graph quantification of the flow cytometry analysis showing the percentage of cells that were biotin + and CD8 + at each indicated concentration of OVA₂₅₇₋₂₆₄ as well as total MFI of biotin labeling at the tested concentrations. Experiment procedure is shown in Figure 5B except iDC-FT were pulsed with indicated amounts of OVA₂₅₇₋₂₆₄. In all figures, mean ± SD (n=3); ns, P > 0.05; *P < 0.05; **P < 0.01; ***P < 0.001; ****P < 0.0001; two-way ANOVA followed by Sidak's multiple comparisons test.

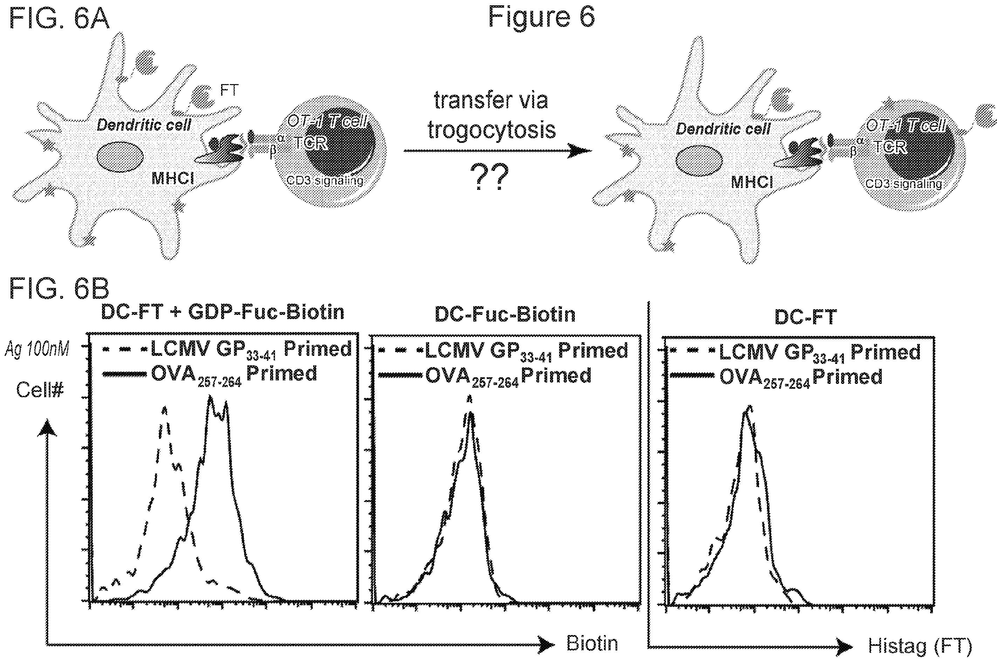

**Figure 6** shows that specific biotinylation between DCs and T cells is not caused by trogocytosis (the bi-directional transfer of plasma membrane fragments between presenting cells and lymphocytes while conjugated). FIG. 6A: shows experimental design to determine if any FT (FT was expressed as a His6-tagged recombinant protein) or Fuc-Bio is transferred via trogocytosis. FIG. 6B shows a histogram of T cells labeled via proximity-based biotinylation (left) or trogocytosis (transfer of Fuc-Bio, mid, or FT-His, right): OT-I CD8+ T cells were robustly labeled via the proximity-based biotinylation by OVA₂₅₇₋₂₆₄ primed DC-FT (left panel). By contrast, no specific biotinylation signals were detected on OT-I CD8+ T cells co-cultured with OVA₂₅₇₋₂₆₄ primed DC pre-labelled with biotin on cell surface (middle panel). In addition, no FT labelled on DC were transferred to OT-I CD8+ T cell surface via trogocytosis (right panel), indicating negligible amounts of Fuc-Bio or FT-His were transferred via trogocytosis.

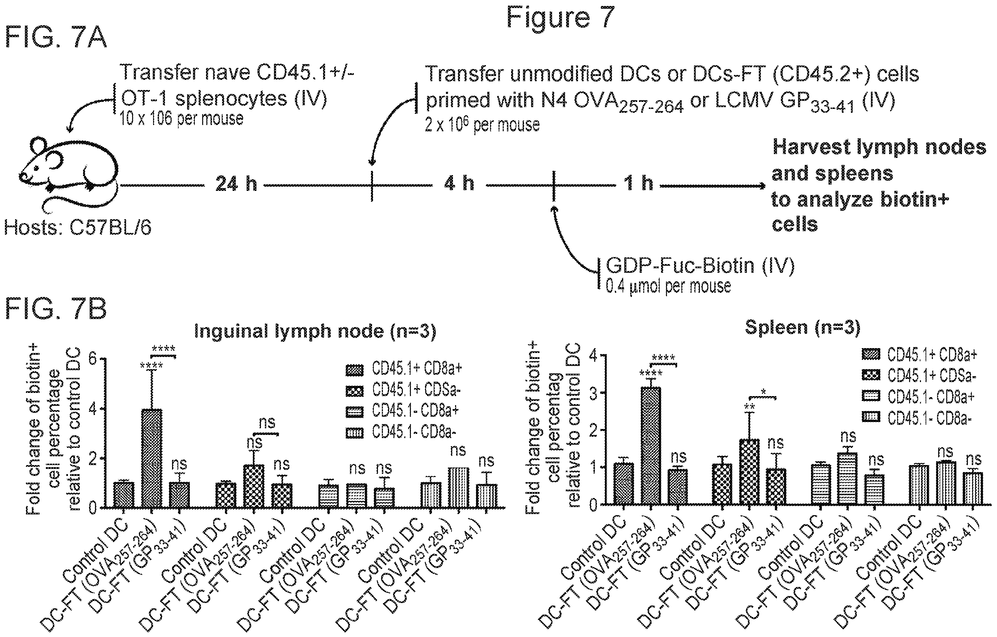

**Figure 7** shows that FT-conjugated dendritic cells (DCs) may be used as probes for the detection of DC-CD8+ T cell interactions *in vivo.* FIG. 7A: shows an illustration of the experimental method of detecting the DC-CD8+ T cell interactions *in vivo* using biotinylated GDP-fucose, the substrate for FT. FIG. 7B shows quantification of flow cytometry results demonstrating that SIITFEKL N4 (SEQ ID NO: 71) pulsed DC-FT could selectively interact and label naive CD8+ T cells from donor mice in both lymph nodes and spleens while this was not observed in control DC and LCMV GP₃₃₋₄₁ pulsed DC-FT. In all figure 7 FIGS, ns P > 0.05; ** P < 0.01; *** P < 0.001; **** P < 0.0001; one-way ANOVA followed by Tukey's multiple comparisons test.

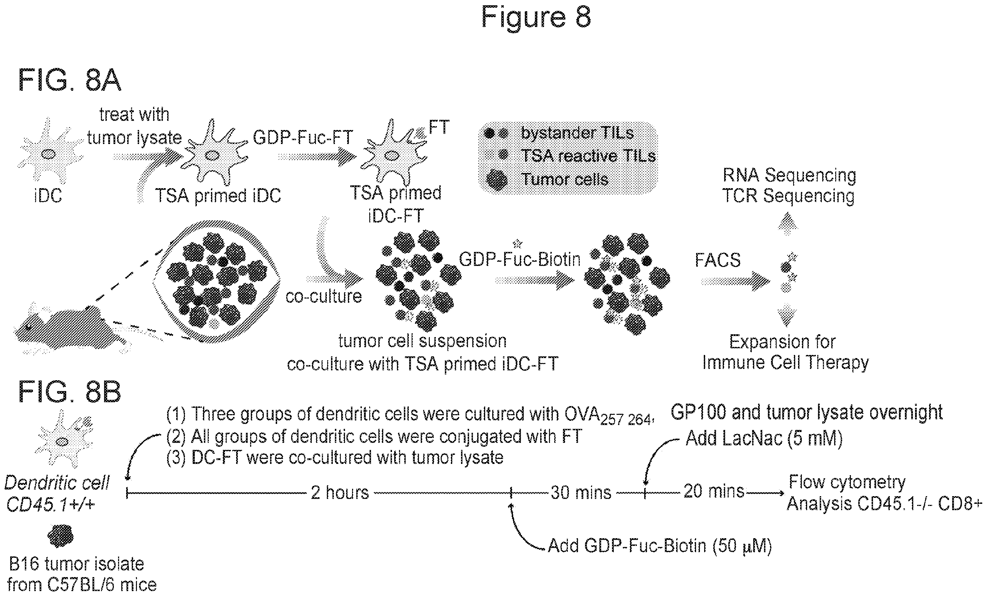

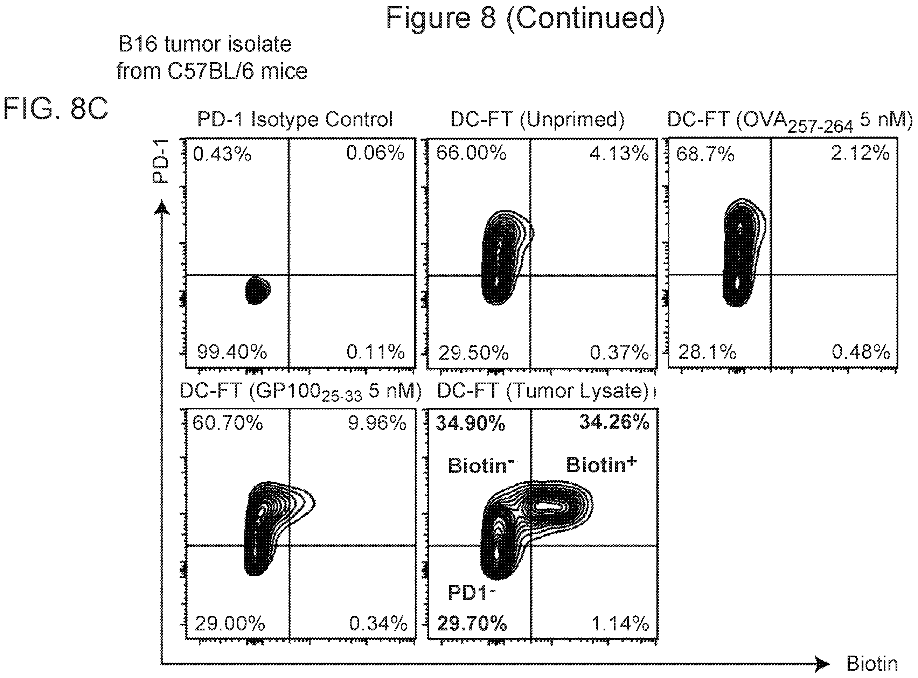

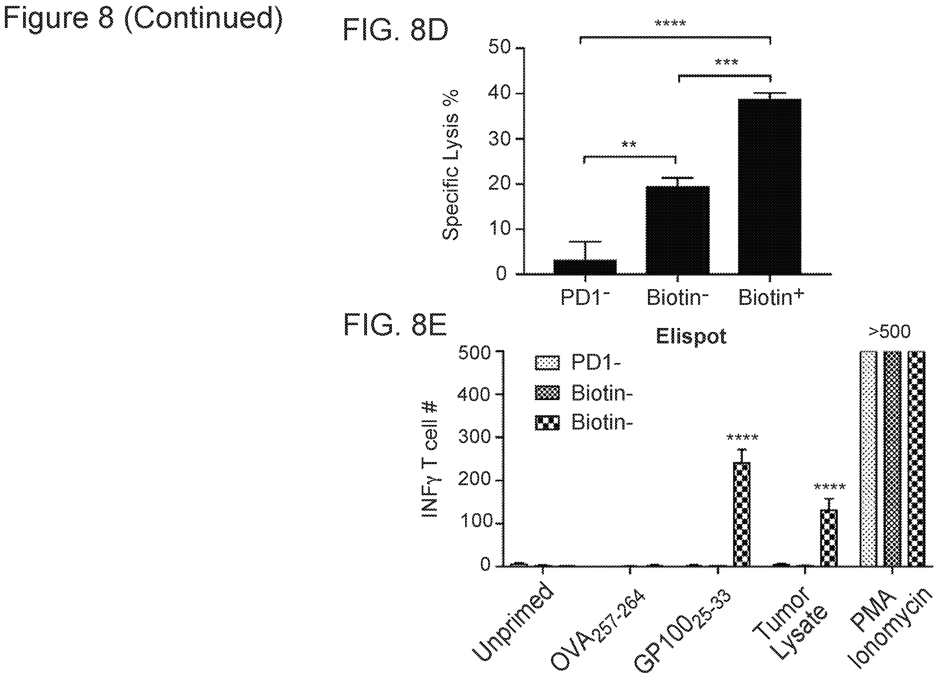

**Figure 8** shows that FT functionalized DCs may be used as probes to detect and isolate tumor reactive T cells from infiltrating lymphocytes (TILs) in B16 melanoma (Overwijk, W. W.; Restifo, N. P. Current protocols in immunology 2001, Chapter 20, Unit 20 21). FIG. 8A shows an illustration of the FACS-based experimental method of detecting and isolating tumor reactive T cells using DC-FT pretreated with tumor lysate. DC-FT cells are capable of presenting neoantigens from tumor lysate, and then upon formation of an immunological synapse with T cells bearing neoantigen specific TCRs, interaction-based biotin tagging of the T cells occurs. By this way, the small portion of neoantigen specific T cells are selected for further analysis. FIG. 8B shows the workflow for the direct detection of isolation of tumor-reactive T cells from TILs of B 16 melanoma. FIG. 8C shows FACS data from the Experiment described in FIG. 8B, in which CD8+ T cells were labeled for the tumor specific antigen TSA-reactive T cell marker PD-1 and the Biotin label in order to identify and sort for TSA-reactive T cells that actually interact with the primed DC-FT cells. FIG. 8D shows results of an *ex vivo* tumor killing assay using cells sorted from the experiment described in FIG. 8C. Biotin+ (red) and biotin - (blue) CD8 + T cells were sorted by FACS, cultured with IL-2 in T cell medium (IL2: 100 IU/mL) for 12hours, then B16 melanoma cells (stably transduced with firefly luciferase) were co-cultured with sorted CD8+ T cells for 20 hours as a ratio of 1:4. Then the killing of B16 tumor were quantified through the luciferase activity . FIG. 8E shows a follow-up experiment using ELISpot assay to assess the tumor-reactivity of the cells expanded as described in FIG. 8D based on IFNγ secretion following co-culturing with DCs pulsed with tumor lysates or amino acids 25 to 33 fragment of human melanoma antigen gp100 (KVPRNQDWL, SEQ ID NO: 73), also known as GP100₂₅₋₃₃. In all figure 8 FIGS, ns P > 0.05; ** P < 0.01; *** P < 0.001; **** P < 0.0001; one-way ANOVA followed by Tukey's multiple comparisons test.

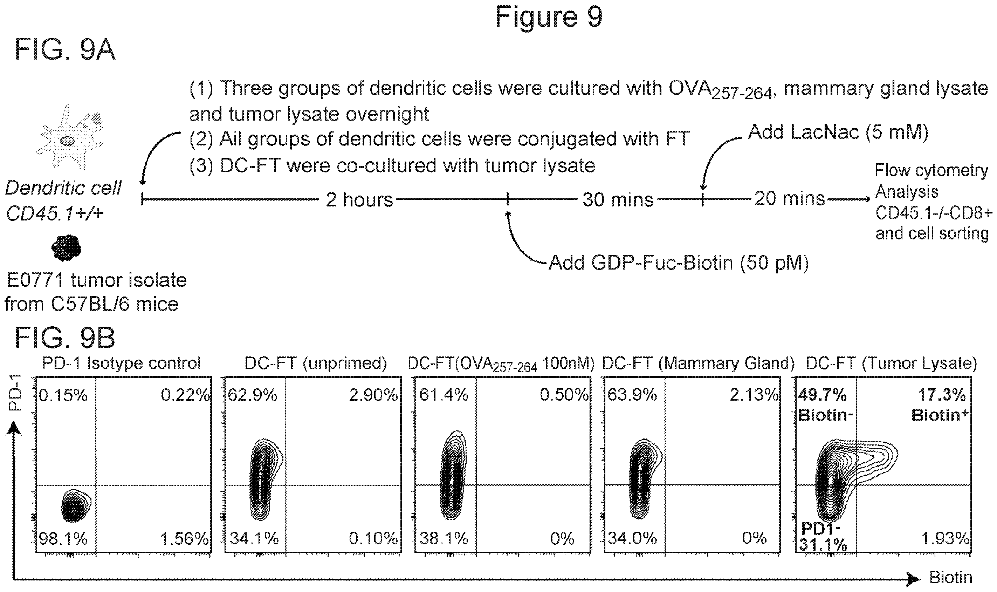

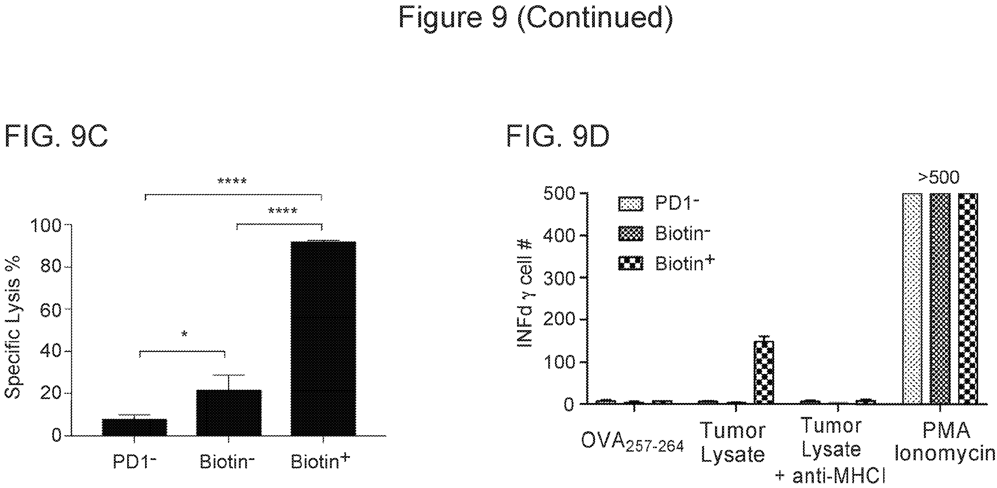

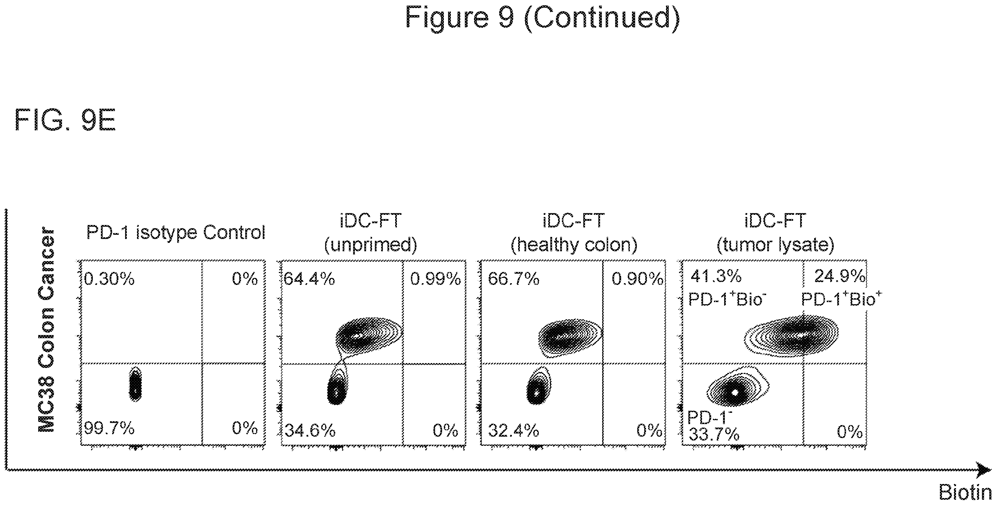

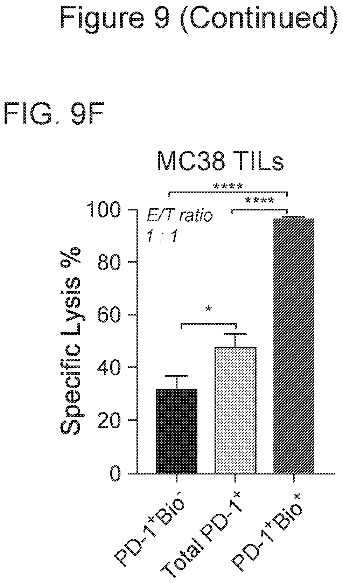

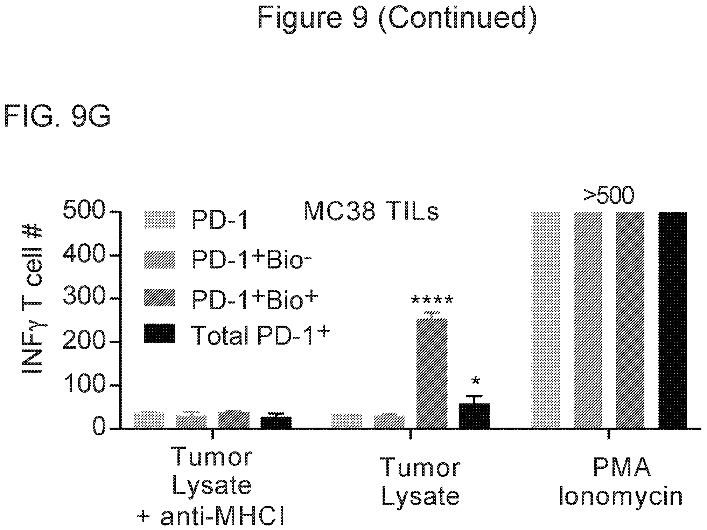

**Figure 9** shows that FT functionalized DCs may be used as probes to detect and isolate tumor reactive T cells from infiltrating lymphocytes (TILs) in triple negative breast tumor E0771. FIG. 9A: shows an illustration of the FACS-based workflow for the direct detection and isolation of tumor-reactive T cells from TILs of E0771 tumors using DC-FT pretreated with tumor lysate. FIG 9B shows FACs results demonstrating that DC-FT primed with E0771 tumor lysate specifically labelled 17.3% CD8+ TILs and all of these cells are PD-1+. In contrast, DC-FT treated with no antigen, OVA₂₅₇₋₂₆₄ and healthy mammary gland lysate gave minimal biotinylation to CD8+ TILs. FIG. 9C shows results of an *ex vivo* tumor killing assay (lysis) using expanded cells that had been isolated according to the experiment described in FIG. 9B. FIG. 9D shows the tumor-reactivity of the expanded cells that had been isolated according to the experiment described in FIG. 9B based on IFNγ secretion following co-culturing with DCs pulsed with no antigens or OVA₂₅₇₋₂₆₄, tumor lysates or tumor lysate + anti-mouse MHCI in an ELISpot assay. In all figures, ns P > 0.05; ** P < 0.01; *** P < 0.001; **** P < 0.0001; one-way ANOVA followed by Tukey's multiple comparisons test. FIG. 9E shows representative flow cytometric analysis of tumor antigen dependent Fuc-biotinylation of CD8+ TILs in MC38 colon cancer models. FIG. 9F shows specific lysis reactivity of expanded total PD-1+ TILs and PD-1+Bio+ TILs against MC38 cancer cells at effector-to-target ratio of 1:1; n=3. FIG. 9G shows IFN-γ ELISPOT showing distinct reactivity of expanded PD-1-, PD-1+Bio- and PD-1+Bio+ TILs upon MC38 tumor antigen re-stimulation. n=3

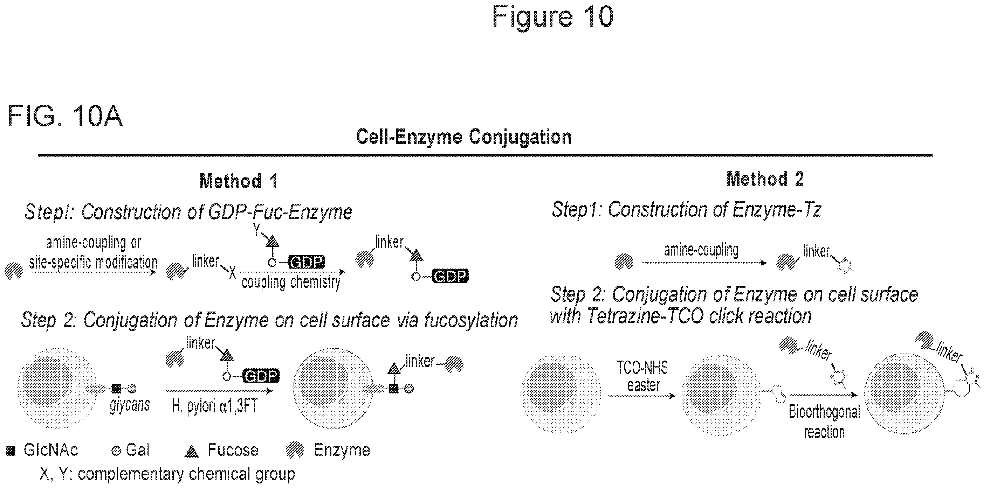

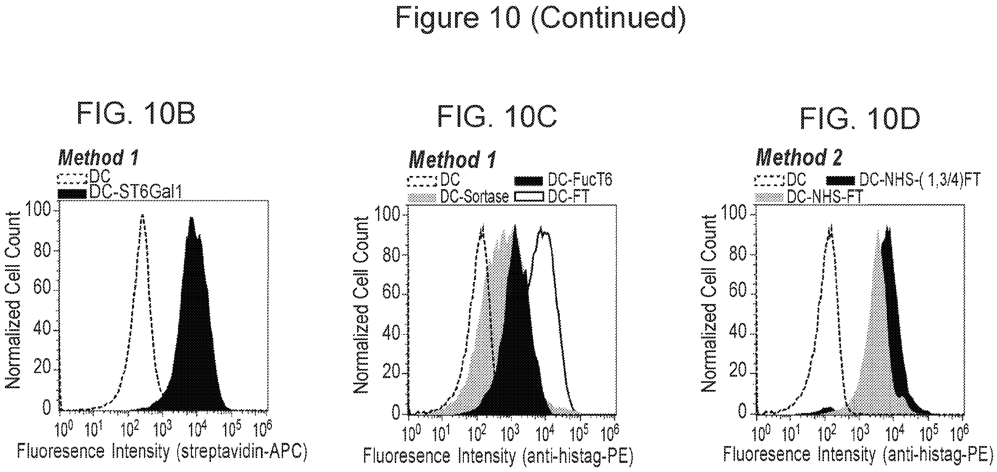

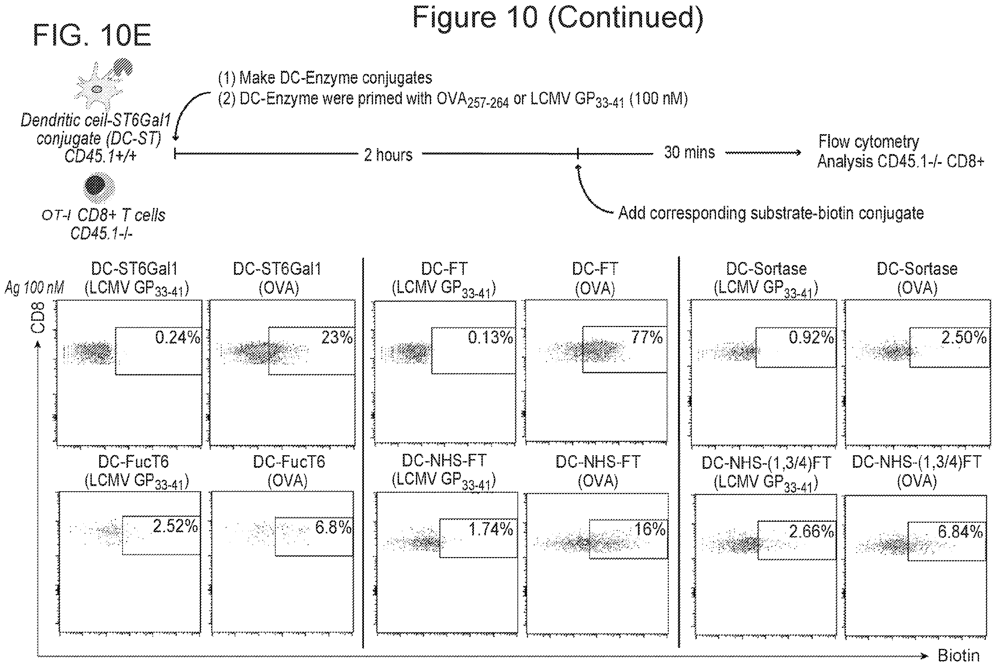

**Figure 10** demonstrates the versatility of the fucosyltransferase-based conjugation method for conjugating enzymes to the surface of a cell and also provides for chemical means of conjugation. FIG. 10A (left side) illustrates a method for conjugating any enzyme to the surface of a cell using the fucosyltransferase-based method described in FIG 2A (Method 1). FIG. 10A (right side) shows a method for conjugating any enzyme to the surface of a cell using a chemical conjugation-based method (Method 2). FIG. 10B shows flow cytometry analysis with streptavidin-APC confirming that biotinylated human 2,6αsialyltransferase (ST6Gal1) was conjugated on the surface of immature DCs using Method 1 to form DC-ST6Gal1 conjugate. FIG. 10C shows flow cytometry analysis with anti-his tag-phycoerythrin (PE) confirming that His-tagged H. pylori α1,3fucosyltransferase (FT), His-tagged human α1,3fucosyltransferase (FUT6), and His-tagged Sortase A (5M) were conjugated on the surface of immature DCs using Method 1 to form DC-FT and DC-Sortase conjugates, respectively.
FIG. 10D shows flow cytometry analysis with anti-his tag-phycoerythrin (PE) confirming that His-tagged H. pylori α1,3fucosyltransferase (FT) and H. pylori α1,3/4fucosyltransferase ((1,3/4)FT) were conjugated on the surface of immature DCs using Method 2 chemical conjugation to form DC-NHS-FT and DC-NHS-(1,3/4)FT conjugates. FIG. 10E shows a comparison of DC-CD8+ T cell proximity labelling induced by DC-Enzyme conjugates made via Method 1 and Method 2. FIG. 10E top panel shows the workflow for this experiment. FIG. 10E bottom panel shows flow cytometry analysis of cell mixtures stained with anti-CD8-Pacific Blue, anti-CD45.1-FITC and streptavidin-APC.

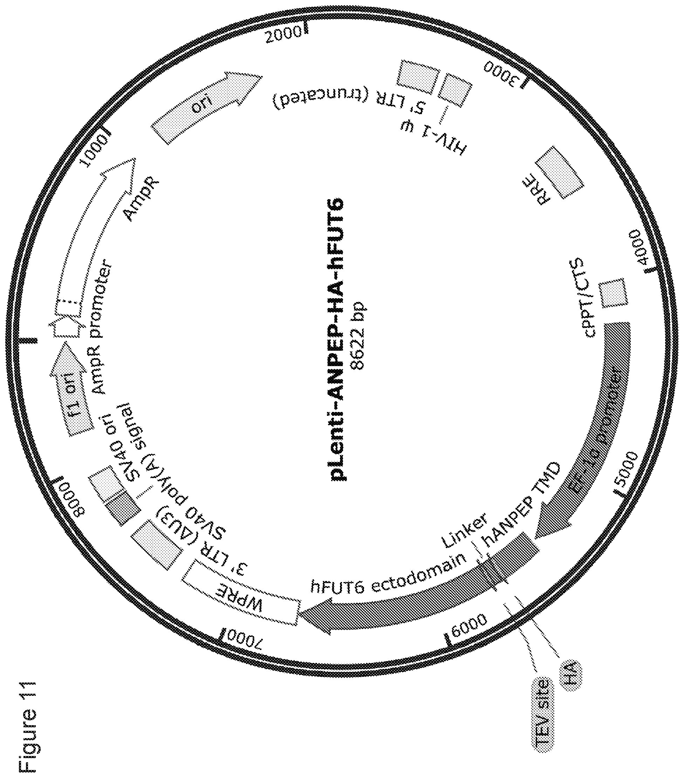

**Figure 11** shows a lentiviral expression vector map for inducing cell-surface expression of *H. pylori* α1,3fucosyltransferase on a bait cell such as, e.g., a dendritic cell.

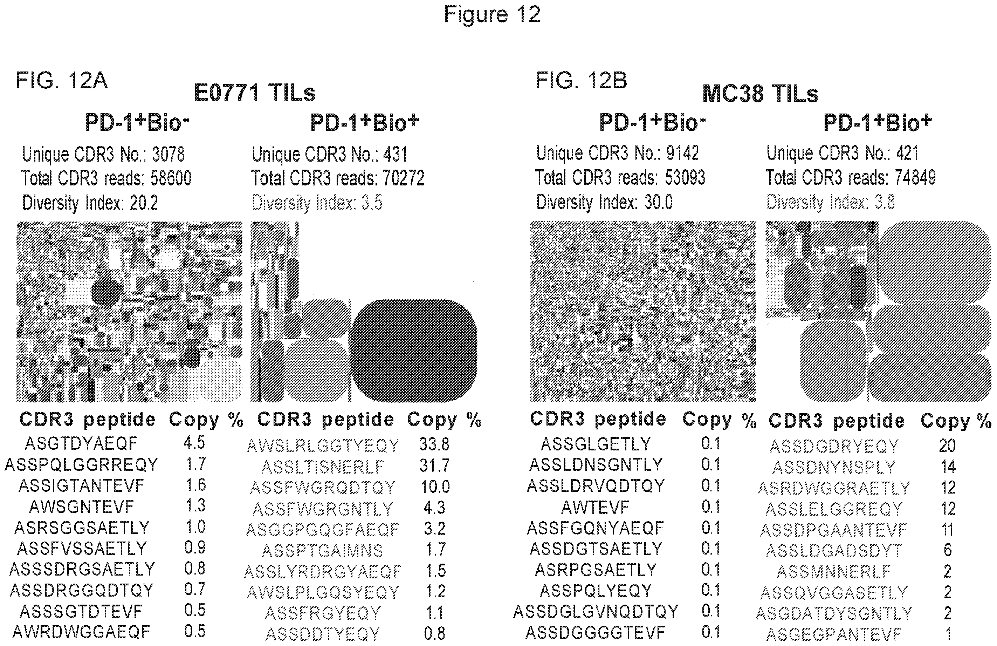

**Figure 12** shows representative TCRβ RNA sequencing results of PD-1+Bio- and PD-1+Bio+ TILs from E0771 tumor (FIG. 12A) and MC38 tumor (FIG. 12B). Each spot in the plot represents a unique clonotype: V-J-CDR3, and the size of a spot denotes the relative frequency. The entire plot area is divided into sub-area according to V usage, which is subdivided according to J usage and then CDR3 frequency, subsequently. The top 10 most abundant CDR3 encoded peptide sequences in each population are shown. Unique sequences only found in PD-1+Bio+ population are highlighted in red. Three independent biological replicates were analyzed to confirm the TCR clonotype enrichment in PD-1+Bio+ TILs.

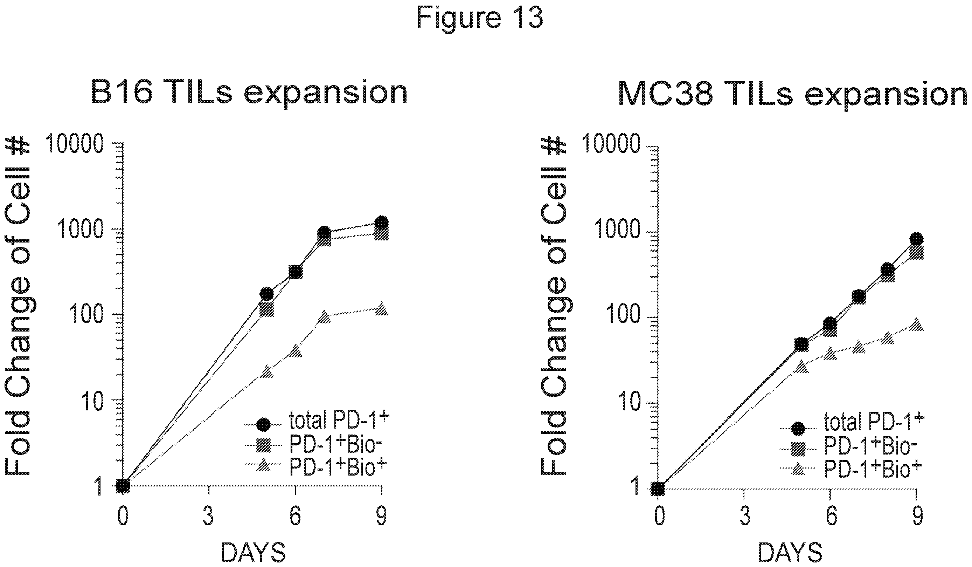

**Figure 13** shows cell expansion curves of total PD-1+, PD-1+Bio- and PD-1+Bio+ TILs under the rapid expansion protocol. Representative expansion curves from three replicates are shown.

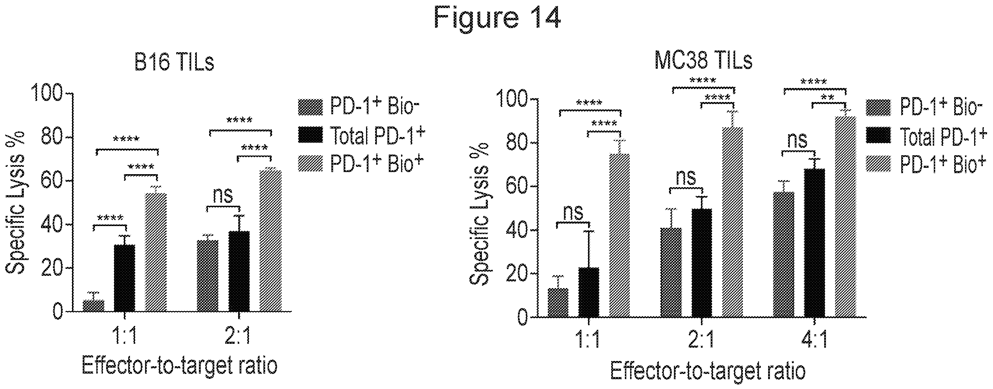

**Figure 14** shows comparisons of the cancer cell killing activity of expanded total PD-1+ TILs and PD-1+Bio+ TILs at different effector-to-target cell ratios, related to FIGS. 8D, 9C, and 9F. Mean ± SD (error bars); n=3; ns, P > 0.05; *P < 0.05; **P < 0.01; ***P < 0.001; ****P < 0.0001; two-way ANOVA followed by Sidak's multiple comparisons test.

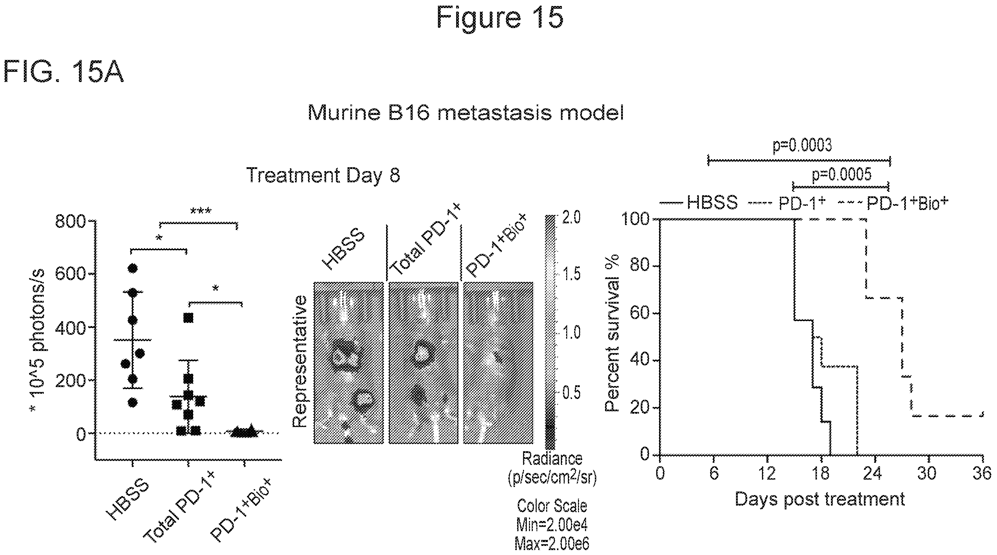

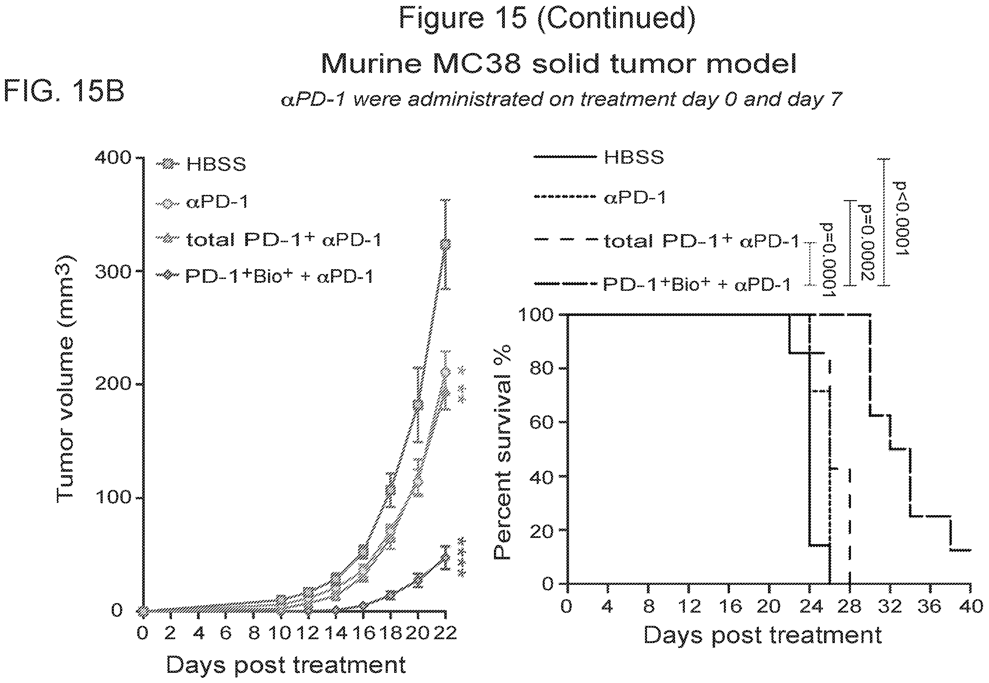

**Figure 15** shows *in vivo* tumor reactivity of PD-1+Bio+ and PD-1+ TILs. FIG. 15A shows *in vivo* tumor reactivity in a murine B16 metastasis tumor model. C57BL/6 mice were intravenously injected with 0.5 x 10⁶ B16-luc cells. TILs treatments were performed as described in method on day 3 of tumor inoculation. On day 8 of TIL transfer, tumors were imaged by IVIS system. The sizes of the tumors and representative images are shown (mean ± SD); HBSS: 7 mice; total PD-1+: 8 mice; PD-1+Bio+: 6 mice. FIG. 15B shows *in vivo* tumor reactivity of PD-1+Bio+ and PD-1+ TILs in a murine MC38 s.c tumor model. MC38 cells were s.c. injected into the right flanks of male C57BL/6 mice (0.5 x 10⁶ cells per mice). Mice were irradiated (5 Gy) on day 2 of tumor inoculation. Then TILs treatments were performed as described in method on day 3 of tumor inoculation. Tumor volumes were measured every 2 days from day 10 of TILs treatment. The average size of tumors of each group until treatment day 22 are shown; HBSS: 7 mice; anti-PD-1: 7 mice; total PD-1+: 7 mice; PD-1+Bio+: 8 mice. In above figures, mean ± SD (error bars); ns, P > 0.05; *P < 0.05; **P < 0.01; ***P < 0.001; ****P < 0.0001; data were analyze by one-way ANOVA followed by Tukey's multiple comparisons test or t-test. Survival data were analyzed by Log-rank (Mantel-Cox) test.

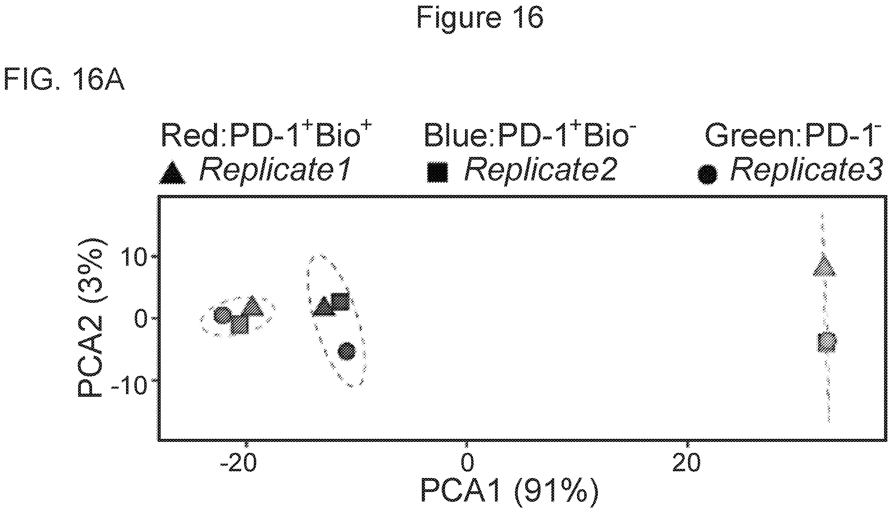

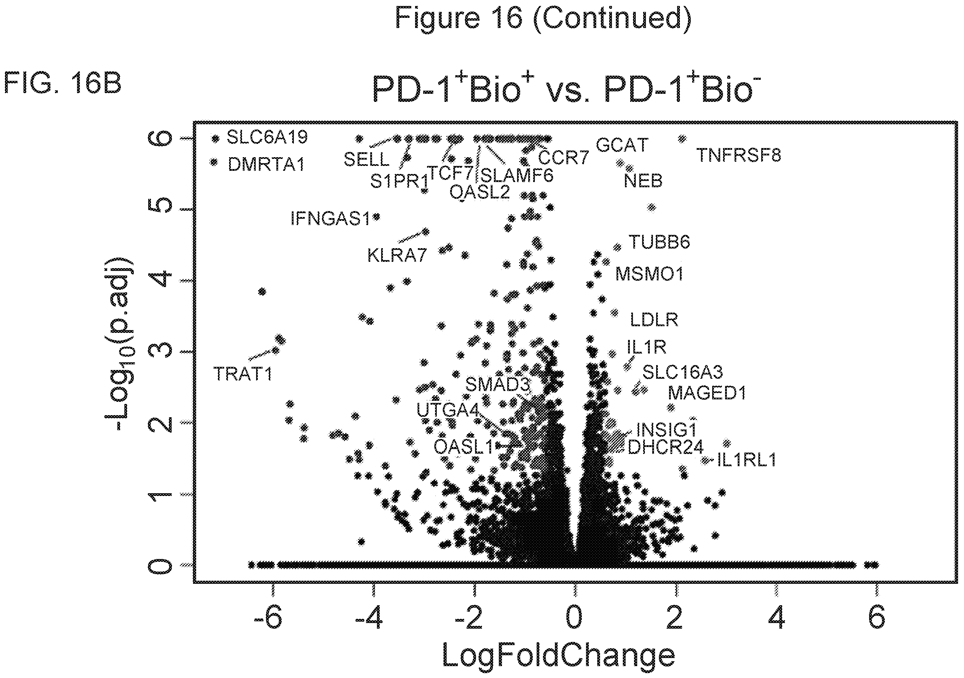

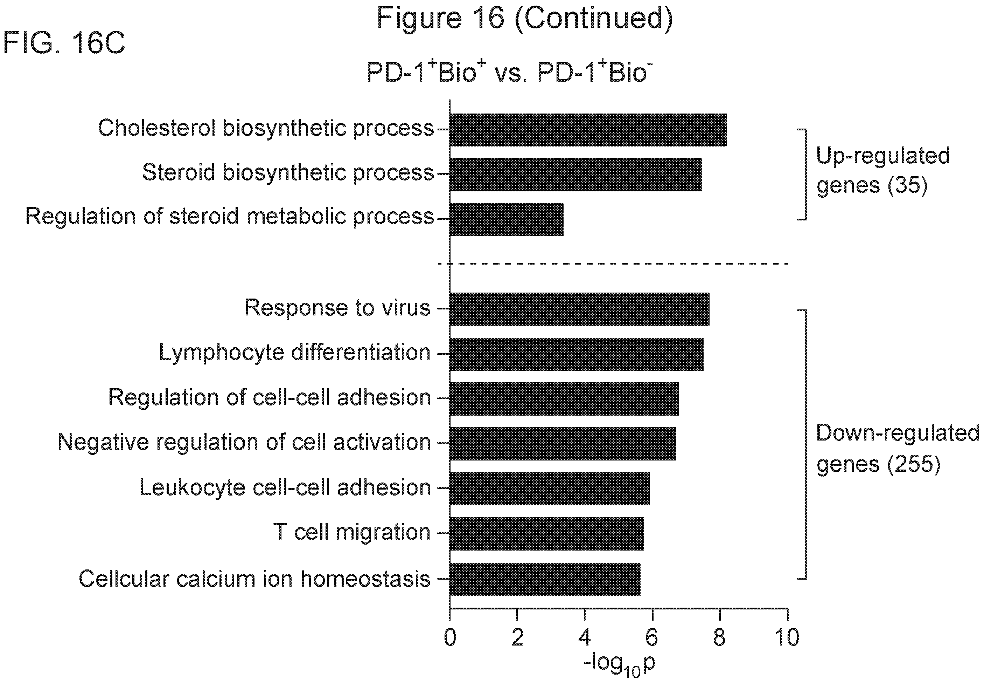

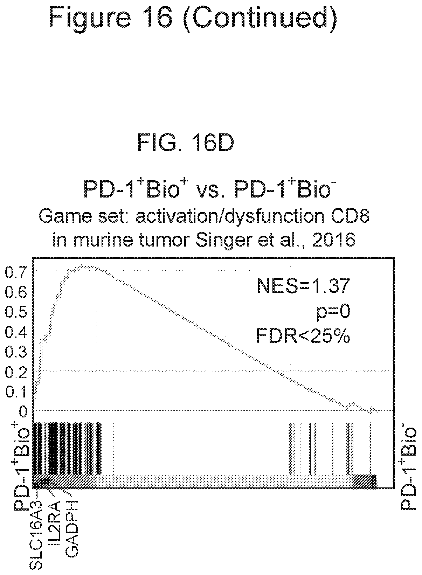

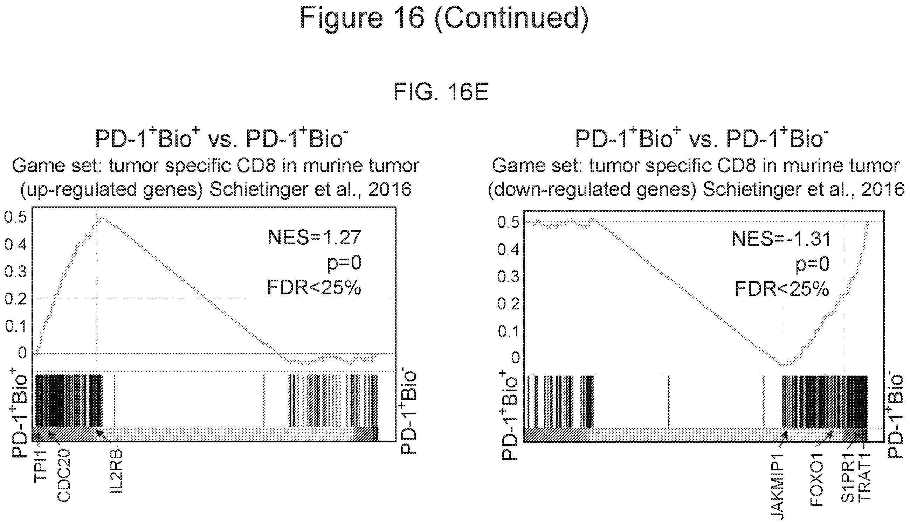

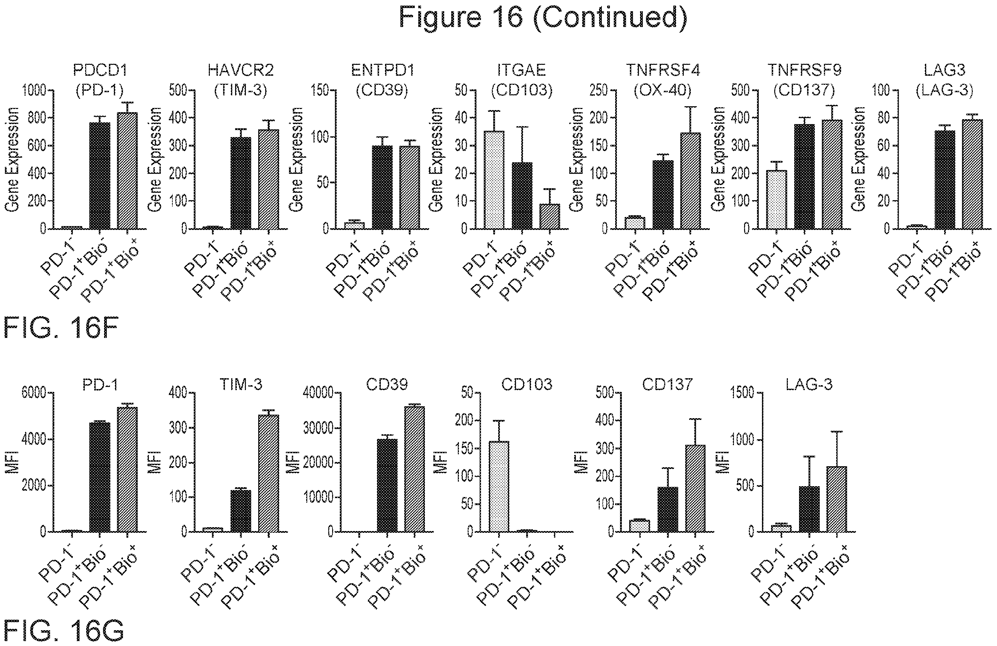

**Figure 16** shows distinct gene-expression signatures of PD-1+Bio+, PD-1+Bio- and PD-1- CD8+ TILs isolated from MC38 tumors. FIG. 16A shows principal component analysis (PCA )of the transcriptome of PD-1+Bio+ (red, far left cluster), PD-1+Bio- (blue, middle clusture) and PD-1- (green, far right cluster) CD8+ TILs isolated from murine MC38 tumors. Dots represent samples of the three different populations (grouped by colors) from a total of three biological replicates (grouped by shapes). PCA 1 and 2 represents the largest source of variation. FIG. 16B shows a volcano plot of up- (red) and down- (blue) regulated genes between PD-1+Bio+ and PD-1+Bio- TILs. Significance was determined as Benjamini-Hochberg FDR (p.adjust) < 0.05 and |log2(fold change)| ≥ 0.6. FIG. 16C shows biological processes (GO terms) enriched in the up- and down-regulated genes identified in FIG. 16B. FIG. 16D shows gene set enrichment analysis (GSEA) of activation/dysfunction CD8 gene module (Singer et al., 2016) in the transcriptome of PD-1+Bio+ vs. that of PD-1+Bio- TILs. FIG. 16E shows GSEA of up- and down-regulated tumor specific CD8 gene signature (Schietinger et al., 2016) in the transcriptome of PD-1+Bio+ vs. that of PD-1+Bio- TILs. FIG. 16F shows the comparison of representative gene expression of PD-1+Bio+, PD-1+Bio- and PD-1- TILs. FIG. 16G shows the expression of PD-1, CD137, TIM-3, CD39 and CD103 in PD-1+Bio+, PD-1+Bio- and PD-1- CD8 TILs from murine MC38 tumor according to flow cytometric analysis. Data obtained from at least two independent replicates.

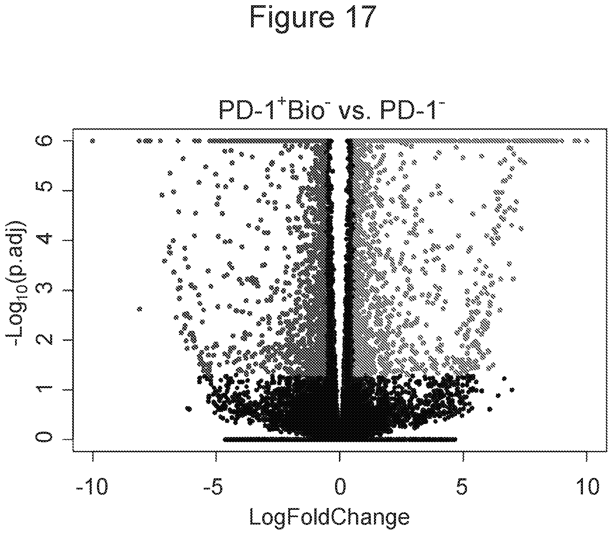

**Figure 17** shows a volcano plot of up- (red) and down- (blue) regulated genes of PD-1+Bio- vs. PD-1- TILs. Significance was determined as Benjamini-Hochberg FDR (p.adjust) < 0.05 and |log2 (foldchange) |≥ 0.6 (± 1.5 fold).

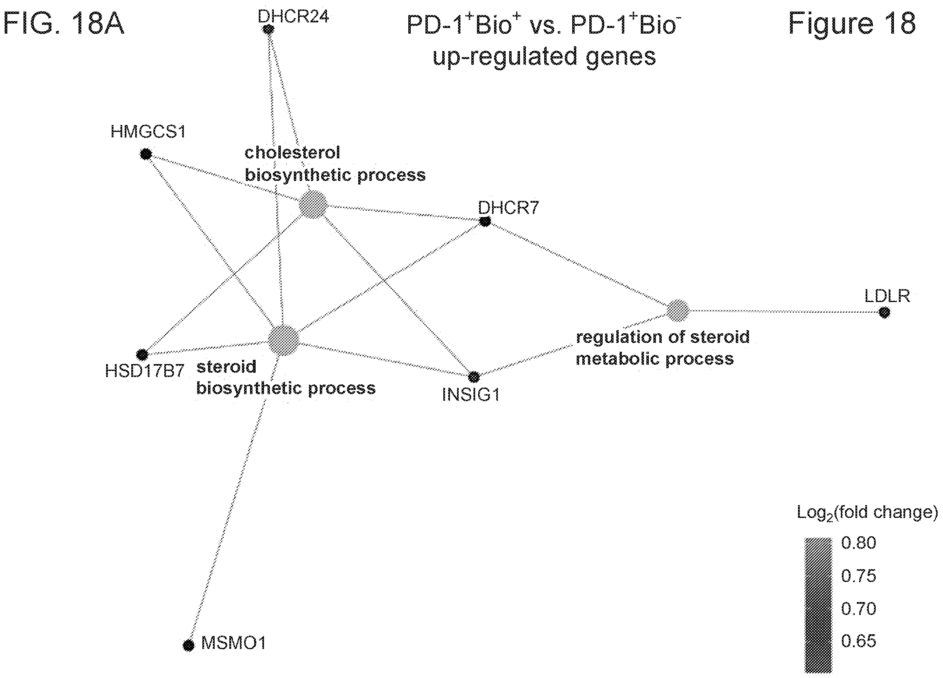

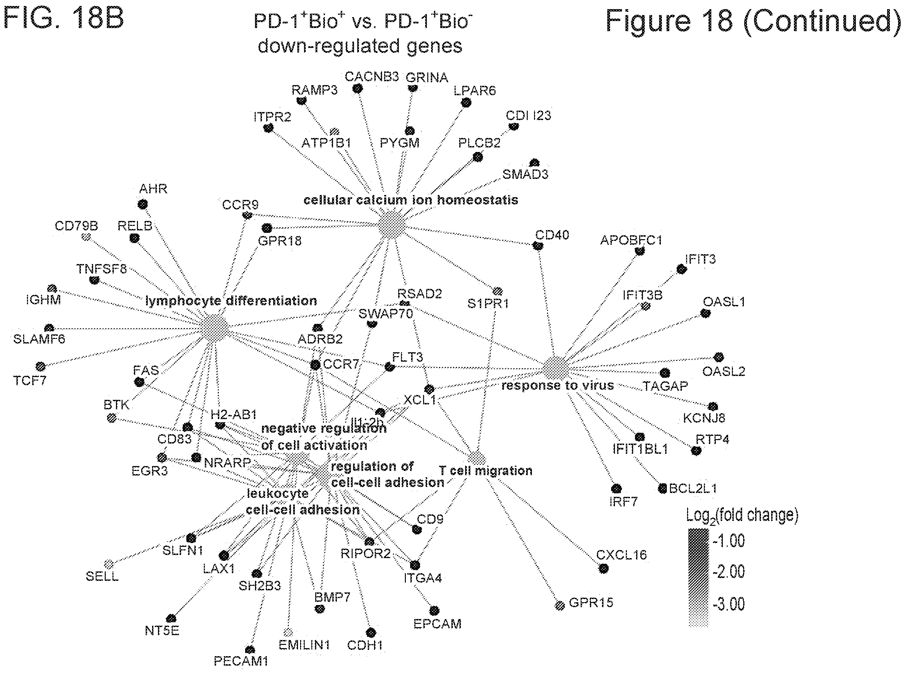

**Figure 18** shows enriched biological process by up- regulated (FIG. 18A) and down-regulated (FIG. 18B) genes in PD-1+Bio+ vs. PD-1+Bio- TILs, related to Figure 16C. Gene concept networks was generated according to gene ontology (GO) over-representation analysis, showing genes involved in each enriched GO term.

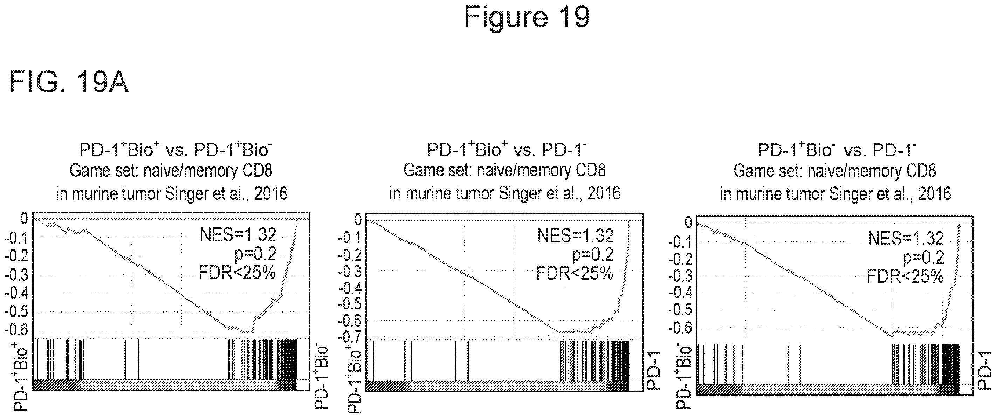

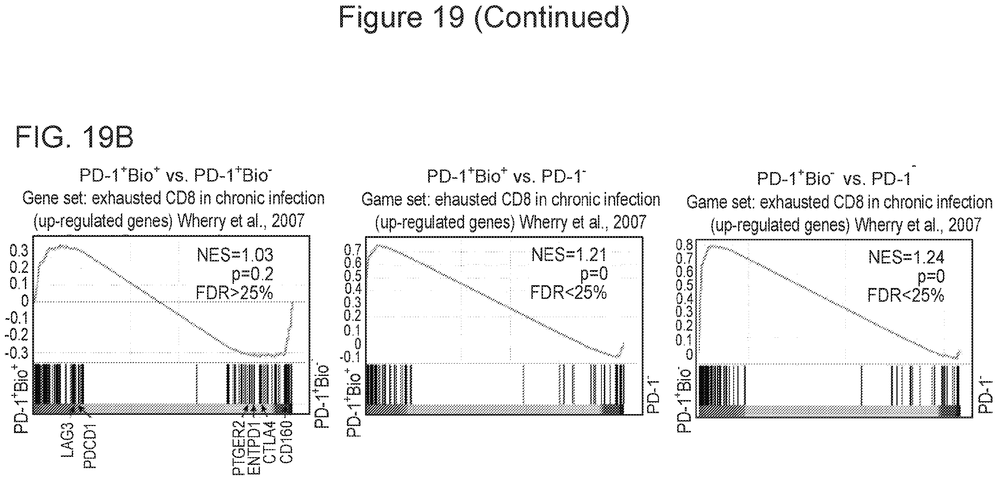

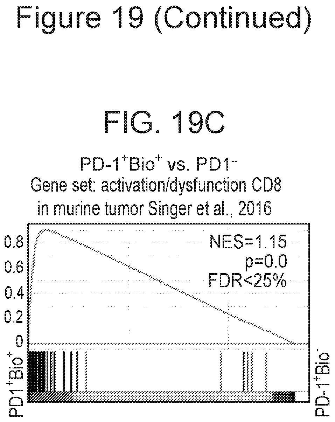

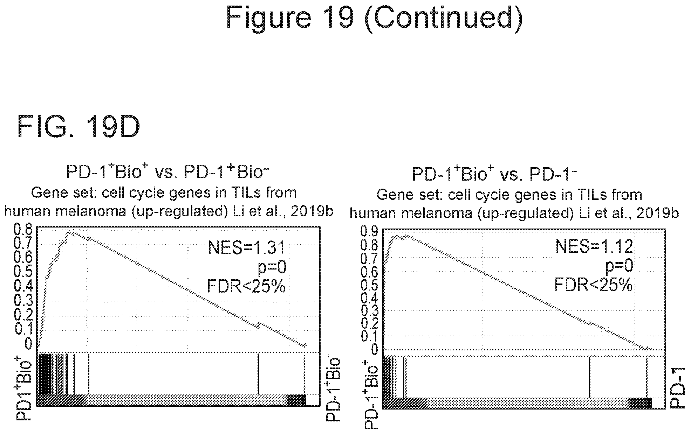

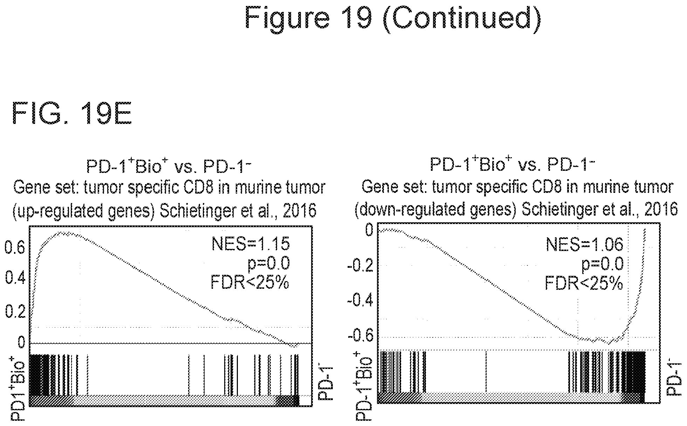

**Figure 19** shows gene set enrichment analysis (GSEA) of the subsets of MC38 TILs using reported gene signatures, related to FIG. 16D and 16E. FIG. 19A shows three subsets of MC38 TILs that were compared with each other. Naive/memory CD8 gene signatures were significantly enriched in the transcriptome of PD-1- TILs. FIG. 19B: three subsets were compared with each other. No enrichment of exhausted CD8 gene signatures were observed when comparing the transcriptome of PD-1+Bio+ with that of PD-1+Bio- TILs. The transcriptome of PD-1+Bio+ and PD-1+Bio- TILs showed similar enrichment of the exhausted CD8 signatures when comparing with that of PD-1- TILs separately. FIG. 19C shows GSEA of activation/dysfunction CD8 gene module in the transcriptome of PD-1+Bio+ vs. that of PD-1- TILs. FIG. 19D shows GSEA of up-regulated cell cycle gene module in the transcriptome of PD-1+Bio+ vs. that of PD-1+Bio- and in the transcriptome of PD-1+Bio+ vs. that of PD-1- TILs. FIG. 19E shows GSEA of up- and down-regulated tumor specific CD8 gene signature in the transcriptome of PD-1+Bio+ vs. that of PD-1- TILs.

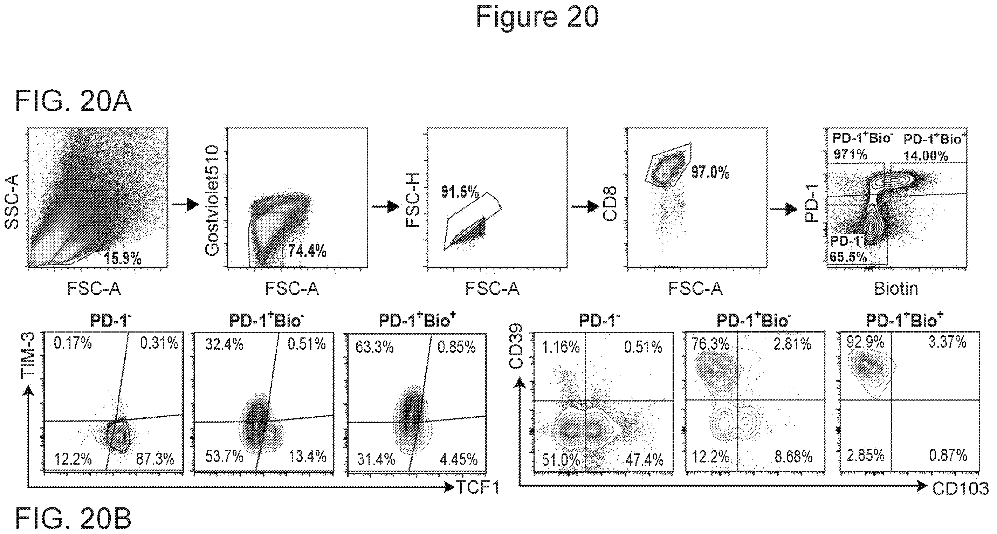

**Figure 20** shows flow cytometric analysis of three subsets of CD8+ TILs from murine MC38 tumor, related to FIG. 16G. FIG. 20A shows gating strategy for analyzing markers of PD-1+Bio+, PD-1+Bio- and PD-1- TILs. FIG. 20B shows representative flow cytometry analysis showing the staining of TIM-3, TCF1, CD39 and CD103 in the three subsets of TILs.

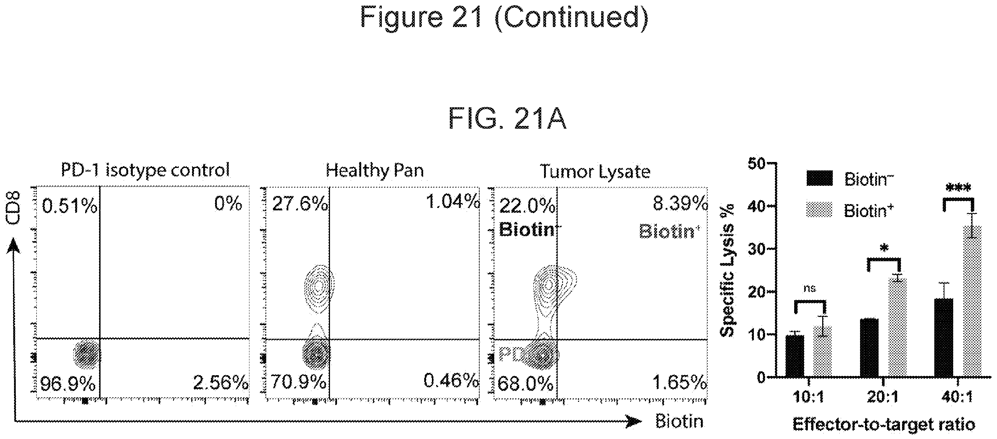

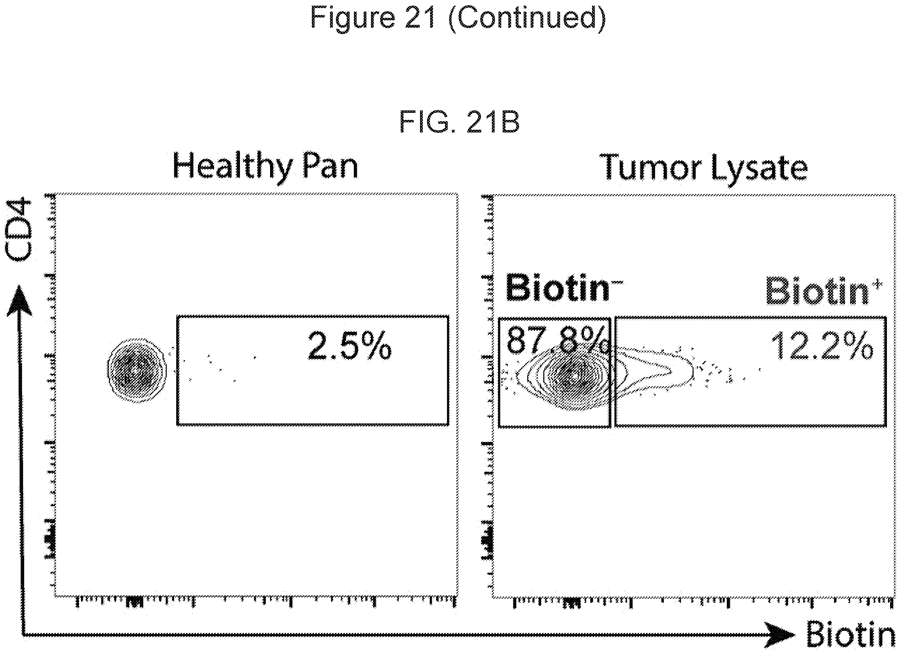

**Figure 21** shows that FT functionalized iDCs may be used as probes to detect and isolate tumor reactive T cells from infiltrating lymphocytes (TILs) in the Pan02 pancreatic cancer murine model (Corbett, et al., Cancer Res. 1984, 44:717-726). FIG. 21A (left panel) shows FACS data in which CD8+ T cells were labeled with Alexa Fluor 647-streptavidin to detect biotin tag deposited on the cells by FucoID proximity labeling. FIG. 21A (right panel) shows the results of an ex vivo tumor killing assay using cells sorted from the experiment described in FIG. 21A. Biotin+ and biotin - CD8 + T cells were sorted by FACS, cultured with IL-2 in T cell medium (IL2: 100 IU/mL) for 12 hours, then Pan02 cells (stably transduced with firefly luciferase) were co-cultured with sorted CD8+ T cells for 20 hours. Then the killing of Pan02 tumor were quantified through the luciferase activity (Bright-Glo, Promega). ns P > 0.05; ** P < 0.01; *** P < 0.001; one-way ANOVA followed by Tukey's multiple comparisons test. FIG. 21B shows FACS data in which CD4+ T cells were labeled with Alexa Fluor 647-streptavidin to detect biotin tag deposited on the cells by FucoID proximity labeling.

### DETAILED DESCRIPTION

### Definitions:

Unless defined otherwise, all technical and scientific terms used herein have the same meaning as commonly understood by one of ordinary skill in the art to which this invention belongs. Although any methods and materials similar or equivalent to those described herein can be used in the practice or testing of the present disclosure, some potential and preferred methods and materials are now described.. It is understood that the present disclosure supersedes any disclosure of a cited publication to the extent there is a contradiction (and in particular, any term definitions specifically set forth in the present application supersede any conflicting definition of that term disclosed in a cited publication).

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g., polypeptides, known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

The term "endotoxin-free" or "substantially endotoxin-free" relates generally to compositions, solvents, and/or vessels that contain at most trace amounts (e.g., amounts having no clinically adverse physiological effects to a subject) of endotoxin, and preferably undetectable amounts of endotoxin. Endotoxins are toxins associated with certain microorganisms, such as bacteria, typically gram-negative bacteria, although endotoxins may be found in gram-positive bacteria, such as *Listeria monocytogenes.* The most prevalent endotoxins are lipopolysaccharides (LPS) or lipo-oligo-saccharides (LOS) found in the outer membrane of various Gram-negative bacteria, and which represent a central pathogenic feature in the ability of these bacteria to cause disease. Small amounts of endotoxin in humans may produce fever, a lowering of the blood pressure, and activation of inflammation and coagulation, among other adverse physiological effects.

Therefore, in pharmaceutical production, it is often desirable to remove most or all traces of endotoxin from drug products and/or drug containers, because even small amounts may cause adverse effects in humans. A depyrogenation oven may be used for this purpose, as temperatures in excess of 300°C are typically required to break down most endotoxins. For instance, based on primary packaging material such as syringes or vials, the combination of a glass temperature of 250°C and a holding time of 30 minutes is often sufficient to achieve a 3 log reduction in endotoxin levels. Other methods of removing endotoxins are contemplated, including, for example, chromatography and filtration methods, as described herein and known in the art. Also included are methods of producing CAR-EC switches in and isolating them from eukaryotic cells such as mammalian cells to reduce, if not eliminate, the risk of endotoxins being present in a composition of the disclosure. Preferred are methods of producing CAR-EC switches in and isolating them from serum free cells.

Endotoxins can be detected using routine techniques known in the art. For example, the Limulus Ameobocyte Lysate assay, which utilizes blood from the horseshoe crab, is a very sensitive assay for detecting presence of endotoxin. In this test, very low levels of LPS can cause detectable coagulation of the limulus lysate due a powerful enzymatic cascade that amplifies this reaction. Endotoxins can also be quantitated by enzyme-linked immunosorbent assay (ELISA). To be substantially endotoxin-free, endotoxin levels may be less than about 0.001, 0.005, 0.01, 0.02, 0.03, 0.04, 0.05, 0.06, 0.08, 0.09, 0.1, 0.5, 1.0, 1.5, 2, 2.5, 3, 4, 5, 6, 7, 8, 9, or 10 EU/mg of protein. Typically, 1 ng lipopolysaccharide (LPS) corresponds to about 1-10 EU.

By a subject polypeptide sequence having an amino acid sequence at least, for example, 95% "identical" to a query amino acid sequence disclosed herein, it is intended that the amino acid sequence of the subject polypeptide is identical to the query sequence except that the subject polypeptide sequence may include up to five amino acid alterations per each 100 amino acids of the query amino acid sequence. In other words, to obtain a subject polypeptide having an amino acid sequence at least 95% identical to a query amino acid sequence, up to 5% of the amino acid residues in the subject sequence may be inserted, deleted, or substituted with another amino acid. These alterations as compared to the reference sequence may occur at the amino- or carboxy-terminal positions of the reference amino acid sequence or anywhere between those terminal positions, interspersed either individually among residues in the reference sequence or in one or more contiguous groups within the reference sequence. The identity of two or more sequences (e.g., amino acid sequences) can be compared to one another, or to published sequences, using the Basic Local Alignment Search Tool or "BLAST" algorithm; described in Johnson M, et al., (2008) NCBI BLAST: a better web interface. Nucleic Acids Res. 36:W5-W9. Similarly, identity can be determined between two nucleotide sequences in the same manner. Thus, to obtain a subject nucleotide sequence (e.g., RNA or DNA sequence, such as a cDNA sequence) that is at least 95% identical to a query nucleotide sequence, up to 5% of the nucleotide residues in the subject sequence may be inserted, deleted, or substituted with another nucleotide.

The term "click chemistry" or "Copper-Catalyzed Azide-Alkyne Cycloaddition reaction" or "CuACC reaction" as used herein, refers to the copper(I)-catalyzed [3+2]-Huisgen 1,3-dipolar cyclo-addition of terminal alkynes and azides leading to 1,2,3-triazoles. It may also refer to a 25 copper free variant of this reaction that might also be used. (J. M. Baskin, J. A Prescher, S. T. Laughlin, N. J. Agard, P. V. Chang, I. A Miller, A Lo, J. A Codelli, C.R. Bertozzi, Proc. Natl. Acad. Sci. U.S.A. 2007, 104, 16793.).

The terms "fucosyltransferase", "FucT" , and "FT are used interchangeably herein and refer to an enzyme (e.g., a glycosyltransferase) that catalyzes the transfer of fucose from GDP-β-L-fucose ("GDP-fucose") to an acceptor substrate. The acceptor substrate can be on an oligosaccharide and/or a protein. For example, fucosyltransferases can transfer fucose to the innermost GlcNAc (N-acetylglucosamine) residue present in an N-glycan (e.g., Type 1: Galβ1,3GlcNAc) resulting in an α-1,6-fucosylation known as "core fucosylation." Fucosyltransferases can also catalyze "terminal fucosylation" by which fucose is attached to terminal galactose residues on oligo saccharides such as Galβ1,4GlcNAc (Type II, also called LacNAc) or Galβ1,3GlcNAc (Type III). Thirteen types of fucosyltransferases have been described to-date (including FUT1-FUT11, POFUT1, and POFUT2), which catalyze the different types of fucosylation. For example, FUT1 (NM_000148.1) and FUT2 (NM_000511.1) catalyze the synthesis of α-1,2-fucosylation; FUT3 (NM_000149.1) catalyzes the synthesis of α-1,3- and α-1,4-fucosylation (α-1,3/4 fucosylation); FUT4 (NM_002033.1), FUT5 (NM_002034.1), FUT6 (NM_000150.1) FUT7 (NM_004479.1), FUT9 (NM_006581.1), FUT10 (NM_032664.2) and FUT11 (NM_173540.1) catalyze α-1,3-fucosylation; FUT8 (NM_004480.1) catalyzes α-1,6-fucosylation; and POFUT1(NM_015352.1) and POFUT2 (NM_015227.1) catalyze the addition of fucose directly to polypeptides via O-glycosidic linkage to serine and threonine residues.

The terms "peptide," "polypeptide" and "protein" are used interchangeably to refer to an isolated polymer of amino acid residues, and are not limited to a minimum length unless otherwise defined. Peptides, oligopeptides, dimers, multimers, and the like, are also composed of linearly arranged amino acids linked by peptide bonds, and whether produced biologically and isolated from the natural environment, produced using recombinant technology, or produced synthetically typically using naturally occurring amino acids. In some aspects, the polypeptide or protein is a "modified polypeptide" comprising non-naturally occurring amino acids. In some aspects, the polypeptides comprise a combination of naturally occurring and non-naturally occurring amino acids, and in some examples, the peptides comprise only non-naturally occurring amino acids. The term "peptide" as used herein encompasses native peptides ( either degradation products, synthetically synthesized peptides or recombinant peptides) and 30 peptidomimetics (typically, synthetically synthesized peptides), as well as peptoids and semipeptoids which are peptide analogs, which may have, for example, modifications rendering the peptides more stable while in a body or more capable of penetrating into cells. Such modifications include, but are not limited to, N-terminus modification, C-terminus modification, peptide bond modification, backbone modification, and/or side chain modification.

The term "polynucleotide," or "nucleotide" as used herein, refer generally to linear polymers of natural or modified nucleosides, including deoxyribonucleosides, ribonucleosides, 15 alpha-anomeric forms thereof, and the like, usually linked by phosphodiester bonds or analogs thereof ranging in size from a few monomeric units, e.g. 2-4, to several hundreds of monomeric units. When a polynucleotide is represented by a sequence of letters, such as "ATGCCTG," it will be understood that the nucleotides are in 5'->3' order from left to right. Polynucleotide as used herein also includes a basic, sugar-phosphate or sugar- phosphorothioate polymers.

The term LacNAc refers to N-acetyllactosamine and referred to interchangeably as Gal 1,4 GlcNAc.

The terms sLacNAc and sialyl LacNAc, refer to α2,3-sialylated LacNAc, also refered to herein as sialyllactosamine.

"Substantially" or "essentially" means of ample or considerable amount, quantity, size; nearly totally or completely; for instance, 95% or greater of some given quantity.

"Substantially similar" sequences are sequences comprising at least about 90% identity in sequence (e.g., amino acid or nucleotide sequence) with one another, or at least about 95%, 96%, 97%, 98%, 99% or more than about 99% identity with one another.

The term "tagged substrate" or "substrate comprising a detectable tag" as used herein refers to an enzyme substrate that comprises a tag, as described herein. In various embodiments, the tagged substrate is a substrate for an enzyme that is conjugated to, or otherwise disposed on, the surface of a bait cell. As will be clear to a person of ordinary skill in the art, in various embodiments, the exact tagged substrate that may be used in connection with a particular embodiment of the present disclosure depends on the enzyme that is conjugated to, or otherwise disposed on, the surface of the bait cell. So, e.g., if a bait cell is engineered to have a glycosyltransferase on its cell surface, then the tagged substrate will comprise a donor sugar nucleotide that is conjugated to a tag, and the donor sugar nucleotide will be one that is a suitable substrate for that enzyme. In contrast, if the bait cell comprises a sortase enzyme on its surface, then the substrate will comprise a peptide comprising a sortase recognition sequence and a tag.

### Overview:

Disclosed herein are compositions and methods for monitoring cell to cell ("cell-cell") interactions *in vitro, ex vivo,* and *in vivo.* In some examples, the present disclosure provides for the use of these compositions and methods (i) to identify and enrich for tumor-specific antigen (TSA) reactive T cells from tumor infiltrating lymphocytes (TILs) or circulating T cells; (ii) to identify and enrich for T cells that recognize autoantigens in a particular autoimmune disease, and/or (iii) to identify and enrich for antigen specific regulatory T cells that have the potential to be exploited to treat autoimmune disease.

In the past ten years, the development of immune checkpoint inhibitor and adoptive cell transfer (ACT)-based therapies have brought enormous hopes to cancer patients.^{7,17-19} Despite the great success of these two types of immunotherapy, more than 50% of cancer patients fail to respond to checkpoint blockade treatment. Successful anti-tumor immune responses following PD-1/PD-L1 blockade are believed to require re-activation and clonal-proliferation of neoantigen-specific T cells present in the tumor microenvironment. ²⁰⁻²⁴ Inadequate generation of neoantigen-specific T cells, suppression of the effector function of neoantigen-specific T cells, and impaired formation of memory T cell are major factors responsible for the failure of checkpoint inhibitor therapies. Hence, strategies that can overcome these suppressive mechanisms are highly sought after.

On the other hand, considerable efforts have been devoted to discovering actionable tumor-specific antigens (TSAs) for cancer immunotherapy. These TSAs can be used either in therapeutic cancer vaccines¹ or to identify antigen-specific tumor infiltrating lymphocytes (TILs)²⁶ and T cell receptors (TCRs) for the construction of TCR-engineered T cells (TCR-T) to be used in cancer patient treatment²⁷.

Currently, the most common strategy to identify TSAs hinges on reverse immunology, in which exome sequencing is performed on tumor cells to identify nonsynonymous mutations.²⁸ In silico tools are used to generate peptides harboring any of the identified nonsynonymous mutations. These peptides are either left unfiltered²⁹, filtered through the use of prediction algorithms for MHC-binding, or used as guides for identifying MHC-associated TSAs by combining with mass spectrometry analysis. The RNA encoding the TSA candidates or the corresponding peptides are then introduced to autologous dendritic cells (DCs) to query T cell reactivity in cultured TILs or isolated circulating T cells. Such methods have found success in identifying TSAs and TSA-reactive TCRs for melanoma patients and a small number of patients with epithelial cancers. Despite these acclaimed successes, a large portion of identified TSA candidates are not immunogenic because available computational tools cannot accurately predict T-cell reactivity.

Due to the fact that the exome constitutes only 2% of the human genome, an alternative proteogenomic technique has been developed to discover TSAs coded by potentially all genomic regions. Using this technique, TSAs derived from allegedly noncoding regions have been discovered, which would have been missed by standard exome-based approaches. However, such methods can only be practiced by a handful of highly specialized labs because "this approach is not compatible with the computation of classical false discovery rates and TSAs must undergo meticulous validation by manual inspection of MS spectra".

To accelerate translational research for developing new cancer immunotherapeutic strategies, a rapid and high throughput method for enriching and isolating TSA-reactive T cells that can be easily adopted in research laboratories and clinical settings will be a welcome advance for the field. The present disclosure provides, *inter alia,* such a strategy.

In various examples, the compositions and methods of the present disclosure provide cells that have been engineered to have an enzyme on their cell surface such that, upon contacting another cell, the enzyme on the engineered cell catalyzes a labeling reaction that attaches a label (or "tag") on the other cell. We refer to this process herein as "interaction-dependent labeling"; and we refer to the engineered cell that catalyzes the labeling reaction as a "bait cell" and the cell that is labeled by the bait cell as a "prey cell." In some examples, by using the techniques described in herein, fucosyltransferase (FT)- (or other enzyme-) modified autologous iDCs primed with antigen, e.g., tumor lysate, can be used as the bait cells that induce proximity-based transfer of biotin (or other) tags to the surface of cells that interact with the DCs, e.g., TILs or circulating T cells. Using fluorescence activated cell sorting (FACS), (for example, or other enrichment methods) the biotinylated T cells (or otherwise tagged cells) can be isolated as bona fide antigen reactive, e.g., TSA-reactive TILs. These isolated cells may also express currently used surface markers designating prospective TSA reactivity (e.g. PD-1, CD134, or CD137) or other markers such as CXCR5 and/or TIM3. In such examples, because the proximity-based biotinylation only takes place on cells that interact with antigen-presenting DCs, using this method, bystander CD8+ T cells found in human tumor infiltrates that also express PD-1, CD134, CD137, CXCR5, and/or TIM3³⁰ are effectively excluded. Thus, in various examples, the present disclosure includes compositions (including pharmaceutical composotions) \ of TSA-reactive TILs or T cells that have been isolated using the proximity labeling methods disclosed herein, as well as expanded populations of such TSA-reactive TILs or T cells. Such compositions may be used in treating or preventing diseases such as, e.g., cancer.

### I. Bait cells

In various examples, the bait cells of the present disclosure comprise an enzyme on their cell surface for catalyzing the transfer of a label (or "tag") to a target prey cell when the bait and prey cells come into contact. Suitable enzymes for use on the surface of a bait cell are disclosed herein, and include without limitation glycosyltransferases (e.g., fucosyltransferases and sialyltransferases), sortases, and promiscuous biotin ligases.

*Bait cell types.* Any cell that is capable of contacting another cell may be used as the bait cell, provided that it may be engineered to have a suitable enzyme on its surface for catalyzing contact-induced interaction-dependent labeling of a contacted prey cell. In some examples, the bait cell is an antigen presenting cell (APC). In some instances the bait cell is a professional APC. In some instances the bait cell is a nonprofessional APC. In some instances the bait cell expresses an MHC class I molecule. In some instances the bait cells expresses an MHC class II molecule. In some instances the bait cell is a leukocyte. In some instances, the bait cell is an atypical APC. In some instances the bait cell is a cell selected from a DC, a macrophage, a B cell, a granulocyte, a mast cell, a neutrophil, an endothelial cell, an epithelial cell. In some examples, the bait cell is an engineered APC, e.g., an artificial APC ("aAPC"). Such aAPCs are known in the art. The aAPC may be a cell-based aAPC or a non-cell-based aAPC. In some instances, the non-cell-based aAPC comprises signal 1 and signal 2 and optionally signal 3 on a nanoparticle or microparticle aAPCs include two signals: a major histocompatibility complex (MHC) signal and a co-stimulatory molecule. In some instances, the MHC signal in the aAPC is MHC class I. In some instances, the MHC signal in the aAPC is MHC class II. In some instances, the co-stimulatory signal in the aAPC is generated by CD80 (B7.1) or CD86 (B7.2). The aAPC may also comprise a third signal that stimulates cytokine secretion to promote T cell stimulation and expansion. In some instances, the aAPC comprises a third signal that stimulates IL-2 secretion after the aAPC binds to a T cell. In some instances the aAPC is a fibroblast engineered to express the first signal and the second signal and optionally the third signal. The fibroblast may also express ICAM-1 and / or LFA-3. For example, in certain examples, the bait cell is a dendritic cell and the prey cell is an effector cell. The dendritic cell may be an immature dendritic cell. The dendritic cell may be primed with an antigen. In some examples, the antigen is from a cancer, a pathogenic infection, an autoimmune disease, an inflammatory disease, or a genetic disorder. In some examples, effector cells used as prey cells in the present disclosure in combination with bait cells that are dendritic cells include effector cells selected from a naive T cell, a memory stem cell T cell, a central memory T cell, an effector memory T cell, a helper T cell, a CD4+ T cell, a CD8+ T cell, a CD8/CD4+ T cell, an αβ T cell, a γδ T cell, a cytotoxic T cell, a natural killer T cell, a natural killer cell, and a macrophage. In such methods, autologous immature DCs primed with tumor lysates may in some examples be modified with an enzyme that induces proximity-based transfer of tags (e.g., biotin) to the surface of prey cells (i.e. TILs or circulating T cells) that interact with the bait cell DCs. Using fluorescence activated cell sorting (FACS) (or other suitable isolation methods), the tagged (e.g., biotinylated) T cells that may also express currently used surface markers designating prospective TSA reactivity (e.g. PD-1, CD134, or CD137)²⁸⁻³⁰ or other markers such as CXCR5 and/or TIM3 may be isolated. The isolated T cells are highly enriched for antigen reactive T cells, e.g., tumor-reactive T cells with reactivity directed toward TSAs. Similarly, DCs primed with tissue lysates from autoimmune patient biopsies may be modified with an enzyme that induces proximity-based transfer of tags (e.g., biotin) to the surface of prey cells (circulating autoreactive T cells) that interact with the bait cell DCs. From this assay, tagged (e.g., biotin+) CD8+ T cells that are prospective auto-reactive T cells, and tagged (e.g., biotin+) CD4+, CD25+, FOXP3+ T cells that are prospective antigen specific regulatory T cells may be isolated.

In some examples, the bait cell is a B cell. In some examples, the bait cell is a B cell and the prey cell is a T cell. In some examples, the bait cell is a naive B cell or a germinal center B cell.

Similarly, in some examples, the bait cell is such an effector cell (e.g., a naive T cell, a memory stem cell T cell, a central memory T cell, an effector memory T cell, a helper T cell, a CD4+ T cell, a CD8+ T cell, a CD8/CD4+ T cell, an αβ T cell, a γδ T cell, a cytotoxic T cell, a natural killer T cell, a natural killer cell, or a macrophage) and the effector cell is, thus, engineered to have a suitable enzyme on its surface for catalyzing contact-induced interaction-dependent labeling of a contacted prey cell. In some examples, the bait cell is a T cell expressing a suitable enzyme on its surface for catalyzing contact-induced interaction-dependent labeling of a contacted prey cell and the prey cell is a B cell. In some examples, the bait cell is a T cell expressing a suitable enzyme on its surface for catalyzing contact-induced interaction-dependent labeling of a contacted prey cell and the prey cell is a naive B cell a germinal center B cell. In some examples, the bait cell is a T cell expressing a suitable enzyme on its surface for catalyzing contact-induced interaction-dependent labeling of a contacted prey cell and the prey cell is a dendritic cell. In certain examples of the present disclosure, the bait cell is a B cell expressing a suitable enzyme on its surface for catalyzing contact-induced interaction-dependent labeling of a contacted prey cell and the prey cell is a CD4+ T cell.

### Bait cell enzymes

Any enzyme capable of catalyzing contact-induced interaction-dependent labeling of a contacted prey cell utilizing a tagged substrate may be disposed on the surface of a bait cell and utilized in the present disclosure. Such an enzyme is referred to herein as a "suitable enzyme." In some examples, suitable enzymes for use on a bait cell to facilitate interaction-dependent labeling in accordance with the present disclosure (i) possess high Km toward the acceptor substrate found on the surface of the prey cells such that background labeling is minimal; and (ii) possess high kcat to trigger interaction-dependent labeling when an interaction between bait and prey cells takes place. The enzyme may be a human enzyme. The enzyme may be a non-human enzyme. The enzyme may be a recombinant enzyme. The enzyme may be an isolated enzyme. The enzyme may be a native enzyme. The enzyme may be a non-native enzyme. The enzyme may comprise a tag (e.g., an epitope tag). Such tags are well known in the art and may be used in the present disclosure including, without limitation, any tag described herein.

In some instances, the enzyme on the surface of the bait cell is a transferase. In general, transferases catalyze the transfer of one molecular group from one molecule to another. For instance, such molecular groups include phosphate, amino, methyl, acetyl, acyl, phosphatidyl, phosphoribosyl, among other groups, and suitable transferases for use in the present disclosure include any transferase that is capable of catalyzing the transfer of one molecule to another, even if the molecule has been labeled with a tag (e.g., a tag known in the art or described herein).

For example, in particular examples, the present disclosure provides bait cells having an enzyme on their surface that is a glycosyltransferase. Glycosyltransferases catalyze the transfer of sugar nucleotide donors to acceptor molecules, which may include, e.g., proteins and other sugar moieties (glycans), e.g., on glycoproteins, glycolipids, oligosaccharides, etc.

In certain examples, the present disclosure provides bait cells having a fucosyltransferase or a sialyltransferase on their surface.

Fucosyltransferases catalyze the transfer of fucose from GDP-Fuc to Gal in a α1,2-linkage and to GlcNAc in a α1,3-, α1,4-, or α1,6-linkage. Since known fucosyltransferases utilize the same nucleotide sugar, it is believed that their specificity resides in the recognition of the acceptor and in the type of linkage formed. On the basis of protein sequence similarities, these enzymes have been classified into four distinct families: (1) the alpha-2-fucosyltransferases, (2) the alpha-3-fucosyltransferases, (3) the mammalian alpha-6-fucosyltransferases, and (4) the bacterial alpha-6-fucosyltransferases. Conserved structural features, as well as a consensus peptide motif have been identified in the catalytic domains of all alpha-2 and alpha-6-fucosyltranferases, from prokaryotic and eukaryotic origin. Based on these sequence similarities, alpha-2 and alpha-6-fucosyltranferases have been grouped into one superfamily. In addition, a few amino acids were found strictly conserved in this superfamily, and two of these residues have been reported to be essential for enzyme activity for a human alpha-2-fucosyltransferase. The alpha-3-fucosyltransferases constitute a distinct family as they lack the consensus peptide, but some regions display similarities with the alpha-2 and alpha-6-fucosyltranferases. All these observations strongly suggest that the fucosyltransferases share some common structural and/or catalytic features. In humans, at least 11 fucosyltransferases have been described, and these are encoded by human genes FUT1; FUT2; FUT3; FUT4; FUT5; FUT6; FUT7; FUTS; FUT9; FUT10; and FUT11.

Sialyltransferases are glycosyltransferases responsible for the terminal sialylation of carbohydrate groups of glycoproteins, glycolipids and oligo saccharides which contain a conserved region of homology in the catalytic domain. Members of the sialyltransferase gene family comprise Gal/31,3GalNAc α2,3 sialyltransferase and Gall,3(4)GlcNAc α2,3 sialyltransferase. Sialylation refers to the transfer of sialic acid to a terminal position on sugar chains of glycoproteins, glycolipids, oligosaccharides and the like. Examples of enzymatically functional sialyltransferases are those capable of transferring sialic acid from CMP-sialic acid to an acceptor oligosaccharide, where the oligosaccharide acceptor varies depending upon the particular sialyltransferase. Numerous sialyltransferases are known in the art and include, without limitation human sialyltransferases SIAT4C; SIAT9; ST3GAL1; ST3GAL2; ST3GAL3; ST3GAL4; ST3GAL5; ST3GAL6; ST3GalIII; ST6GAL1; ST6GAL2; ST6Gal; ST8SIA1; ST8SIA2; ST8SIA3; ST8SIA4; ST8SIA5; ST8SIA6; and ST8Sia.

Typically, glycosyltransferases have stringent donor substrate specificities; however the present inventors have discovered and recently reported that *H. pylori* α1,3fucosyltransferase has remarkable substrate tolerance (PCT/US2018/016503, published as WO2018/144769) and will essentially permit anything desirable to be conjugated, e.g., via a linker, to its GDP-Fucose substrate and still be capable of catalyzing fucosylation reaction. The present inventors have similarly shown in their prior work that ST6Gal1; *Pasteurella multocida* α(2,3) sialyltransferase M144D mutant (Pm2,3ST-M144D); and Photobacterium damsel α(2,6) sialyltransferase (Pd2,6ST)) are permissive to functionalized CMP-sialic acid donor substrates. *Id.* and Hong, S, et al., Bacterial glycosyltransferase-mediated cell-surface chemoenzymatic glycan modification. Nature Communications 10, Article number: 1799 (2019). Additionally, we show herein that *H. pylori* α1,3/1,4 fucosyltransferase; human α1,3 fucosyltransferase (FUT6); and human α2,6αsialyltransferase (ST6Gal1), are also similarly permissive to conjugated donor substrates; as is the human α1,3 fucosyltransferase FUT9 (data not shown).

Thus, some examples of the present invention provide bait cells comprising a fucosyltransferase or sialyltransferase disposed on its surface and methods of using such bait cells to achieve interaction-dependent labeling, thereby detecting whether the bait cells have contacted another cell. In certain aspects, if a bait cell bearing a fucosyltransferase or a sialyltransferase disposed on its surface comes into contact with a prey cell comprising a suitable glycan acceptor moiety in the presence of a tagged conjugate of the appropriate donor sugar nucleotide for the respective glycosyltransferases (i.e., GDP-fucose for fucosyltransferases and CMP-Neu5Ac for the sialyltransferases), then the enzyme on the bait cell will catalyze a glycosyltransferase reaction that results in the attachment of the respective tagged donor sugar nucleotide to the prey cell; thus resulting in a lasting indicator that a cell-cell interaction has occurred between the bait and prey cells.

In one embodiment, the present disclosure provides bait cells comprising a human fucosyltransferase enzyme on their surface, and methods of using the same in interaction-dependent labeling a prey cell. In one embodiment, the human fucosyltransferase enzyme is human α 1,3-fucosyltransferase. In one embodiment, the human α 1,3 -fucosyltransferase is recombinantly prepared. In one embodiment, the present disclosure provides bait cells comprising an *H. pylori* fucosyltransferase enzyme on their surface. In one embodiment, the *H. pylori* fucosyltransferase is *H. pylori* α 1,3-fucosyltransferase. In one embodiment, the *H. pylori* fucosyltransferase is *H. pylori* α 1,3/1,4 -fucosyltransferase.

In one embodiment, the present disclosure provides bait cells comprising a human sialyltransferase enzyme on their surface, and methods of using the same in interaction-dependent labeling a prey cell. In one embodiment, the human sialyltransferase is ST6GAL1. In one embodiment, the human sialyltransferase is ST6GalNAc1. In one embodiment of the present disclosure, the enzyme on the surface of the bait cell is a non-human sialyltransferase. In one embodiment the non-human sialyltransferase is *Pasteurella multocida* α (2,3) sialyltransferase M144D mutant (Pm2,3ST-M144D) or *Photobacterium damsela* α (2,6) sialyltransferase (Pd2,6ST).

In one example, the present disclosure provides bait cells comprising a sortase enzyme on their surface, and methods of using the same in interaction-dependent labeling a prey cell. Sortases are a family of enzymes capable of carrying out a transpeptidation reaction conjugating the C-terminus of a first protein to the N-terminus of second protein via transamidation. If a sortase enzyme comprised on the surface of a bait cell comes into contact with a prey cell having on its surface a polypeptide comprising a sortase acceptor peptide (e.g., a GGG residue) at its N-terminus in the presence of a tagged peptide comprising a sortase recognition sequence (e.g., LPTXG (SEQ ID NO: 28), wherein X is any amino acid for sortase A), then the sortase will catalyze the attachment of the tagged peptide to the N-terminus of the polypeptide comprising the sortase recognition sequence; thereby attaching the tag to the prey cell. Any sortase known in the art or disclosed herein may be utilized in connection with the present disclosure, as a suitable enzyme for conjugation to the surface of a bait cell for the purpose of facilitating interaction-dependent labeling of a prey cell as described herein.

Sortases are also referred to as transamidases, and typically exhibit both a protease and a transpeptidation activity. Various sortases from prokaryotic organisms have been identified. For example, some sortases from Gram-positive bacteria cleave and translocate proteins to proteoglycan moieties in intact cell walls. Among the sortases that have been isolated from Staphylococcus aureus, are sortase A (Srt A) and sortase B (Srt B). Thus, in certain examples, a transamidase used in accordance with the interaction-dependent labeling methods described herein is a sortase A, e.g., from S. aureus, also referred to herein as SrtAaureus. In other examples, a transamidase is a sortase B, e.g., from S. aureus, also referred to herein as SrtBaureus.

Sortases have been classified into four classes, designated A, B, C, and D (i.e., sortase A, sortase B, sortase C, and sortase D, respectively) based on sequence alignment and phylogenetic analysis of 61 sortases from Gram-positive bacterial genomes (Dramsi et al., Res Microbiol. 156(3):289-97, 2005). These classes correspond to the following subfamilies, into which sortases have also been classified by Comfort and Clubb (Comfort et al., Infect Immun., 72(5):2710-22, 2004): Class A (Subfamily 1), Class B (Subfamily 2), Class C (Subfamily 3), and Class D (Subfamilies 4 and 5). The aforementioned references disclose numerous sortases and their recognition motifs. See also Pallen et al., TRENDS in Microbiology, 2001, 9(3), 97-101). Those skilled in the art will readily be able to assign a sortase to the correct class based on its sequence and/or other characteristics such as those described in Drami, et al., supra.

The term "sortase A" is used herein to refer to a class A sortase, usually named SrtA in any particular bacterial species, e.g., SrtA from S. aureus. Likewise "sortase B" is used herein to refer to a class B sortase, usually named SrtB in any particular bacterial species, e.g., SrtB from S. aureus. The present disclosure encompasses examples relating to any of the sortase classes known in the art (e.g., a sortase A from any bacterial species or strain, a sortase B from any bacterial species or strain, a class C sortase from any bacterial species or strain, and a class D sortase from any bacterial species or strain).

In some examples, the sortase used in the interaction-dependent labeling methods described herein is a wild-type enzyme. In other examples, the sortase is a modified version which may possess a superior feature as compared to the wild-type counterpart (e.g., higher catalytic activity). In some examples, the sortase can be a mutant of SrtA, which may comprise one or more of the following positions: P94, S102, A104, E105, K138, K152, D160, K162, T164, D165, K173, I182, K190, and K196. For example, a SrtA mutant may comprise one or more of the following mutations: P94R or P94S, S 102C, A104H, E105D, K138P, K152I, D160K or D160N, K162H, T164N, D165A, K173E, I182V, K190E, and K196S or K196T. In one example, the sortase is a triple mutant P94S/D160N/K196T of SrtA from S. aureus.

In other examples, modified sortase having altered substrate specificity can be used in the intercellular labeling methods described herein. For example, sortase A mutants having one or more mutations at positions S102 (e.g., S102C), A104 (e.g., A104H or A104V), E105 (e.g., E105D), K138 (e.g., K138P), K152 (e.g., K152I), N162 (e.g., N162N), T164 (e.g., T164N), K173 (e.g., K173E), I182 (e.g., I182V), T196 (e.g., T196S), N98 (e.g., N98D), A118 (e.g., A118T), F122 (e.g., F122A), K134 (e.g., K134R), F144 (e.g., F144L), and E189 (e.g., E189F). Such a modified sortase may recognize sequences such as LAXTG (SEQ ID NO: 6) and/or LPXSG (SEQ ID NO: 7), in which X can be any amino acid residue. Examples include mutant S102C/A104H/E105D/K138P/K152I/N162N/T164N/K173E/1182V/T196S, and mutant N98D/A104V/A118T/F122A/K134R/F144L/E189F. Additional sortase mutants having altered substrate specificity are disclosed in US20140057317 and Dorr et al., PNAS 111 (37):13343-13348 (2014).

A modified version of a wild-type sortase may share at least 85% (e.g., 90%, 95%, 98%, or above) sequence identity to the wild-type counterpart. It may contain mutations at one or more positions corresponding to those described above, which can be identified by analyzing the amino acid sequence of a wild-type sortase with the amino acid sequence of a SrtA. The "percent identity" of two amino acid sequences can be determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of the disclosure. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (e.g., XBLAST and NBLAST) can be used.

In some examples, the interaction-dependent labeling methods can use an active fragment of a sortase. Such a fragment of a specific sortase can be identified based on knowledge in the art or by comparing the amino acid sequence of that sortase with a sortase having known structure/function correlation (e.g., active domain being identified). In some examples, the sortase used herein can be an active fragment of a sortase A such as SrtA from S. aureus, e.g., a sortase A fragment lacking the N-terminal 59 or 60 amino acid residues, or a functional variants thereof, which may contain one or more of the mutations described herein.

Amino acid sequences of Srt A and Srt B and the nucleotide sequences that encode them are known to those of skill in the art and are disclosed in a number of references cited herein. See, e.g., GenBank accession numbers NP_375640 and YP_043193. The amino acid sequences of S. aureus SrtA and SrtB are homologous, sharing, for example, 22% sequence identity and 37% sequence similarity. The amino acid sequence of a sortase-transamidase from Staphylococcus aureus also has substantial homology with sequences of enzymes from other Gram-positive bacteria, and such transamidases can be utilized in the ligation processes described herein. For example, for SrtA there is about a 31% sequence identity (and about 44% sequence similarity) with best alignment over the entire sequenced region of the S. pyogenes open reading frame. There is about a 28% sequence identity with best alignment over the entire sequenced region of the A. naeslundii open reading frame. It will be appreciated that different bacterial strains may exhibit differences in sequence of a particular polypeptide, and the sequences herein are exemplary.

In certain examples a transamidase bearing 18% or more sequence identity, 20% or more sequence identity, or 30% or more sequence identity with an S. pyogenes, A. naeslundii, S. mutans, E. faecalis or B. subtilis open reading frame encoding a sortase can be screened, and enzymes having transamidase activity comparable to Srt A or Srt B from S. aureus can be utilized (e.g., comparable activity sometimes is 10% of Srt A or Srt B activity or more).

In some examples, the interaction-dependent labeling methods described herein use a sortase A (SrtA) or an active fragment thereof. SrtA recognizes the motif LPXTX (SEQ ID NO: 4); wherein each occurrence of X represents independently any amino acid residue), with common recognition motifs being, e.g., LPKTG (SEQ ID NO: 8), LPATG (SEQ ID NO: 9), or LPNTG (SEQ ID NO: 10). In some examples LPETG (SEQ ID NO: 5) is used as the sortase recognition motif. However, motifs falling outside this consensus may also be recognized. For example, in some examples the motif comprises an 'A' rather than a 'T' at position 4, e.g., LPXAG (SEQ ID NO: 11), or LPNAG (SEQ ID NO: 12). In some examples the motif comprises an 'A' rather than a 'G' at position 5, e.g., LPXTA (SEQ ID NO: 13), or LPNTA (SEQ ID NO: 14). In some examples the motif comprises a 'G' rather than 'P' at position 2, e.g., LGXTG (SEQ ID NO: 15) or LGATG (SEQ ID NO: 16). In some examples the motif comprises an 'I' rather than 'L' at position 1, e.g., IPXTG (SEQ ID NO: 17), IPNTG (SEQ ID NO: 18) or IPETG (SEQ ID NO: 19). Additional suitable sortase recognition motifs will be apparent to those of skill in the art, and the disclosure is not limited in this respect. It will be appreciated that the terms "recognition motif" and "recognition sequence", with respect to sequences recognized by a transamidase or sortase, are used interchangeably. In some examples, the SrtA is a mutant as described herein, which may possess improved enzymatic activity relative to the wild-type counterpart. Such a mutant may recognize LAETG (SEQ ID NO: 20) and use a peptide comprising the recognition sequence as a substrate. Such sortase recognition motifs can be used in any of the methods described herein.

In some examples of the disclosure the sortase is a sortase B (SrtB) or an active fragment thereof, e.g., a sortase B of S. aureus, B. anthracis, or L. monocytogenes. Motifs recognized by sortases of the B class (SrtB) often fall within the consensus sequences NPXTX, e.g., NP[Q/K]-[T/s]-[N/G/s] (SEQ ID NO: 21), such as NPQTN (SEQ ID NO: 22) or NPKTG (SEQ ID NO: 23). For example, sortase B of S. aureus or B. anthracis cleaves the NPQTN (SEQ ID NO: 22) or NPKTG (SEQ ID NO: 23) motif of IsdC in the respective bacteria (see, e.g., Marraffini et al., Journal of Bacteriology, 189(17): 6425-6436, 2007). Other recognition motifs found in putative substrates of class B sortases are NSKTA (SEQ ID NO: 24), NPQTG (SEQ ID NO: 25), NAKTN (SEQ ID NO: 26), and NPQSS (SEQ ID NO: 27). For example, SrtB from L. monocytogenes recognizes certain motifs lacking P at position 2 and/or lacking Q or K at position 3, such as NAKTN (SEQ ID NO: 26) and NPQSS (SEQ ID NO: 27) (Mariscotti et al., J Biol Chem. 2009 Jan. 7). Such sortase recognition motifs can also be used in any of the methods described herein.

In one embodiment, the sortase enzyme is selected from a sortase A, a sortase B, a sortase C, or a sortase D, or an active fragment thereof.

The sortase acceptor peptide may comprise sortase recognition sequence (e.g., LPTXG (SEQ ID NO: 28) for sortase A in which X is any amino acid residue), wherein the peptide is associated with a detectable label or tag, e.g., biotin or a fluorescent dye.

In some examples, the sortase disposed on the surface of the bait cell is a mutant sortase (e.g., a mutant sortase A) that exhibits improved catalytic activity as compared to its wild-type counterpart. In some examples, the mutant sortase A (SrtA) comprises one or more mutations of P94R or P94S, S102C, A104H, E105D, K138P, K152I, D160K or D160N, K162H, T164N, D165A, K173E, I182V, K190E, and K196S or K196T. In one example, the mutant SrtA includes mutations P94S, D160N, and K196T.

In one embodiment, the sortase enzyme is selected from sortase A: (5M) and mgSrtA.

In some particular examples, a bate cell is engineered to comprise on its cell surface a sortase enzyme described above. In one particular embodiment, the bate cell is engineered to comprise the sortase enzyme described above on its surface by means of a glycoconjugation method as described as Method 1 in Example 7, whereby the GDP-Fuc-Enzyme conjugate is a GDP-Fuc-Sortase enzyme conjugate, which is used as the donor nucleotide substrate to conjugate the sortase enzyme onto the surface of the cell. In one particular embodiment, the bate cell is engineered to comprise the sortase enzyme described above on its surface by means of a glycoconjugation method as described as Method 1 in Example 7, except that instead of utilizing a fucosyltransferase to attach a GDP-Fuc-Sortase enzyme conjugate, the method utilizes a sialyltransferase enzyme according and a CMP-NeuAc-Sortase enzyme conjugate to attach the sortase onto the surface of the cell via a sialyation reaction.

In some particular examples, a sortase described above is chemically conjugated to the surface of a bait cell, e.g., according to Method 2 in Example 7.

In some particular examples, the sortase is disposed on the surface of the bait cell via a method that does not comprise expressing the sortase enzyme via genetic modification of the bait cell.

In one embodiment, the present disclosure provides bait cells comprising an enzyme on their surface, wherein the enzyme is a promiscuous biotin ligase selected from TurboID, miniTurbo, BioID, and BioID2; and methods of using the same in interaction-dependent labeling a prey cell. In the case of the promiscuous biotin ligases, interaction-dependent labeling occurs when bait cells contacting prey cells with vicinal proteins on their surface in the presence of biotin; in which case the promiscuous biotin ligase will transfer biotin to the vicinal proteins.

***Acceptor molecules.*** As will be clear to a person of ordinary skill in the art, the nature of the acceptor molecule on the prey cell and the donor sugar nucleotide-tag-conjugate or other tagged substrate that is attached to the prey cell by the bait cell is in various examples driven by the enzyme that is disposed on the bait cell. For example, in various examples, when the bait cell comprises a fucosyltransferase on its cell surface, the acceptor molecule on the prey cell is a fucose acceptor capable of being fucosylated by the fucosyltransferase, and the donor sugar nucleotide-tag conjugate is a GDP-fucose conjugate comprising a tag. Such fucose acceptors are known in the art and include LacNAc and α2,3-sialylated LacNAc (sLacNAc), which are commonly found in complex and hybrid N-glycans decorating most cell surfaces. Similarly, when the bait cell comprises a sialyltransferase on its cell surface, the acceptor molecule on the prey cell is a sialic acid (NeuAc)-acceptor capable of being sialylated by the sialyltransferase, and the donor sugar nucleotide-tag conjugate is a CMP-Neu5Ac conjugate comprising a tag. Such NeuAc acceptors are known in the art and include Galactose and N-acetylgalactosamine GalNAc.

### II. Tagged Substrates

In various examples, the compositions and methods disclosed herein utilize donor nucleotide sugar substrates that are tagged; tagged sortase acceptor peptides comprising sortase recognition sequences; or other tagged substrates. Any suitable tag may be conjugated to such substrates to enable detection of a proximity label transfer. Such tags may include, without limitation, mono- or poly-histidine sequences (e.g.. 6xHis), FLAG-tag, myc-tag, HA-tag, V5, VSVG, GFP (and variants thereof), horseradish peroxidase (HRP); alkaline phosphatase (AP), glucose oxidase, maltose binding protein; SUMO tag, thioredoxin, poly(NANP), poly-Arg, calmodulin binding protein, PurF fragment, ketosteroid isomerase, PaP3.30, TAF12 histone fold domain, FKBP-tag, SNAP tag, Halo-tag, immunoglobulin Fc portions (and variants thereof), biotin, streptavidin, avidin, calmodulin, S-tag, SBP, CBP, softag 1, softag 3, Xpress, isopeptag, spytag, BCCP, glutathione-S-transferase (GST), maltose binding protein (MBP), Nus, thioredocin, NANP, TC, Ty, GCN4, fluorescent molecules or probes (e.g., Alexa Fluors; fluoresceins such as, e.g., FAM), fluorescent probe Cy2, Cy3, Cy3B, Cy3.5, Cy5, Cy5.5, Cy7 (or other Cyanine dyes), a peridinin chlorophyll protein complex, a fluorescent protein (e.g., a green fluorescent protein (GFP) or red fluorescent protein (RFP)), phycoerythrin (PE); and the like. If desired, the tag may be cleavable and, thus, able to be removed, e.g., by a protease. In some examples, this is achieved by including a protease cleavage site in the tag, e.g., adjacent or linked to a functional portion of the tag. Non-limiting examples of protease tags that may be used in this manner include thrombin, TEV protease, Factor Xa, and PreScission protease. In particular examples, the tag is biotin. Additionally, in some examples, the substrate and the tag are one and the same. For example, if the enzyme that is disposed on the surface of the bait cell is a promiscuous biotin ligase, then the substrate is biotin. As noted above, biotin is a suitable tag. Thus, no conjugation of a tag to the biotin substrate is needed to facilitate detection of the presence of the substrate on the prey cell after a contact-induced conjugation of the tagged substrate onto the prey cell.

### III. Methods for Conjugating Bait cells with an enzyme

The bait cells of the present disclosure may be engineered to have the enzyme on their surface via any suitable method. In certain examples, the bait cells comprise an enzyme bound to their cell surface via conjugation. The conjugation may be via any suitable method. For example, in some examples, the conjugation is via chemical or enzymatic conjugation. Such conjugations methods are known in the art and disclosed herein. In some examples, the enzyme is expressed on the surface of the bait cell via genetic modification.

### Enzymatic conjugation

For example, in certain examples, the bait cells comprise an enzyme bound to their cell surface via a glycosylation-based conjugation method such as, e.g., we have previously described in international application PCT/US2018/016503 (published as WO2018/144769). Suitable glycosylation-based conjugations include the methods disclosed herein in the present Examples and illustrated in the present Figures (see, e.g., FIG. 1B and FIG. 2). *H. pylori* α1,3fucosyltransferase has remarkable substrate tolerance, and essentially anything desirable may be conjugated, e.g., via a linker, to a GDP-Fucose and still be utilized by the enzyme in a glycosylation reaction. Thus, in some examples, the enzyme may be conjugated to the surface of a bait cell by a method comprising the following steps (*see*, *e.g.,* Example 7, Method 1): First, an enzyme is linked with a "clickable" group such as tetrazine, azide or alkyne by amine-coupling or site-specific modification (such as aldehyde tag or unnatural amino acid modification). Then the enzyme is further linked with easily accessible GDP-Fucose derivatives bearing complementary "clickable" groups to form GDP-Fuc-Enzyme via click chemistry. Finally, the GDP-Fuc-Enzyme is transferred onto a cell surface catalyzed by *H. pylori* α1,3fucosyltransferase, which glycosylates glyco-acceptors on the surface of the cell such a LacNAc/sialylLacNAc glycans. Similarly, in some examples, other fucosyltransferases may be used to catalyze the transfer of GDP-Fuc-Enzyme onto the surface of the bait cell. For example, in certain examples, *Helicobacter mustelae* α1-2-fucosyltransferase (Hm1,2FT), H. pylori α1,3/1,4 fucosyltransferase; or human α1,3 fucosyltransferase (FUT6) is used to catalyze the transfer of GDP-FUC-Enzyme onto the surface of a bait cell. Similarly, in certain examples, a sialyltransferase is used to catalyze the transfer CMP-NeuAc-Enzyme onto the surface of a bait cell. For example, in certain examples, ST6GAL1; ST6Ga1NAc1; ST3Gal1; *Pasteurella multocida* α (2,3) sialyltransferase M144D mutant (Pm2,3ST-M144D); or *Photobacterium damsela* α (2,6) sialyltransferase (Pd2,6ST) is used to catalyze the transfer of CMP-NeuAc-Enzyme onto the surface of a bait cell.

### Chemical conjugation

In some examples, the enzyme is conjugated to the surface of a bait cell by a chemical conjugation method. In certain examples, the enzyme is chemically conjugated to the surface of the bait cell comprising the steps set forth in Example 7, Method 2: first enzyme is linked with tetrazine by amine-coupling to form enzyme-tetrazine conjugates (Enzyme-Tz). Next, the cells that are to be conjugated with the enzyme are treated with TCO-NHS ester to introduce TCO moieties onto their cell surfaces. Finally, the enzyme-Tz conjugates are reacted with the TCO-NHS moieties on the cell surfaces by biorthogonal reaction to form cell-enzyme surface conjugates.

### Genetic expression

In some examples, the enzyme is expressed on the surface of the bait cell via genetic modification. Methods for genetically altering a cell are well known in the art, and any suitable method may be used to engineer a bait cell of the present disclosure. In some examples, the enzyme is expressed on the surface of the bait cell using standard recombinant techniques in molecular biology that are well known, (e.g., see, Joseph Sambrook, et al., Molecular Cloning: A Laboratory Manual, 2nd ed., 1.53 [Cold Spring Harbor Laboratory Press 1989]). The methodology is not limited to any particular cloning strategy. A person of ordinary skill in the art will fully appreciate that one may use any variety of cloning strategies to produce an expression vector containing an enzyme for expression on a bait cell in accordance with the present disclosure. For example, one or more polynucleotides encoding the enzyme may be cloned into an expression vector along with suitable vector elements to drive expression of the enzyme onto the surface of the bait cell. In some instances, once the nucleotide sequence for the enzyme has been determined (optionally the 'codon optimized' sequence) the gene (or "polynucleotide," interchangeably) is synthesized de novo to form a cDNA that contains the gene plus additional nucleotide sequences containing unique restriction enzyme cleavage sites on the 5' and 3' ends of the gene. Several direct gene synthesis methods can be employed for this purpose, these methods are well known to those in the art (Montague MG, Lartigue C, Vashee S. (2012) Synthetic genomics: potential and limitations. Curr. Opin. Biotechnol. 23(5):659-665). Polynucleotides representing the entire enzyme gene, plus the aforementioned additional nucleotide sequences, can be directly synthesized, or alternatively subfragments of the gene may be directly synthesized followed by ligation of these fragments using PCR primers to create the full-length gene. Following gene synthesis the nucleotide sequence of the novel gene with the flanking restriction sites, and optional regulatory elements, may be confirmed by direct gene sequencing of the cDNA that are well known to those skilled in the art (Pettersson E, Lundeberg J, Ahmadian A.

(2009) Generations of sequencing technologies. Genomics 93:(2)105-111). Once the cDNA sequence has been confirmed the cDNA is cloned into a recombinant protein expression vector using standard recombinant techniques in molecular biology. Expression vectors are typically selected to match the particular host cell used for expressing the recombinant protein in order to optimize the quantity and quality of recombinant protein expressed. Expression vectors are typically engineered with nucleotide sequences that represent additional elements needed to optimize the expression of the novel gene in a particular host cell, including but not limited to, cloning sites to facilitate insertion of the cDNA containing the novel gene, a promoter/enhancer element to allow efficient, high-level gene expression, primer sites to allow sequencing of the cDNA insert, a polyadenylation signal to allow efficient transcription termination and polyadenylation of the novel gene's mRNA, and selection genes to allow for selection of transformants in bacterial and mammalian cells. The expression vector may be a eukaryotic expression vector. The expression vector may be a mammalian expression vector. The expression vector may be a viral expression vector. The expression vector may be a lentiviral expression vector. The methods may further comprise validating the cloning of the one or more polynucleotides encoding the enzyme into the expression vector comprising sequencing the expression vector, running gel electrophoresis of the vector and/or viewing the enzyme on an SDS page gel. The methods may further comprise amplifying a polynucleotide encoding the enzyme and cloning the enzyme into the expression vector. Amplifying the polynucleotide encoding the enzyme may comprise synthesizing oligonucleotides at least partially complementary to the gene. The oligonucleotides may be sufficiently complementary to the gene to anneal to the polynucleotide. The oligonucleotides may comprise linker sequences. Many suitable linkers are known in the art and are suitable for use in the present disclosure.

The methods may comprise transfecting or infecting a cell with the expression vector. The methods may further comprise expressing the enzyme in the cell. The methods may further comprise expressing the enzyme in a cell free system. The methods may further comprise producing a virus comprising the expression vector. The methods may further comprise propagating the virus. The methods may further comprise infecting a cell with the virus comprising the expression vector. The methods may further comprise propagating the cell.

In one particular embodiment, the enzyme is expressed using the lentiviral vector shown in Figure 11, which shows a vector map for expressing human FUT6 on the surface of a bait cell. This construct includes from N- to C-terminus a membrane alanyl aminopeptidase transmembrane domain (TMD) linked to an HA tag via two glycine amino acids; and a Linker (containing a TEV cleavage site) linking the HA tagged TMD to the FUT6 ectodomain. The protein sequence of the FUT6 lentivirus construct insert is 416aa and is predicted to encode a 47.21kDa protein with the following amino acid sequence:

MAKGFYISKSLGILGILLGVAAVCTIIALSVVYSQEKNKNANSSPVAST
TPSASATTNPASATTLGGYPYDVPDYAEFASTSLYKKAGSENLYFQGD
PTVYPNGSRFPDSTGTPAHSIPLILLWTWPFNKPIALPRCSEMVPGTAD
CNITADRKVYPQADAVIVHHREVMYNPSAQLPRSPRRQGQRWIWFSM
ESPSHCWQLKAMDGYFNLTMSYRSDSDIFTPYGWLEPWSGQPAHPPL
NLSAKTELVAWAVSNWGPNSARVRYYQSLQAHLKVDVYGRSHKPLP
QGTMMETLSRYKFYLAFENSLHPDYITEKLWRNALEAWAVPVVLGPS
RSNYERFLPPDAFIHVDDFQSPKDLARYLQELDKDHARYLSYFRWRET
LRPRSFSWALAFCKACWKLQEESRYQTRGIAAWFT (SEQ ID NO: 1)

The FUT6 lentivirus construct insert may be encoded by the following DNA sequence (1251bp).

ATGGCCAAGGGCTTCTATATTTCCAAGTCCCTGGGCATCCTGGGGA
TCCTCCTGGGCGTGGCAGCCGTGTGCACAATCATCGCACTGTCAGT
GGTGTACTCCCAGGAGAAGAACAAGAACGCCAACAGCTCCCCCGT
GGCCTCCACCACCCCGTCCGCCTCAGCCACCACCAACCCCGCCTCG
GCCACCACCTTGGGCGGCTACCCATACGATGTTCCAGATTACGCTG
AGTTCGCCAGCACCAGCCTGTACAAGAAGGCCGGCAGCGAGAACC
TGTACTTCCAGGGCGATCCCACTGTGTACCCTAATGGGTCCCGCTT
CCCAGACAGCACAGGGACCCCCGCCCACTCCATCCCCCTGATCCTG
CTGTGGACGTGGCCTTTTAACAAACCCATAGCTCTGCCCCGCTGCT
CAGAGATGGTGCCTGGCACGGCTGACTGCAACATCACTGCCGACC
GCAAGGTGTATCCACAGGCAGACGCGGTCATCGTGCACCACCGAG
AGGTCATGTACAACCCCAGTGCCCAGCTCCCACGCTCCCCGAGGCG
GCAGGGGCAGCGATGGATCTGGTTCAGCATGGAGTCCCCAAGCCA
CTGCTGGCAGCTGAAAGCCATGGACGGATACTTCAATCTCACCATG
TCCTACCGCAGCGACTCCGACATCTTCACGCCCTACGGCTGGCTGG
AGCCGTGGTCCGGCCAGCCTGCCCACCCACCGCTCAACCTCTCGGC
CAAGACCGAGCTGGTGGCCTGGGCAGTGTCCAACTGGGGGCCAAA
CTCCGCCAGGGTGCGCTACTACCAGAGCCTGCAGGCCCATCTCAAG
GTGGACGTGTACGGACGCTCCCACAAGCCCCTGCCCCAGGGAACC
ATGATGGAGACGCTGTCCCGGTACAAGTTCTATCTGGCCTTCGAGA
ACTCCTTGCACCCCGACTACATCACCGAGAAGCTGTGGAGGAACGC
CCTGGAGGCCTGGGCCGTGCCCGTGGTGCTGGGCCCCAGCAGAAG
CAACTACGAGAGGTTCCTGCCACCCGACGCCTTCATCCACGTGGAC
GACTTCCAGAGCCCCAAGGACCTGGCCCGGTACCTGCAGGAGCTG
GACAAGGACCACGCCCGCTACCTGAGCTACTTTCGCTGGCGGGAG
ACGCTGCGGCCTCGCTCCTTCAGCTGGGCACTCGCTTTCTGCAAGG
CCTGCTGGAAACTGCAGGAGGAATCCAGGTACCAGACACGCGGCA
TAGCGGCTTGGTTCACCTGA (SEQ ID NO: 2)

The FUT6 lentivirus construct with the insert in the lentiviral expression vector may be encoded by the following DNA sequence (8622bp)

caggtggcacttttcggggaaatgtgcgcggaacccctatttgtttatttttctaaatacattcaaatatgtatccgct
catgagacaataaccctgataaatgcttcaataatattgaaaaaggaagagtatgagtattcaacatttccgtgtcg
cccttattcccttttttgcggcattttgccttcctgtttttgctcacccagaaacgctggtgaaagtaaaagatgctga
agatcagttgggtgcacgagtgggttacatcgaactggatctcaacagcggtaagatccttgagagttttcgccc
cgaagaacgttttccaatgatgagcacttttaaagttctgctatgtggcgcggtattatcccgtattgacgccgggc
aagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttac
ttctgacaacgatcggaggaccgaaggagctaaccgcttttttgcacaacatgggggatcatgtaactcgccttg
atcgttgggaaccggagctgaatgaagccataccaaacgacgagcgtgacaccacgatgcctgtagcaatgg
caacaacgttgcgcaaactattaactggcgaactacttactctagcttcccggcaacaattaatagactggatgga
ggcggataaagttgcaggaccacttctgcgctcggcccttccggctggctggtttattgctgataaatctggagc
cggtgagcgtgggtctcgcggtatcattgcagcactggggccagatggtaagccctcccgtatcgtagttatcta
cacgacggggagtcaggcaactatggatgaacgaaatagacagatcgctgagataggtgcctcactgattaag

cattggtaactgtcagaccaagtttactcatatatactttagattgatttaaaacttcatttttaatttaaaaggatctag
gtgaagatcctttttgataatctcatgaccaaaatcccttaacgtgagttttcgttccactgagcgtcagaccccgta
gaaaagatcaaaggatcttcttgagatcctttttttctgcgcgtaatctgctgcttgcaaacaaaaaaaccaccgct
accagcggtggtttgtttgccggatcaagagctaccaactctttttccgaaggtaactggcttcagcagagcgca
gataccaaatactgtccttctagtgtagccgtagttaggccaccacttcaagaactctgtagcaccgcctacatac
ctcgctctgctaatcctgttaccagtggctgctgccagtggcgataagtcgtgtcttaccgggttggactcaagac
gatagttaccggataaggcgcagcggtcgggctgaacggggggttcgtgcacacagcccagcttggagcga
acgacctacaccgaactgagatacctacagcgtgagctatgagaaagcgccacgcttcccgaagggagaaag
gcggacaggtatccggtaagcggcagggtcggaacaggagagcgcacgagggagcttccagggggaaac
gcctggtatctttatagtcctgtcgggtttcgccacctctgacttgagcgtcgatttttgtgatgctcgtcagggggg
cggagcctatggaaaaacgccagcaacgcggcctttttacggttcctggccttttgctggccttttgctcacatgtt
ctttcctgcgttatcccctgattctgtggataaccgtattaccgcctttgagtgagctgataccgctcgccgcagcc
gaacgaccgagcgcagcgagtcagtgagcgaggaagcggaagagcgcccaatacgcaaaccgcctctccc
cgcgcgttggccgattcattaatgcagctggcacgacaggtttcccgactggaaagcgggcagtgagcgcaac
gcaattaatgtgagttagctcactcattaggcaccccaggctttacactttatgcttccggctcgtatgttgtgtgga
attgtgagcggataacaatttcacacaggaaacagctatgaccatgattacgccaagcgcgcaattaaccctcac
taaagggaacaaaagctggagctgcaagcttaatgtagtcttatgcaatactcttgtagtcttgcaacatggtaac
gatgagttagcaacatgccttacaaggagagaaaaagcaccgtgcatgccgattggtggaagtaaggtggtac
gatcgtgccttattaggaaggcaacagacgggtctgacatggattggacgaaccactgaattgccgcattgcag
agatattgtatttaagtgcctagctcgatacaataaacgggtctctctggttagaccagatctgagcctgggagctc
tctggctaactagggaacccactgcttaagcctcaataaagcttgccttgagtgcttcaagtagtgtgtgcccgtct
gttgtgtgactctggtaactagagatccctcagacccttttagtcagtgtggaaaatctctagcagtggcgcccga
acagggacctgaaagcgaaagggaaaccagagctctctcgacgcaggactcggcttgctgaagcgcgcacg
gcaagaggcgaggggcggcgactggtgagtacgccaaaaattttgactagcggaggctagaaggagagag
atgggtgcgagagcgtcagtattaagcgggggagaattagatcgcgatgggaaaaaattcggttaaggccag
ggggaaagaaaaaatataaattaaaacatatagtatgggcaagcagggagctagaacgattcgcagttaatcct
ggcctgttagaaacatcagaaggctgtagacaaatactgggacagctacaaccatcccttcagacaggatcag
aagaacttagatcattatataatacagtagcaaccctctattgtgtgcatcaaaggatagagataaaagacaccaa
ggaagctttagacaagatagaggaagagcaaaacaaaagtaagaccaccgcacagcaagcggccgctgatc
ttcagacctggaggaggagatatgagggacaattggagaagtgaattatataaatataaagtagtaaaaattgaa
ccattaggagtagcacccaccaaggcaaagagaagagtggtgcagagagaaaaaagagcagtgggaatag
gagctttgttccttgggttcttgggagcagcaggaagcactatgggcgcagcctcaatgacgctgacggtacag
gccagacaattattgtctggtatagtgcagcagcagaacaatttgctgagggctattgaggcgcaacagcatctg
ttgcaactcacagtctggggcatcaagcagctccaggcaagaatcctggctgtggaaagatacctaaaggatca
acagctcctggggatttggggttgctctggaaaactcatttgcaccactgctgtgccttggaatgctagttggagt
aataaatctctggaacagattggaatcacacgacctggatggagtgggacagagaaattaacaattacacaagc
ttaatacactccttaattgaagaatcgcaaaaccagcaagaaaagaatgaacaagaattattggaattagataaat
gggcaagtttgtggaattggtttaacataacaaattggctgtggtatataaaattattcataatgatagtaggaggct
tggtaggtttaagaatagtttttgctgtactttctatagtgaatagagttaggcagggatattcaccattatcgtttcag
acccacctcccaaccccgaggggacccgacaggcccgaaggaatagaagaagaaggtggagagagagac
agagacagatccattcgattagtgaacggatctcgacggttaacttttaaaagaaaaggggggattggggggta
cagtgcaggggaaagaatagtagacataatagcaacagacatacaaactaaagaattacaaaaacaaattaca
aaaattcaaaattttatcgagctttgcaaagatggataaagttttaaacagagaggaatctttgcagctaatggacc
ttctaggtcttgaaaggagtgcctCGTGAGGCTCCGGTGCCCGTCAGTGGGCAGA
GCGCACATCGCCCACAGTCCCCGAGAAGTTGGGGGGAGGGGTCGG
CAATTGAACCGGTGCCTAGAGAAGGTGGCGCGGGGTAAACTGGGA
AAGTGATGTCGTGTACTGGCTCCGCCTTTTTCCCGAGGGTGGGGGA
GAACCGTATATAAGTGCAGTAGTCGCCGTGAACGTTCTTTTTCGCA
ACGGGTTTGCCGCCAGAACACAGGTAAGTGCCGTGTGTGGTTCCCG
CGGGCCTGGCCTCTTTACGGGTTATGGCCCTTGCGTGCCTTGAATTA

CTTCCACCTGGCTGCAGTACGTGATTCTTGATCCCGAGCTTCGGGTT
GGAAGTGGGTGGGAGAGTTCGAGGCCTTGCGCTTAAGGAGCCCCT
TCGCCTCGTGCTTGAGTTGAGGCCTGGCCTGGGCGCTGGGGCCGCC
GCGTGCGAATCTGGTGGCACCTTCGCGCCTGTCTCGCTGCTTTCGAT
AAGTCTCTAGCCATTTAAAATTTTTGATGACCTGCTGCGACGCTTTT
TTTCTGGCAAGATAGTCTTGTAAATGCGGGCCAAGATCTGCACACT
GGTATTTCGGTTTTTGGGGCCGCGGGCGGCGACGGGGCCCGTGCGT
CCCAGCGCACATGTTCGGCGAGGCGGGGCCTGCGAGCGCGGCCAC
CGAGAATCGGACGGGGGTAGTCTCAAGCTGGCCGGCCTGCTCTGGT
GCCTGGCCTCGCGCCGCCGTGTATCGCCCCGCCCTGGGCGGCAAGG
CTGGCCCGGTCGGCACCAGTTGCGTGAGCGGAAAGATGGCCGCTTC
CCGGCCCTGCTGCAGGGAGCTCAAAATGGAGGACGCGGCGCTCGG
GAGAGCGGGCGGGTGAGTCACCCACACAAAGGAAAAGGGCCTTTC
CGTCCTCAGCCGTCGCTTCATGTGACTCCACGGAGTACCGGGCGCC
GTCCAGGCACCTCGATTAGTTCTCGAGCTTTTGGAGTACGTCGTCTT
TAGGTTGGGGGGAGGGGTTTTATGCGATGGAGTTTCCCCACACTGA
GTGGGTGGAGACTGAAGTTAGGCCAGCTTGGCACTTGATGTAATTC
TCCTTGGAATTTGCCCTTTTTGAGTTTGGATCTTGGTTCATTCTCAA
GCCTCAGACAGTGGTTCAAAGTTTTTTTCTTCCATTTCAGGTGTCGT
GAggaattcggtaccgcggccgcccggggatccATGGCCAAGGGCTTCTATATTTCC
AAGTCCCTGGGCATCCTGGGGATCCTCCTGGGCGTGGCAGCCGTGT
GCACAATCATCGCACTGTCAGTGGTGTACTCCCAGGAGAAGAACA
AGAACGCCAACAGCTCCCCCGTGGCCTCCACCACCCCGTCCGCCTC
AGCCACCACCAACCCCGCCTCGGCCACCACCTTGGGCGGCTACCCA
TACGATGTTCCAGATTACGCTGAGTTCGCCAGCACCAGCCTGTACA
AGAAGGCCGGCAGCGAGAACCTGTACTTCCAGGGCGATCCCACTG
TGTACCCTAATGGGTCCCGCTTCCCAGACAGCACAGGGACCCCCGC
CCACTCCATCCCCCTGATCCTGCTGTGGACGTGGCCTTTTAACAAA
CCCATAGCTCTGCCCCGCTGCTCAGAGATGGTGCCTGGCACGGCTG
ACTGCAACATCACTGCCGACCGCAAGGTGTATCCACAGGCAGACG
CGGTCATCGTGCACCACCGAGAGGTCATGTACAACCCCAGTGCCCA
GCTCCCACGCTCCCCGAGGCGGCAGGGGCAGCGATGGATCTGGTTC
AGCATGGAGTCCCCAAGCCACTGCTGGCAGCTGAAAGCCATGGAC
GGATACTTCAATCTCACCATGTCCTACCGCAGCGACTCCGACATCT
TCACGCCCTACGGCTGGCTGGAGCCGTGGTCCGGCCAGCCTGCCCA
CCCACCGCTCAACCTCTCGGCCAAGACCGAGCTGGTGGCCTGGGCA
GTGTCCAACTGGGGGCCAAACTCCGCCAGGGTGCGCTACTACCAG
AGCCTGCAGGCCCATCTCAAGGTGGACGTGTACGGACGCTCCCACA
AGCCCCTGCCCCAGGGAACCATGATGGAGACGCTGTCCCGGTACA
AGTTCTATCTGGCCTTCGAGAACTCCTTGCACCCCGACTACATCAC
CGAGAAGCTGTGGAGGAACGCCCTGGAGGCCTGGGCCGTGCCCGT
GGTGCTGGGCCCCAGCAGAAGCAACTACGAGAGGTTCCTGCCACC
CGACGCCTTCATCCACGTGGACGACTTCCAGAGCCCCAAGGACCTG
GCCCGGTACCTGCAGGAGCTGGACAAGGACCACGCCCGCTACCTG
AGCTACTTTCGCTGGCGGGAGACGCTGCGGCCTCGCTCCTTCAGCT
GGGCACTCGCTTTCTGCAAGGCCTGCTGGAAACTGCAGGAGGAATC
CAGGTACCAGACACGCGGCATAGCGGCTTGGTTCACCTGAgtcgacaat
caacctctggattacaaaatttgtgaaagattgactggtattcttaactatgttgctccttttacgctatgtggatacgc
tgctttaatgcctttgtatcatgctattgcttcccgtatggctttcattttctcctccttgtataaatcctggttgctgtctct
ttatgaggagttgtggcccgttgtcaggcaacgtggcgtggtgtgcactgtgtttgctgacgcaacccccactgg

ttggggcattgccaccacctgtcagctcctttccgggactttcgctttccccctccctattgccacggcggaactca
tcgccgcctgccttgcccgctgctggacaggggctcggctgttgggcactgacaattccgtggtgttgtcgggg
aagctgacgtcctttccatggctgctcgcctgtgttgccacctggattctgcgcgggacgtccttctgctacgtcc
cttcggccctcaatccagcggaccttccttcccgcggcctgctgccggctctgcggcctcttccgcgtcttcgcct
tcgccctcagacgagtcggatctccctttgggccgcctccccgcctggaattcgagctcggtacctttaagacca
atgacttacaaggcagctgtagatcttagccactttttaaaagaaaaggggggactggaagggctaattcactcc
caacgaagacaagatctgctttttgcttgtactgggtctctctggttagaccagatctgagcctgggagctctctgg
ctaactagggaacccactgcttaagcctcaataaagcttgccttgagtgcttcaagtagtgtgtgcccgtctgttgt
gtgactctggtaactagagatccctcagacccttttagtcagtgtggaaaatctctagcagtagtagttcatgtcatc
ttattattcagtatttataacttgcaaagaaatgaatatcagagagtgagaggaacttgtttattgcagcttataatggt
tacaaataaagcaatagcatcacaaatttcacaaataaagcatttttttcactgcattctagttgtggtttgtccaaact
catcaatgtatcttatcatgtctggctctagctatcccgcccctaactccgcccagttccgcccattctccgccccat
ggctgactaattttttttatttatgcagaggccgaggccgcctcggcctctgagctattccagaagtagtgaggag
gcttttttggaggcctaggcttttgcgtcgagacgtacccaattcgccctatagtgagtcgtattacgcgcgctcac
tggccgtcgttttacaacgtcgtgactgggaaaaccctggcgttacccaacttaatcgccttgcagcacatcccc
ctttcgccagctggcgtaatagcgaagaggcccgcaccgatcgcccttcccaacagttgcgcagcctgaatgg
cgaatggcgcgacgcgccctgtagcggcgcattaagcgcggcgggtgtggtggttacgcgcagcgtgaccg
ctacacttgccagcgccctagcgcccgctcctttcgctttcttcccttcctttctcgccacgttcgccggctttcccc
gtcaagctctaaatcgggggctccctttagggttccgatttagtgctttacggcacctcgaccccaaaaaacttga
ttagggtgatggttcacgtagtgggccatcgccctgatagacggtttttcgccctttgacgttggagtccacgttctt
taatagtggactcttgttccaaactggaacaacactcaaccctatctcggtctattcttttgatttataagggattttgc
cgatttcggcctattggttaaaaaatgagctgatttaacaaaaatttaacgcgaattttaacaaaatattaacgtttac
aatttcc (SEQ ID NO: 3)

### IV. Methods of using Bait cells

In some examples, the present disclosure provides a method for interaction-dependent labeling a prey cell with a bait cell, the method comprising contacting a prey cell with a bait cell in the presence of a suitable tagged substrate. Reference herein to a "suitable tagged substrate" means a substrate that may be utilized in an interaction-dependent labeling reaction by an enzyme that is bound on the surface of a bait cell, wherein the substrate comprises a tag, as disclosed herein. So, e.g., but not to be limited in any way, if the enzyme disposed on the bait cell is a fucosyltransferase, then reference to a "suitable tagged substrate" means a tagged-GDP-Fucose (e.g., GDP-Fuc-Biotin). Similarly, if the enzyme disposed on the bait cell is a sialyltransferase, then reference to a "suitable tagged substrate" means a tagged CMP-sialic acid (e.g., CMP-NeuAc-Biotin). Due to the presence of the enzyme on the surface of the bait cell, such a contacting event results in interaction-dependent labeling; i.e., the contact-induced conjugation of the tagged substrate onto the prey cell. As discussed above, the choice of a suitable substrate will be clear to a person of skill in the art and will depend on the enzyme that is engineered on the bait cell.

The presence of a tag on a prey cell (i.e., "the labeled cell") may be determined by any suitable means including, e.g., without limitation via immunofluorescence; immunohistochemistry; immunoblot; flow cytometry; FACS; microarray analysis, SDS page; mass spectrometry; HPLC: The labeled cell may be enriched for, e.g., utilizing FACS sorting for the presence of the label. The labeled calls may be further sorted by the existence or lack of existence of other markers, e.g., cell surface markers such as e.g. PD-1, CD134, CD137, CXCR5, and/or TIM3 and/or additional markers for indicating cell type, e.g., CD45, CD8, CD4, and the like.

In one embodiment, the present disclosure provides compositions and methods by which an enzyme, for example, fucosyltransferase (FT) is conjugated to its substrate GDP-Fuc *via* a short PEG linker to form GDP-Fuc-FT. GDP-Fuc-FT serves as the self-catalyst to transfer Fuc-FT to LacNAc in the cell-surface glycocalyx in approximately 15 mins. The cell-FT conjugate is capable of transferring probe molecules (e.g., GDP-Fuc-biotin or GDP-Fuc-tag) to the surface glycans of contact prey cells, for the detection of a cell-cell interaction. Moreover, data provided herein showing successful conjugation of several other enzymes to cell surfaces demonstrates the versatility of this conjugation method for imparting new enzymatic functions to cells. This technique has several advantages: (1) It is suitable for different cell types since GDP-fucose acceptor-glycans LacNAc or α2,3-sialylated LacNAc (sLacNAc) are commonly found in complex and hybrid N-glycans decorating most cell surfaces; (2) no time-consuming and complicated genetic modification is necessary; and (3) common laboratory techniques such as fluorescent microscopic imaging and flow cytometry / FACS are used to detect/monitor cell-cell interactions / sort for cells that have been proximity labeled in this manner.

In some examples, the present disclosure provides a method for tagging a tumor-specific antigen (TSA) reactive T cell present in a population of tumor infiltrating lymphocytes (TILs), the method comprising
providing a bait cell that is a dendritic cell engineered to comprise on its cell surface a suitable enzyme for catalyzing an interaction-dependent labeling reaction on a prey cell;
incubating the bait cell with one or more TSAs or a source of one or more TSAs (e.g., a tumor cell lysate) in order to prime the dendritic cell with one or more TSAs;
contacting the bait cell with a population of tumor infiltrating lymphocytes (e.g., comprised in a population of cells obtained from a tumor sample); wherein the population comprises at least one tumor-specific antigen (TSA) reactive T cell; and wherein the contacting occurs in the presence of a tagged substrate for the enzyme.

In some examples, the method further comprises enriching for the tagged TSA reactive T cells by sorting the tagged cells by FACS for the presence of the tag alone or in combination with one or more additional cell marker to enrich for the desired cell population. For example, in some examples, the cells are sorted to enrich for tagged cells expressing T cell markers (i.e., enrich for CD8+ cells for cytotoxic T-cells; CD4+ for helper T cells); to exclude cells expressing DC markers (i.e., enrich for CD45.1 ^{-/-} cells); and/or to enrich for markers indicative of prospective TSA reactivity (i.e., enrich for cells that are PD-1+, CD134+, CD137+) and or other markers such as CXCR5+, and/or TIM3+. In some examples of the method, the enzyme is a fucosyltransferase and the tagged substrate is GDP-fucose conjugated to a tag. In some examples, the tag is any one of the tags disclosed herein. In some examples, the tag is biotin. In some embodiment, the fucosyltransferase enzyme is a human fucosyltransferase. In some embodiment, the fucosyltransferase enzyme is human α 1,3-fucosyltransferase. In one embodiment, the human α 1,3 -fucosyltransferase is recombinantly prepared. In some embodiment, the fucosyltransferase enzyme is not a human fucosyltransferase. In some embodiment, the fucosyltransferase enzyme is an *H. pylori* fucosyltransferase. In one embodiment, the *H. pylori* fucosyltransferase is *H. pylori* α 1,3-fucosyltransferase. In one embodiment, the *H. pylori* fucosyltransferase is *H. pylori* α 1,3/1,4 -fucosyltransferase. In some examples of the method, the enzyme is a sialyltransferase and the tagged substrate is CMP-Neu5Ac conjugated to a tag. In some examples, the tag is biotin. In some examples, the sialyltransferase enzyme is a human sialyltransferase. In some examples, the sialyltransferase enzyme is human ST6GAL1. In one embodiment, the human ST6GAL1 is recombinantly prepared. In some embodiment, the sialyltransferase enzyme is human ST6GalNAc1. In one embodiment, the human ST6GalNAc1 is recombinantly prepared. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST). In some examples, the bait cell is engineered to comprise the enzyme on its surface via conjugation of the enzyme to the cell's surface or via recombinant expression of the enzyme in the cell. In some examples, the conjugation is a chemical conjugation. In some examples, the chemical conjugation is via Method 2 disclosed in Example 7, herein. In some examples, the conjugation is via enzymatic conjugation of the enzyme to the cell surface. In some examples, the enzymatic conjugation is via fucosylation of the cell with a GDP-Fuc-Enzyme conjugate according to Method 1, disclosed in Example 7, herein. In particular examples, the fucosylation enzyme catalyzing the conjugation of the enzyme to the surface of the cell is *H. pylori* α1,3fucosyltransferase enzyme. In some examples, the fucosylation enzyme catalyzing the conjugation of the enzyme to the surface of the cell is human α1,3fucosyltransferase (FUT6) or *H. pylori* α1,3/4fucosyltransferase). In some examples, the enzymatic conjugation is performed as described in Method 1, disclosed in Example 7, herein, except that instead of using a fucosylation reaction with a fucosyltransferase enzyme and a GDP-Fuc-Enzyme conjugate as the donor nucleotide substrate to conjugate the enzyme onto the surface of the cell, the method utilizes a sialyltransferase enzyme according to and a CMP-NeuAc-Enzyme to conjugate the enzyme onto the surface of the cell. In some examples, the sialyltransferase enzyme is a human sialyltransferase. In some examples, the sialyltransferase enzyme is human ST6GAL1 human ST6GalNAc1. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST).

In some examples, the present disclosure provides a method for tagging a tumor-specific antigen (TSA) reactive T cell present in a population of tumor infiltrating lymphocytes (TILs), the method comprising
providing a dendritic cell;
incubating the dendritic cell with one or more TSAs or a source of one or more TSAs (e.g., a tumor cell lysate) in order to prime the dendritic cell with one or more TSAs;
conjugating the dendritic cell on its cell surface with a suitable enzyme for catalyzing an interaction-dependent labeling reaction on a prey cell;
contacting the bait cell with a population of tumor infiltrating lymphocytes (e.g., comprised in a population of cells obtained from a tumor sample); wherein the population comprises at least one tumor-specific antigen (TSA) reactive T cell; and wherein the contacting occurs in the presence of a tagged substrate for the enzyme.

In some examples, the method further comprises enriching for the tagged TSA reactive T cells by sorting the tagged cells by FACS for the presence of the tag alone or in combination with one or more additional cell marker to enrich for the desired cell population. For example, in some examples, the cells are sorted to enrich for tagged cells expressing T cell markers (i.e., enrich for CD8+ cells for cytotoxic T-cells; CD4+ for helper T cells); to exclude cells expressing DC markers (i.e., enrich for CD45.1^{-/-} cells); and/or to enrich for markers indicative of prospective TSA reactivity (i.e., enrich for cells that are PD-1+, CD134+, CD137+) or other makers such as CXCR5+, and/or TIM3+. In some examples of the method, the enzyme is a fucosyltransferase and the tagged substrate is GDP-fucose conjugated to a tag. In some examples, the tag is any one of the tags disclosed herein. In some examples, the tag is biotin. In some embodiment, the fucosyltransferase enzyme is a human fucosyltransferase. In some embodiment, the fucosyltransferase enzyme is human α 1,3-fucosyltransferase. In one embodiment, the human α 1,3 -fucosyltransferase is recombinantly prepared. In some embodiment, the fucosyltransferase enzyme is not a human fucosyltransferase. In some embodiment, the fucosyltransferase enzyme is an *H. pylori* fucosyltransferase. In one embodiment, the *H. pylori* fucosyltransferase is *H. pylori* α 1,3-fucosyltransferase. In one embodiment, the *H. pylori* fucosyltransferase is *H. pylori* α 1,3/1,4 -fucosyltransferase. In some examples of the method, the enzyme is a sialyltransferase and the tagged substrate is CMP-Neu5Ac conjugated to a tag. In some examples, the tag is biotin. In some examples, the sialyltransferase enzyme is a human sialyltransferase. In some examples, the sialyltransferase enzyme is human ST6GAL1. In one embodiment, the human ST6GAL1 is recombinantly prepared. In some embodiment, the sialyltransferase enzyme is human ST6GalNAc1. In one embodiment, the human ST6GalNAc1 is recombinantly prepared. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST). In some examples, the conjugation of the enzyme on the cell surface of the dendritic cell is via chemical conjugation. In some examples, the chemical conjugation is performed as in Method 2, disclosed in Example 7, herein using a fucosyltransferase or a sialyltransferase. In some examples, the conjugation of the enzyme on the cell surface of the dendritic cell is via enzymatic conjugation of the enzyme to the cell surface. In some examples, the enzymatic conjugation is via fucosylation of the cell with a GDP-Fuc-Enzyme conjugate according to Method 1, disclosed in Example 7, herein. In particular examples, the fucosylation enzyme catalyzing the conjugation of the enzyme to the surface of the cell is *H. pylori* α1,3fucosyltransferase enzyme. In some examples, the fucosylation enzyme catalyzing the conjugation of the enzyme to the surface of the cell is human α1,3fucosyltransferase (FUT6) or *H. pylori* α1,3/4fucosyltransferase). In some examples, the enzymatic conjugation is performed as described in Method 1, disclosed in Example 7, herein, except that instead of using a fucosylation reaction with a fucosyltransferase enzyme and a GDP-Fuc-Enzyme conjugate as the donor nucleotide substrate to conjugate the enzyme onto the surface of the cell, the method utilizes a sialyltransferase enzyme according to and a CMP-NeuAc-Enzyme to conjugate the enzyme onto the surface of the cell. In some examples, the sialyltransferase enzyme is a human sialyltransferase. In some examples, the sialyltransferase enzyme is human ST6GAL1 human ST6GalNAc1. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST).

In some examples, the present disclosure provides a method for identifying and enriching for tumor-specific antigen (TSA) reactive T cells from tumor infiltrating lymphocytes (TILs), the method comprising
(a) providing a bait cell that is a dendritic cell engineered to comprise on its cell surface a suitable enzyme for catalyzing an interaction-dependent labeling reaction on a prey cell;
(b) incubating the bait cell with one or more TSAs or a source of one or more TSAs (e.g., a tumor cell lysate) in order to prime the dendritic cell with one or more TSAs;
(c) contacting the bait cell with a population of tumor infiltrating lymphocytes (e.g., comprised in a population of cells obtained from a tumor sample); wherein the population comprises at least one tumor-specific antigen (TSA) reactive T cell; and wherein the contacting occurs in the presence of a suitable tagged substrate for the enzyme on the bait cell; and
(d) isolating any cells comprising the tagged substrate from the population of tumor infiltrating lymphocytes that do not contain the tagged substrate.

The isolating step (d) may be performed in any suitable way. For example, in one embodiment, the isolating step (d) is performed via a pulldown assay using a solid substrate, e.g., beads, conjugated to a binding moiety having specificity for the tag on the substrate. In one preferred embodiment, the isolating step (d) is performed using FACS.

In some examples, the method further comprises enriching for the tagged TSA reactive T cells by sorting the tagged cells by FACS for the presence of the tag alone or in combination with one or more additional cell marker to enrich for the desired cell population. For example, in some examples, the cells are sorted to enrich for tagged cells expressing T cell markers (i.e., enrich for CD8+ cells for cytotoxic T-cells; CD4+ for helper T cells); to exclude cells expressing DC markers (i.e., enrich for CD45.1 ^{-/-} cells); and/or to enrich for markers indicative of prospective TSA reactivity (i.e., enrich for cells that are PD-1+, CD134+, and/or CD137+) or enrich based upon expression profiles for other markers, e.g., CXCR5+, and/or TIM3+). In one embodiment, cytotoxic TSA reactive T cells are enriched for by isolating tagged cells that are also CD8+ and PD-1+ and/or tagged cells that are also CD4+. Such TSA reactive T cells are expanded and used in adoptive T cell therapies. Such TSA reactive T cells are also isolated and sequenced by single cell T cell receptor (TCR) sequencing to determine the sequence of the TSA reactive TCRs enabling the recombinant construction of TSA-reactive TCR T-cells for research and therapeutic purposes. In some examples of the method, the enzyme is a fucosyltransferase and the tagged substrate is GDP-fucose conjugated to a tag. In some examples, the tag is any one of the tags disclosed herein. In some examples, the tag is biotin. In some embodiment, the fucosyltransferase enzyme is a human fucosyltransferase. In some embodiment, the fucosyltransferase enzyme is human α 1,3-fucosyltransferase. In one embodiment, the human α 1,3 -fucosyltransferase is recombinantly prepared. In some embodiment, the fucosyltransferase enzyme is not a human fucosyltransferase. In some embodiment, the fucosyltransferase enzyme is an *H. pylori* fucosyltransferase. In one embodiment, the *H. pylori* fucosyltransferase is *H. pylori* α 1,3-fucosyltransferase. In one embodiment, the *H. pylori* fucosyltransferase is *H. pylori* α 1,3/1,4

-fucosyltransferase. In some examples of the method, the enzyme is a sialyltransferase and the tagged substrate is CMP-Neu5Ac conjugated to a tag. In some examples, the tag is biotin. In some examples, the sialyltransferase enzyme is a human sialyltransferase. In some examples, the sialyltransferase enzyme is human ST6GAL1. In one embodiment, the human ST6GAL1 is recombinantly prepared. In some embodiment, the sialyltransferase enzyme is human ST6GalNAc1. In one embodiment, the human ST6GalNAc1 is recombinantly prepared. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST). In some examples, the conjugation of the enzyme on the cell surface of the dendritic cell is via chemical conjugation. In some examples, the chemical conjugation is performed as in Method 2, disclosed in Example 7, herein using a fucosyltransferase or a sialyltransferase. In some examples, the conjugation of the enzyme on the cell surface of the dendritic cell is via enzymatic conjugation of the enzyme to the cell surface. In some examples, the enzymatic conjugation is via fucosylation of the cell with a GDP-Fuc-Enzyme conjugate according to Method 1, disclosed in Example 7, herein. In particular examples, the fucosylation enzyme catalyzing the conjugation of the enzyme to the surface of the cell is *H. pylori* α1,3fucosyltransferase enzyme. In some examples, the fucosylation enzyme catalyzing the conjugation of the enzyme to the surface of the cell is human α1,3fucosyltransferase (FUT6) or *H. pylori* α1,3/4fucosyltransferase). In some examples, the enzymatic conjugation is performed as described in Method 1, disclosed in Example 7, herein, except that instead of using a fucosylation reaction with a fucosyltransferase enzyme and a GDP-Fuc-Enzyme conjugate as the donor nucleotide substrate to conjugate the enzyme onto the surface of the cell, the method utilizes a sialyltransferase enzyme according to and a CMP-NeuAc-Enzyme to conjugate the enzyme onto the surface of the cell. In some examples, the sialyltransferase enzyme is a human sialyltransferase. In some examples, the sialyltransferase enzyme is human ST6GAL1 human ST6GalNAc1. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST).

For example, Examples 5-6 demonstrate one embodiment of the instant method, wherein the bait cell is a dendritic cell engineered to have a fucosyltransferase (e.g., H. pylori α 1,3-fucosyltransferase) on its surface and the tagged substrate is biotin-conjugated GDP-fucose. During step (b), incubating the dendritic cell bait cells with a tumor cell lysate causes the DCs to bind to and present TSAs present in the lysate. During the contacting step of (c), any T cells that are present in the population of tumor infiltrating lymphocytes that express T cell receptors (TCRs) with affinity for a TSA present in the lysate and presented by the DCs are proximity labeled by the DC-surface-bound fucosyltransferase resulting in the incorporation of a biotin moiety onto the T cell surface by fucosylation of LacNAc and sialo-LacNAc on the glycocalx of the cell. Cells are then stained for biotin (using, e.g., streptavidin-APC or fluorescein-conjugated streptavidin) and other markers of cell type such as CD45, CD8, CD4, PD-1, CD134, CD137, CXCR5, and TIM3 and cells having streptavidin signal are isolated via FACS and further sorted for the presence or absence of other markers. Biotinylated CD8+ cells that express PD-1 are highly enriched for cytotoxic TSA reactive T cells. Single cell TCR sequencing of these cell is subsequently utilized to determine the identity of the TSA-specific TCRs.

In some examplesexamples, the present disclosure provides a population of TSA reactive T cells, all, or substantially all of which (i) are conjugated to one or more tagged fucose or tagged NeuAc moiety on their surface; (ii) are CD4+ or CD8+; and, optionally, are PD-1+ and CD45.1-/-. In some examples, the TSA reactive T cells are substantially or entirely CD8+. In some examples, the TSA reactive T cells are substantially or entirely CD4+.

In some examples, the present disclosure provides a method for tagging auto-reactive T cells present in a population of tissue infiltrating lymphocytes (TiILs) or circulating T cells, the method comprising
providing a dendritic cell;
incubating the dendritic cell with one or more autoantigens or a source of one or more autoantigens (e.g., a diseased tissue biopsy cell lysate from a subject having an autoimmune disease) in order to prime the dendritic cell with one or more autoantigen;
conjugating the dendritic cell on its cell surface with a suitable enzyme for catalyzing an interaction-dependent labeling reaction on a prey cell;
contacting the bait cell with a population of TiILs (e.g., comprised in a population of cells obtained from a diseased tissue biopsy from the subject having an autoimmune disease; wherein the population comprises at least one auto-reactive T cell; and wherein the contacting occurs in the presence of a tagged substrate for the enzyme.

In some examples, the method further comprises enriching for the tagged auto-reactive T cells by sorting the tagged cells by FACS for the presence of the tag alone or in combination with one or more additional cell marker to enrich for the desired cell population. For example, in some examples, the cells are sorted to enrich for tagged cells expressing T cell markers (i.e., enrich for CD8+ cells for cytotoxic T-cells; CD4+, CD25+, FOXP3+ for T cells that are prospective antigen specific regulatory T cells); and/or to exclude cells expressing DC markers (i.e., enrich for CD45.1 ^{-/-} cells). In some examples of the method, the enzyme is a fucosyltransferase and the tagged substrate is GDP-fucose conjugated to a tag. In some examples, the tag is any one of the tags disclosed herein. In some examples, the tag is biotin. In some embodiment, the fucosyltransferase enzyme is a human fucosyltransferase. In some embodiment, the fucosyltransferase enzyme is human α 1,3-fucosyltransferase. In one embodiment, the human α 1,3 -fucosyltransferase is recombinantly prepared. In some embodiment, the fucosyltransferase enzyme is not a human fucosyltransferase. In some embodiment, the fucosyltransferase enzyme is an *H. pylori* fucosyltransferase. In one embodiment, the *H. pylori* fucosyltransferase is *H. pylori* α 1,3-fucosyltransferase. In one embodiment, the *H. pylori* fucosyltransferase is *H. pylori* α 1,3/1,4 -fucosyltransferase. In some examples of the method, the enzyme is a sialyltransferase and the tagged substrate is CMP-Neu5Ac conjugated to a tag. In some examples, the tag is biotin. In some examples, the sialyltransferase enzyme is a human sialyltransferase. In some examples, the sialyltransferase enzyme is human ST6GAL1. In one embodiment, the human ST6GAL1 is recombinantly prepared. In some embodiment, the sialyltransferase enzyme is human ST6GalNAc1. In one embodiment, the human ST6GalNAc1 is recombinantly prepared. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST). In some examples, the conjugation of the enzyme on the cell surface of the dendritic cell is via chemical conjugation. In some examples, the chemical conjugation is performed as in Method 2, disclosed in Example 7, herein using a fucosyltransferase or a sialyltransferase. In some examples, the conjugation of the enzyme on the cell surface of the dendritic cell is via enzymatic conjugation of the enzyme to the cell surface. In some examples, the enzymatic conjugation is via fucosylation of the cell with a GDP-Fuc-Enzyme conjugate according to Method 1, disclosed in Example 7, herein. In particular examples, the fucosylation enzyme catalyzing the conjugation of the enzyme to the surface of the cell is *H. pylori* α1,3fucosyltransferase enzyme. In some examples, the fucosylation enzyme catalyzing the conjugation of the enzyme to the surface of the cell is human α1,3fucosyltransferase (FUT6) or *H. pylori* α1,3/4fucosyltransferase). In some examples, the enzymatic conjugation is performed as described in Method 1, disclosed in Example 7, herein, except that instead of using a fucosylation reaction with a fucosyltransferase enzyme and a GDP-Fuc-Enzyme conjugate as the donor nucleotide substrate to conjugate the enzyme onto the surface of the cell, the method utilizes a sialyltransferase enzyme according to and a CMP-NeuAc-Enzyme to conjugate the enzyme onto the surface of the cell and instead of using tumor cell lysates to identify TSA reactive T cells, the method comprises the use of diseased tissue biopsy cell lysate from a subject having an autoimmune disease to identify auto-reactive T cells. In some examples, the sialyltransferase enzyme is a human sialyltransferase. In some examples, the sialyltransferase enzyme is human ST6GAL1 human ST6GalNAc1. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST).

In some examples, the present disclosure provides a method for identifying and enriching for auto-reactive T cells from tissue infiltrating lymphocytes (TiILs) or circulating T cells, the method comprising
(a) providing a bait cell that is a dendritic cell engineered to comprise on its cell surface a suitable enzyme for catalyzing an interaction-dependent labeling reaction on a prey cell;
(b) incubating the bait cell with one or more autoantigens or a source of one or more autoantigens (e.g., a diseased tissue biopsy cell lysate from a subject having an autoimmune disease) in order to prime the dendritic cell with one or more autoantigen;
(c) contacting the bait cell with a population of TiILs (e.g., comprised in a population of cells obtained from a diseased tissue biopsy from the subject having an autoimmune disease); wherein the population comprises at least one auto-reactive T cell; and wherein the contacting occurs in the presence of a suitable tagged substrate for the enzyme and the bait cell; and
(d) isolating any cells comprising the tagged substrate from the population of TiILs or circulating T cells that do not contain the tagged substrate.

The isolating step (d) may be performed in any suitable way. For example, in one embodiment, the isolating step (d) is performed via a pulldown assay using a solid substrate, e.g., beads, conjugated to a binding moiety having specificity for the tag on the substrate. In one preferred embodiment, the isolating step (d) is performed using FACS.

In some examples, the method further comprises enriching for the tagged auto-reactive T cells by sorting the tagged cells by FACS for the presence of the tag alone or in combination with one or more additional cell marker to enrich for the desired cell population.

For example, in some examples, the cells are sorted to enrich for tagged cells expressing T cell markers (i.e., enrich for CD8+ cells for cytotoxic T-cells; CD4+, CD25+, FOXP3+ for T cells that are prospective antigen specific regulatory T cells); and/or to exclude cells expressing DC markers (i.e., enrich for CD45.1 ^{-/-} cells). In some examples of the method, the enzyme is a fucosyltransferase and the tagged substrate is GDP-fucose conjugated to a tag. In some examples, the tag is any one of the tags disclosed herein. In some examples, the tag is biotin. In some embodiment, the fucosyltransferase enzyme is a human fucosyltransferase. In some embodiment, the fucosyltransferase enzyme is human α 1,3-fucosyltransferase. In one embodiment, the human α 1,3 -fucosyltransferase is recombinantly prepared. In some embodiment, the fucosyltransferase enzyme is not a human fucosyltransferase. In some embodiment, the fucosyltransferase enzyme is an *H. pylori* fucosyltransferase. In one embodiment, the *H. pylori* fucosyltransferase is *H. pylori* α 1,3-fucosyltransferase. In one embodiment, the *H. pylori* fucosyltransferase is *H. pylori* α 1,3/1,4 -fucosyltransferase. In some examples of the method, the enzyme is a sialyltransferase and the tagged substrate is CMP-Neu5Ac conjugated to a tag. In some examples, the tag is biotin. In some examples, the sialyltransferase enzyme is a human sialyltransferase. In some examples, the sialyltransferase enzyme is human ST6GAL1. In one embodiment, the human ST6GAL1 is recombinantly prepared. In some embodiment, the sialyltransferase enzyme is human ST6GalNAc1. In one embodiment, the human ST6GalNAc1 is recombinantly prepared. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST). In some examples, the conjugation of the enzyme on the cell surface of the dendritic cell is via chemical conjugation. In some examples, the chemical conjugation is performed as in Method 2, disclosed in Example 7, herein using a fucosyltransferase or a sialyltransferase. In some examples, the conjugation of the enzyme on the cell surface of the dendritic cell is via enzymatic conjugation of the enzyme to the cell surface. In some examples, the enzymatic conjugation is via fucosylation of the cell with a GDP-Fuc-Enzyme conjugate according to Method 1, disclosed in Example 7, herein. In particular examples, the fucosylation enzyme catalyzing the conjugation of the enzyme to the surface of the cell is *H. pylori* α1,3fucosyltransferase enzyme. In some examples, the fucosylation enzyme catalyzing the conjugation of the enzyme to the surface of the cell is human α1,3fucosyltransferase (FUT6) or *H. pylori* α1,3/4fucosyltransferase). In some examples, the enzymatic conjugation is performed as described in Method 1, disclosed in Example 7, herein, except that instead of using a fucosylation reaction with a fucosyltransferase enzyme and a GDP-Fuc-Enzyme conjugate as the donor nucleotide substrate to conjugate the enzyme onto the surface of the cell, the method utilizes a sialyltransferase enzyme according to and a CMP-NeuAc-Enzyme to conjugate the enzyme onto the surface of the cell and instead of using tumor cell lysates to identify TSA reactive T cells, the method comprises the use of diseased tissue biopsy cell lysate from a subject having an autoimmune disease to identify auto-reactive T cells. In some examples, the sialyltransferase enzyme is a human sialyltransferase. In some examples, the sialyltransferase enzyme is human ST6GAL1 human ST6GalNAc1. In some examples, the sialyltransferase enzyme is not a human sialyltransferase. In some examples, the sialyltransferase enzyme is *Pasteurella multocida* α (2,3) sialyltransferase M144D mutant (Pm2,3ST-M144D) or *Photobacterium damsela* α (2,6) sialyltransferase (Pd2,6ST).

Additionally, in some examples, the methods disclosed above for tagging and isolating TSA reactive and autoreactive T cells are easily adapted to apply to any antigen without undue experimentation. For example, in order to tag and isolate T cells with specificity for another antigens, e.g., pathogenic antigens such as bacterial or viral antigens, one need only follow the same protocols set forth above, but use relevant antigen sources to prime the bait cell dendritic cells and then use appropriate sources of tissue infiltrating or circulating T cells from appropriate sources such that these sources contain one or more T cells with TCR-specificity for the primed antigens. So, briefly, to identify T cells with virus specific reactivity, one need only incubate a bait cell dendritic cell with either virus specific antigens or a source of the same (e.g., diseased tissue cell lysate) to prime the DCs, then the primed DC-enzyme conjugates are mixed with the tissue infiltrating or circulating T cells in the presence of the suitable tagged substrate and tagged T cells are detected and enriched for as described above. In various examples, the same applies for any antigen of interest with only minor changes to the methods described with respect to the methods of isolating suitable antigens/antigen sources for priming dendritic cells and minor changes to the methods of isolating relevant populations of T cells that comprise antigen-specific T cells to be identified and isolated using the interaction-dependent labeling methods described herein.

For example, in order to identify prospective TSA-specific T cells for hematologic malignancies, such as AML; ALL; CLL; the following protocol may in some examples be followed. Cancer cells are isolated from a patient (bone marrow or blood) and lysed for priming iDCs derived from the same patient. The primed iDCs or un-primed (control) iDCs are stained with CellTracker^{™} Green CMFDA, and conjugated with fucosyltransferase (FT) on the cell surface, and cultured with autologous PBMC of the same patient at different ratios for 1-2 hours. Then GDP-Fuc-biotin (50 µM) is added and incubated for another 30 min. After quenching the reaction with N-Acetyl-D-lactosamine (LacNAc), the cell mixture is stained with Alexa Fluor 647-streptavidin and cell identity markers, and subjected to flow cytometry analysis. CD4+ (Foxp3-) and or CD8+ T cells that are also Alexa Fluor 647+ will be isolated as prospective TSA-specific T cells.

Similarly, to identify prospective TSA-specific T cells for solid tumors (human, the following protocol may in some emodiments be followed). Tumors isolated from a patient are cross-cut into small pieces, minced to prepare tumor lysates or prepare single cell suspensions. Preparation of single cell suspensions are performed using pre-established Ficoll-paque density gradient centrifugation protocols that are well-known in the art. *See, e.g.,* Tan Y.S., Lei Y.L. (2019) Isolation of Tumor-Infiltrating Lymphocytes by Ficoll-Paque Density Gradient Centrifugation. In: Allen I. (eds) Mouse Models of Innate Immunity. Methods in Molecular Biology, vol 1960. Humana Press, New York, NY. Tumor lysates are used to prime iDCs derived from the same patient. The primed iDCs or unprimed (negative control) iDCs or iDC primed with normal tissue lysates (negative control) are stained with CellTracker^{™} Green CMFDA, conjugated with fucosyltransferase (FT) on the cell surface, and cultured with autologous single cell suspension isolated from the tumor at different ratios (autologous single cells:DC = 5:1 to 10:1) for 1-2 hours. Then GDP-Fuc-biotin (50 µM) is added and incubated for another 30 min. After quenching the reaction with LacNAc, the cell mixture is stained with Alexa Fluor 647-streptavidin and cell identity and phenotype markers, and subjected to FACs. CD3+/CD4+/CD25-/Alexa Fluor 647+ or CD3+/CD8+/PD-1+/Alexa Fluor 647+ cells are isolated as prospective TSA-specific CD4 or CD8 T cells, respectively.

### V. T cells, T cell Receptors and Pharmaceutical Compositions Containing the Same

In some examples, the present disclosure provides an *in vitro* or *ex vivo* expanded population of antigen-reactive T cells, wherein the T cells were isolated using a method described herein. The antigen reactive T cells may in some examples recognize an antigen from a cancer, a pathogenic infection, autoimmune disease, inflammatory disease, or a genetic disorder.

In some examplesexamples, the present disclosure provides an *in vitro* or *ex vivo* expanded population of TSA reactive T cells, wherein the T cells were isolated using a method described herein.

In one embodiment, the *in vitro* or *ex vivo* expanded population of TSA reactive T cells was isolated using a method described in Example 5 or 6. In some examples, the TSA reactive T cells are substantially or entirely CD4+. In some examples, the TSA reactive T cells are substantially or entirely CD8+. The TSA reactive T cells, and expanded populations of the same, may be formulated into a pharmaceutical composition. The pharmaceutical composition may be any composition disclosed herein.

In particular examples, the T cells express a TSA specific T cell receptor (TCR), or an antigen-binding fragment thereof, comprising a Vα and a Vβ derived from a wild type T cell receptor, wherein the Vα and Vβ each comprise a complementarity determining region 1 (CDR-1), a complementarity determining region 2 (CDR-2), and a complementarity determining region 3 (CDR-3), wherein the Vα CDR-3 comprises an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

In some examples, such T cells are engineered to express the Vα CDR-3 sequence selected from the above group. The engineered T cell may express a chimeric TCR comprising the Vα CDR-3 sequence selected from the above group. In some examples, the present disclosure provides pharmaceutical compositions comprising one or more TSA reactive T cells and one or more pharmaceutically acceptable carrier, salt, vehicle, and/or excipient. The pharmaceutical compositions may be endotoxin-free or substantially endotoxin-free.

In some examples, the present disclosure provides pharmaceutical compositions comprising one or more TSA reactive T cells, all, or substantially all of which (i) are conjugated to one or more tagged fucose or tagged NeuAc moiety on their surface; (ii) are CD4+ or CD8+; and, optionally, are PD-1+ and CD45.1-/- and a pharmaceutically acceptable carrier or excipient or pharmaceutical compositions comprising an expanded population of such TSA reactive T cells. In some examples, all or substantially all of the the expanded population does not comprise the tag. Rather, in such examples, the tagging methods described herein may be utilized for isolating the proper populations of TSA reactive T cells for expansion. In one embodiment, the pharmaceutical composition is for the treatment of a disease in a subject. In one embodiment, provided herein is a method of treating, decreasing, inhibiting, or reducing cancer in a subject, comprising: administering to the subject a therapeutically effective dosage of a pharmaceutical composition comprising one or more TSA reactive T cells, all, or substantially all of which (i) are conjugated to one or more tagged fucose or tagged NeuAc moiety on their surface; (ii) are CD4+ or CD8+; and, optionally, are PD-1+ and CD45.1-/- and a pharmaceutically acceptable carrier salt, vehicle, and/or excipient. The pharmaceutical compositions may be endotoxin-free or substantially endotoxin-free. In one embodiment, the cancer is selected from a melanoma tumor; a breast cancer tumor; and a tumor selected from the group consisting of Pilocytic astrocytoma; AML; ALL; Thyroid; Kidney chromophobe; CLL; Medulloblastoma; Neuroblastoma; Glioma low grade; Glioblastoma; Prostate; Ovary; Myeloma; Pancreas; Kidney papillary; Lymphoma B-cell; Kidney clear cell; Head and neck; Liver; Cervix; Uterus; Bladder; Colorectum; Lung small cell; Esophagus; Stomach; Lung adeno; and Lung squamous. In one embodiment, the cancer is breast cancer.

In some examples, the TSA reactive T cells are substantially or entirely CD8+. In some examples, the TSA reactive T cells are substantially or entirely CD4+.

In some examples, the present disclosure provides an *in vitro* or *ex vivo* expanded population of TSA reactive T cells, wherein the T cells were isolated using a method described herein. In one embodiment, the *in vitro* or *ex vivo* expanded population of TSA reactive T cells was isolated using a method described in Example 5 or 6. In some examples, the TSA reactive T cells are substantially or entirely CD4+. In some examples, the TSA reactive T cells are substantially or entirely CD8+.

In some examples, the present disclosure provides a TSA specific T cell receptor (TCR), or an antigen-binding fragment thereof, isolated using a method described herein. The TSA specific T cell receptor (TCR), or an antigen-binding fragment thereof, may be isolated using a method described in Example 7. In particular examples, the present disclosure provides a TSA specific T cell receptor (TCR), or an antigen-binding fragment thereof, comprising a Vα and a Vβ derived from a wild type T cell receptor, wherein the Vα and Vβ each comprise a complementarity determining region 1 (CDR-1), a complementarity determining region 2 (CDR-2), and a complementarity determining region 3 (CDR-3), wherein the Vα CDR-3 comprises an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

The TSA reactive T cell receptor may be a chimeric T cell receptor.

The TSA reactive T cell receptor may be a single chain T cell receptor. The TSA reactive T cell receptor may be a single chain T cell receptor comprising the structure Vα-linker-Vβ or Vβ-linker-Vα. The single chain TCR may comprise a Vα CDR-3 comprising an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

The TSA reactive T cell receptor may be a bispecific T cell receptor comprising the TCR or an antigen binding fragment thereof and an antibody. The TCR may comprise a Vα CDR-3 comprising an amino acid sequence selected from the group consisting of:

| | | | |
|---|---|---|---|
| ASGTDYAEQF | SEQ ID NO: 29 | AWSLRLGGTYEQY | SEQ ID NO: 49 |
| ASSPQLGGRREQY | SEQ ID NO: 30 | ASSLTISNERLF | SEQ ID NO: 50 |
| ASSIGTANTEVF | SEQ ID NO: 31 | ASSFWGRQDTQY | SEQ ID NO: 51 |
| AWSGNTEVF | SEQ ID NO: 32 | ASSFWGRGNTLY | SEQ ID NO: 52 |
| ASRSGGSAETLY | SEQ ID NO: 33 | ASGGPGQGFAEQF | SEQ ID NO: 53 |
| ASSFVSSAETLY | SEQ ID NO: 34 | ASSPTGAIMNS | SEQ ID NO: 54 |
| ASSSDRGSAETLY | SEQ ID NO: 35 | ASSLYRDRGYAEQF | SEQ ID NO: 55 |
| ASSDRGGQDTQY | SEQ ID NO: 36 | AWSLPLGQSYEQY | SEQ ID NO: 56 |
| ASSSGTDTEVF | SEQ ID NO: 37 | ASSFRGYEQY | SEQ ID NO: 57 |
| AWRDWGGAEQF | SEQ ID NO: 38 | ASSDDTYEQY | SEQ ID NO: 58 |
| ASSGLGETLY | SEQ ID NO: 39 | ASSDGDRYEQY | SEQ ID NO: 59 |
| ASSLDNSGNTLY | SEQ ID NO: 40 | ASSDNYNSPLY | SEQ ID NO: 60 |
| ASSLDRVQDTQY | SEQ ID NO: 41 | ASRDWGGRAETLY | SEQ ID NO: 61 |
| AWTEVF | SEQ ID NO: 42 | ASSLELGGREQY | SEQ ID NO: 62 |
| ASSFGQNYAEQF | SEQ ID NO: 43 | ASSDPGAANTEVF | SEQ ID NO: 63 |
| ASSDGTSAETLY | SEQ ID NO: 44 | ASSLDGADSDYT | SEQ ID NO: 64 |
| ASRPGSAETLY | SEQ ID NO: 45 | ASSMNNERLF | SEQ ID NO: 65 |
| ASSPQLYEQY | SEQ ID NO: 46 | ASSQVGGASETLY | SEQ ID NO: 66 |
| ASSDGLGVNQDTQY | SEQ ID NO: 47 | ASGDATDYSGNTLY | SEQ ID NO: 67 |
| ASSDGGGGTEVF | SEQ ID NO: 48 | ASGEGPANTEVF | SEQ ID NO: 68 |

The present disclosure also provides pharmaceutical compositions comprising the TCRs, chimeric TCRs, single chain TCRs, and bispecific TCRs disclosed herein. In one embodiment, the such a pharmaceutical composition comprises a TCR, single chain TCR and/or a bispecific TCR disdclosed herein and one or more pharmaceuticlaly acceptable carrier salt, vehicle, and/or excipient. The pharmaceutical compositions may be endotoxin-free or substantially endotoxin-free.

### VI. Methods of Treatment.

The present disclosure also provides methods of treating diseases and conditions using an antigen-reactive cell isolated via the methods described herein or pharmaceutical compositions comprising the same. The present disclosure also provides methods of treating diseases and conditions using a TCR, a chimeric TCR, a single chain TCRs, and/or a bispecific TCRs disclosed above. In some examples, any immune disease or disorder may be treated with the compositions and methods disclosed herein. In some instances present disclosure also provides methods of treating diseases and conditions using an antigen-reactive T cell (or a population of antigen-reactive T cells) isolated via the methods described herein or pharmaceutical compositions comprising the same. In some examples, any disease or disorder that results in the expression of a disease-specific antigen on the surface of a cell may be treated with an antigen-reactive T cell isolated via the methods described herein or a pharmaceutical composition comprising the same. The methods may comprise administering to a subject in need thereof one or more dfifferent populations of T cells isolated and expanded via the methods described herein. For example, a population of T cells directed to a particular TSA may be identified via the methods disclosed herein and expanded and another population of T cells directed to another different TSA may be identified via the methods disclosed herein and expanded and both populations may be administered to a subject in need thereof.

In some instances present disclosure provides methods of treating diseases and conditions using an antigen-reactive B cell (or a population of antigen-reactive B cells) isolated via the methods described herein or pharmaceutical compositions comprising the same. Such antigen-reactive B cells are known in the art and have been shown to be effective in treating various diseases and disorders including cancer and influenza, *see e.g.,* Wennhold, K., et al., Cancer Immunol Res September 1 2017 (5)(9) 730-743; DOI:10.1158/2326-6066; and Dougan SK, et al., Nature. 2013 Nov 21;503(7476):406-9. doi: 10.1038/nature12637.

In some examples, any disease or disorder that results in the expression of a disease-specific antigen on the surface of a cell may be treated with an antigen-reactive B cell isolated via the methods described herein or a pharmaceutical composition comprising the same. The methods may comprise administering to a subject in need thereof one or more different populations of B cells isolated and expanded via the methods described herein and methods known in the art. For example, B cell expansion kits are available commercially and are well known (e.g., CellXVivo Human B Cell Expansion Kit, R&D Systems, Minneapolis, MN; *see also,* von Bergwelt-Baildon MS., et al., Blood 2002;99:3319-25; Schultz JL, et al., J Clin Invest 1997;100:2757-65; and Lapointe R., et al., Cancer Res 2003;63:2836-43). For example, a population of B cells directed to a particular TSA may be identified via the methods disclosed herein and expanded and another population of B cells directed to another different TSA may be identified via the methods disclosed herein and expanded and both populations may be administered to a subject in need thereof.

Similarly, the methods may comprise administering to a subject in need thereof one or more different populations of T cells isolated and expanded via the methods described herein and one or more different populations of B cells isolated and expanded via the methods described herein.

The methods may comprise administering about 1, 2, 3, 4, 5, 6, 7, 8, 9, 10, 12, 15, 20, 24, 30, 35, 48, 50, 55, 60, 65, 70, 75, 80, 85, 90, 96, 100, 120, 150, 200, 300, 384, 400, 500, 600, 700, 800, 900, 1000 or more populations of antigen-reactive T cells isolated and expanded via the methods described herein. The methods may comprise administering about 1, 2, 3, 4, 5, 6, 7, 8, 9, 10, 12, 15, 20, 24, 30, 35, 48, 50, 55, 60, 65, 70, 75, 80, 85, 90, 96, 100, 120, 150, 200, 300, 384, 400, 500, 600, 700, 800, 900, 1000 or more populations of antigen-reactive B cells isolated and expanded via the methods described herein. Administering the antigen-reactive T cell or population of antigen-reactive T cells or antigen-reactive B cells or population of antigen reactive B cells may comprise intravenous delivery. Administering the antigen-reactive T cell or population of antigen-reactive T cells or antigen-reactive B cells or population of antigen reactive B cells may comprise intraperitoneal delivery. Administering the antigen-reactive T cell or population of antigen-reactive T cells or antigen-reactive B cells or population of antigen reactive B cells may comprise intravenous delivery and intraperitoneal delivery. Administering the antigen-reactive T cell or population of antigen-reactive T cells or antigen-reactive B cells or population of antigen reactive B cells cell may occur once. Administering antigen-reactive T cell or population of antigen-reactive T cells or antigen-reactive B cells or population of antigen reactive B cells may occur more than once (*e.g.,* repeat injection).

In one embodiment, a TSA reactive T cell identified and enriched via a method described herein is expanded and administered to a patient to treat a cancer. In one embodiment, two different TSA reactive T cells are identified and individually enriched for via a method described herein and the separate T cells are expanded and administered to a patient to treat a cell proliferative disorder. In one embodiment, two different TSA reactive T cells are identified and individually enriched for via a method described herein and the separate T cells are expanded and administered to a patient to treat cancer. In one embodiment, a TSA reactive B cell identified and enriched via a method described herein is expanded and administered to a patient to treat a cancer. In one embodiment, two different TSA reactive B cells are identified and individually enriched for via a method described herein and the separate B cells are expanded and administered to a patient to treat a cell proliferative disorder. In one embodiment, two different TSA reactive B cells are identified and individually enriched for via a method described herein and the separate B cells are expanded and administered to a patient to treat cancer. In some examples, the cancer is selected from a melanoma tumor; a breast cancer tumor; and a tumor selected from the group consisting of Pilocytic astrocytoma; AML; ALL; Thyroid; Kidney chromophobe; CLL; Medulloblastoma; Neuroblastoma; Glioma low grade; Glioblastoma; Prostate; Ovary; Myeloma; Pancreas; Kidney papillary; Lymphoma B-cell; Kidney clear cell; Head and neck; Liver; Cervix; Uterus; Bladder; Colorectum; Lung small cell; Esophagus; Stomach; Lung adeno; and Lung squamous. In some examples, the administration is as a pharmaceutical composition.

In one embodiment, an antigen reactive T cell or a population of antigen reactive T cells are identified and enriched via a method described herein and are expanded and administered to a patient to treat a disease or condition. The disease or condition may be a cell proliferative disorder. The cell proliferative disorder may be selected from a solid tumor, a lymphoma, a leukemia and a liposarcoma. The cell proliferative disorder may be acute, chronic, recurrent, refractory, accelerated, in remission, stage I, stage II, stage III, stage IV, juvenile or adult. The cell proliferative disorder may be selected from myelogenous leukemia, lymphoblastic leukemia, myeloid leukemia, an acute myeloid leukemia, myelomonocytic leukemia, neutrophilic leukemia, myelodysplastic syndrome, B-cell lymphoma, burkitt lymphoma, large cell lymphoma, mixed cell lymphoma, follicular lymphoma, mantle cell lymphoma, hodgkin lymphoma, recurrent small lymphocytic lymphoma, hairy cell leukemia, multiple myeloma, basophilic leukemia, eosinophilic leukemia, megakaryoblastic leukemia, monoblastic leukemia, monocytic leukemia, erythroleukemia, erythroid leukemia and hepatocellular carcinoma. The cell proliferative disorder may comprise a hematological malignancy. The hematological malignancy may comprise a B cell malignancy. The cell proliferative disorder may comprise a chronic lymphocytic leukemia. The cell proliferative disorder may comprise an acute lymphoblastic leukemia. The cell proliferative disorder may comprise a CD19-positive Burkitt's lymphoma.

The disease or condition may be a cancer, a pathogenic infection, autoimmune disease, inflammatory disease, or genetic disorder.

In some instances, the one or more diseases comprise a cancer. The cancer may comprise a recurrent and/or refractory cancer. Examples of cancers include, but are not limited to, sarcomas, carcinomas, lymphomas or leukemias.

The cancer may comprise a neuroendocrine cancer. The cancer may comprise a pancreatic cancer. The cancer may comprise an exocrine pancreatic cancer. The cancer may comprise a thyroid cancer. The thyroid cancer may comprise a medullary thyroid cancer. The cancer may comprise a prostate cancer.

The cancer may comprise an epithelial cancer. The cancer may comprise a breast cancer. The cancer may comprise an endometrial cancer. The cancer may comprise an ovarian cancer. The ovarian cancer may comprise a stromal ovarian cancer. The cancer may comprise a cervical cancer.

The cancer may comprise a skin cancer. The skin cancer may comprise a neo-angiogenic skin cancer. The skin cancer may comprise a melanoma.

The cancer may comprise a kidney cancer.

The cancer may comprise a lung cancer. The lung cancer may comprise a small cell lung cancer. The lung cancer may comprise a non-small cell lung cancer.

The cancer may comprise a colorectal cancer. The cancer may comprise a gastric cancer. The cancer may comprise a colon cancer.

The cancer may comprise a brain cancer. The brain cancer may comprise a brain tumor. The cancer may comprise a glioblastoma. The cancer may comprise an astrocytoma.

The cancer may comprise a blood cancer. The blood cancer may comprise a leukemia. The leukemia may comprise a myeloid leukemia. The cancer may comprise a lymphoma. The lymphoma may comprise a non-Hodgkin's lymphoma.

The cancer may comprise a sarcoma. The sarcoma may comprise an Ewing's sarcoma.

Sarcomas are cancers of the bone, cartilage, fat, muscle, blood vessels, or other connective or supportive tissue. Sarcomas include, but are not limited to, bone cancer, fibrosarcoma, chondrosarcoma, Ewing's sarcoma, malignant hemangioendothelioma, malignant schwannoma, bilateral vestibular schwannoma, osteosarcoma, soft tissue sarcomas (e.g., alveolar soft part sarcoma, angiosarcoma, cystosarcoma phylloides, dermatofibrosarcoma, desmoid tumor, epithelioid sarcoma, extraskeletal osteosarcoma, fibrosarcoma, hemangiopericytoma, hemangiosarcoma, Kaposi's sarcoma, leiomyosarcoma, liposarcoma, lymphangiosarcoma, lymphosarcoma, malignant fibrous histiocytoma, neurofibrosarcoma, rhabdomyosarcoma, and synovial sarcoma).

Carcinomas are cancers that begin in the epithelial cells, which are cells that cover the surface of the body, produce hormones, and make up glands. By way of non-limiting example, carcinomas include breast cancer, pancreatic cancer, lung cancer, colon cancer, colorectal cancer, rectal cancer, kidney cancer, bladder cancer, stomach cancer, prostate cancer, liver cancer, ovarian cancer, brain cancer, vaginal cancer, vulvar cancer, uterine cancer, oral cancer, penile cancer, testicular cancer, esophageal cancer, skin cancer, cancer of the fallopian tubes, head and neck cancer, gastrointestinal stromal cancer, adenocarcinoma, cutaneous or intraocular melanoma, cancer of the anal region, cancer of the small intestine, cancer of the endocrine system, cancer of the thyroid gland, cancer of the parathyroid gland, cancer of the adrenal gland, cancer of the urethra, cancer of the renal pelvis, cancer of the ureter, cancer of the endometrium, cancer of the cervix, cancer of the pituitary gland, neoplasms of the central nervous system (CNS), primary CNS lymphoma, brain stem glioma, and spinal axis tumors. In some instances, the cancer is a skin cancer, such as a basal cell carcinoma, squamous, melanoma, nonmelanoma, or actinic (solar) keratosis.

In some instances, the cancer is a lung cancer. Lung cancer may start in the airways that branch off the trachea to supply the lungs (bronchi) or the small air sacs of the lung (the alveoli). Lung cancers include non-small cell lung carcinoma (NSCLC), small cell lung carcinoma, and mesotheliomia. Examples of NSCLC include squamous cell carcinoma, adenocarcinoma, and large cell carcinoma. The mesothelioma may be a cancerous tumor of the lining of the lung and chest cavity (pleura) or lining of the abdomen (peritoneum). The mesothelioma may be due to asbestos exposure. The cancer may be a brain cancer, such as a glioblastoma.

Alternatively, the cancer may be a central nervous system (CNS) tumor. CNS tumors may be classified as gliomas or nongliomas. The glioma may be malignant glioma, high grade glioma, diffuse intrinsic pontine glioma. Examples of gliomas include astrocytomas, oligodendrogliomas (or mixtures of oligodendroglioma and astocytoma elements), and ependymomas. Astrocytomas include, but are not limited to, low-grade astrocytomas, anaplastic astrocytomas, glioblastoma multiforme, pilocytic astrocytoma, pleomorphic xanthoastrocytoma, and subependymal giant cell astrocytoma. Oligodendrogliomas include low-grade oligodendrogliomas (or oligoastrocytomas) and anaplastic oligodendriogliomas. Nongliomas include meningiomas, pituitary adenomas, primary CNS lymphomas, and medulloblastomas. In some instances, the cancer is a meningioma.

The leukemia may be an acute lymphocytic leukemia, acute myelocytic leukemia, chronic lymphocytic leukemia, or chronic myelocytic leukemia. Additional types of leukemias include hairy cell leukemia, chronic myelomonocytic leukemia, and juvenile myelomonocytic leukemia.

Lymphomas are cancers of the lymphocytes and may develop from either B or T lymphocytes. The two major types of lymphoma are Hodgkin's lymphoma, previously known as Hodgkin's disease, and non-Hodgkin's lymphoma. Hodgkin's lymphoma is marked by the presence of the Reed-Sternberg cell. Non-Hodgkin's lymphomas are all lymphomas which are not Hodgkin's lymphoma. Non-Hodgkin lymphomas may be indolent lymphomas and aggressive lymphomas. Non-Hodgkin's lymphomas include, but are not limited to, diffuse large B cell lymphoma, follicular lymphoma, mucosa-associated lymphatic tissue lymphoma (MALT), small cell lymphocytic lymphoma, mantle cell lymphoma, Burkitt's lymphoma, mediastinal large B cell lymphoma, Waldenström macroglobulinemia, nodal marginal zone B cell lymphoma (NMZL), splenic marginal zone lymphoma (SMZL), extranodal marginal zone B cell lymphoma, intravascular large B cell lymphoma, primary effusion lymphoma, and lymphomatoid granulomatosis.

The cancer may comprise a solid tumor. The cancer may comprise a sarcoma. The cancer may be selected from a group consisting of a bladder cancer, a breast cancer, a colon cancer, a rectal cancer, an endometrial cancer, a kidney cancer, a lung cancer, melanoma, a myeloma, a thyroid cancer, a pancreatic cancer, a glioma, a malignant glioma of the brain, a glioblastoma, an ovarian cancer, and a prostate cancer. The cancer may have non-uniform antigen expression. The cancer may have modulated antigen expression. The antigen may be a surface antigen. The cancer may not comprise a myeloma. The cancer may not comprise a melanoma. The cancer may not comprise a colon cancer. The cancer may be acute lymphoblastic leukemia (ALL). The cancer may be relapsed ALL. The cancer may be refractory ALL. The cancer may be relapsed, refractory ALL. The cancer may be chronic lymphocytic leukemia (CLL). The cancer may be relapsed CLL. The cancer may be refractory CLL. The cancer may be relapsed, refractory CLL.

The cancer may comprise a breast cancer. The breast cancer may be triple positive breast cancer (estrogen receptor, progesterone receptor and Her2 positive). The breast cancer may be triple negative breast cancer (estrogen receptor, progesterone receptor and Her2 negative). The breast cancer may be estrogen receptor positive. The breast cancer may be estrogen receptor negative. The breast cancer may be progesterone receptor positive. The breast cancer may be progesterone receptor negative. The breast cancer may comprise a Her2 negative breast cancer. The breast cancer may comprise a low-expressing Her2 breast cancer. The breast cancer may comprise a Her2 positive breast cancer. Cell lines expressing Her2 have been well-characterized for antigen density, reflecting clinical immunohistochemistry characterization which classifies malignancies as 0 (<20,000 Her2 antigens per cell), 1+ (100,000 Her2 antigens per cell), 2+ (500,000 Her2 antigens per cell), and 3+ (>2,000,000 Her2 antigens per cell). The present disclosure provides for methods of treating breast cancers of these classifications. The breast cancer may comprise a breast cancer classified as Her2 0. The breast cancer may comprise a breast cancer classified as Her2 1+. The breast cancer may comprise a breast cancer classified as Her2 2+. The breast cancer may comprise a breast cancer classified as a Her2 3+.

The disease or condition may be a pathogenic infection. Pathogenic infections may be caused by one or more pathogens. In some instances, the pathogen is a bacterium, fungi, virus, or protozoan.

Exemplary pathogens include but are not limited to: Bordetella, Borrelia, Brucella, Campylobacter, Chlamydia, Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio, or Yersinia. In some cases, the disease or condition caused by the pathogen is tuberculosis and the heterogeneous sample comprises foreign molecules derived from the bacterium Mycobacterium tuberculosis and molecules derived from the subject. In some instances, the disease or condition is caused by a bacterium is tuberculosis, pneumonia, which may be caused by bacteria such as Streptococcus and Pseudomonas, a foodborne illness, which may be caused by bacteria such as Shigella, Campylobacter and Salmonella, and an infection such as tetanus, typhoid fever, diphtheria, syphilis and leprosy. The disease or condition may be bacterial vaginosis, a disease of the vagina caused by an imbalance of naturally occurring bacterial flora. Alternatively, the disease or condition is a bacterial meningitis, a bacterial inflammation of the meninges (e.g., the protective membranes covering the brain and spinal cord). Other diseases or conditions caused by bacteria include, but are not limited to, bacterial pneumonia, a urinary tract infection, bacterial gastroenteritis, and bacterial skin infection. Examples of bacterial skin infections include, but are not limited to, impetigo which may be caused by Staphylococcus aureus or Streptococcus pyogenes; erysipelas which may be caused by a streptococcus bacterial infection of the deep epidermis with lymphatic spread; and cellulitis which may be caused by normal skin flora or by exogenous bacteria.

The pathogen may be a fungus, such as, Candida, Aspergillus, Cryptococcus, Histoplasma, Pneumocystis, and Stachybotrys. Examples of diseases or conditions caused by a fungus include, but are not limited to, jock itch, yeast infection, ringworm, and athlete's foot.

The pathogen may be a virus. Examples of viruses include, but are not limited to, adenovirus, coxsackievirus, Epstein-Barr virus, Hepatitis virus (e.g., Hepatitis A, B, and C), herpes simplex virus (type 1 and 2), cytomegalovirus, herpes virus, HIV, influenza virus, measles virus, mumps virus, papillomavirus, parainfluenza virus, poliovirus, respiratory syncytial virus, rubella virus, and varicella-zoster virus. Examples of diseases or conditions caused by viruses include, but are not limited to, cold, flu, hepatitis, AIDS, chicken pox, rubella, mumps, measles, warts, and poliomyelitis.

The pathogen may be a protozoan, such as Acanthamoeba (e.g., A. astronyxis, A. castellanii, A. culbertsoni, A. hatchetti, A. polyphaga, A. rhysodes, A. healyi, A. divionensis), Brachiola (e.g., B connori, B. vesicularum), Cryptosporidium (e.g., C. parvum), Cyclospora (e.g., C. cayetanensis), Encephalitozoon (e.g., E. cuniculi, E. hellem, E. intestinalis), Entamoeba (e.g., E. histolytica), Enterocytozoon (e.g., E. bieneusi), Giardia (e.g., G. lamblia), Isospora (e.g, I. belli), Microsporidium (e.g., M. africanum, M. ceylonensis), Naegleria (e.g., N. fowleri), Nosema (e.g., N. algerae, N. ocularum), Pleistophora, Trachipleistophora (e.g., T. anthropophthera, T. hominis), and Vittaforma (e.g., V. corneae).

The disease or condition may be an autoimmune disease or autoimmune related disease. An autoimmune disorder may be a malfunction of the body's immune system that causes the body to attack its own tissues. Examples of autoimmune diseases and autoimmune related diseases include, but are not limited to, Addison's disease, alopecia areata, ankylosing spondylitis, antiphospholipid syndrome (APS), autoimmune aplastic anemia, autoimmune hemolytic anemia, autoimmune hepatitis, autoimmune myocarditis, Behcet's disease, celiac sprue, Crohn's disease, dermatomyositis, eosinophilic fasciitis, erythema nodosum, giant cell arteritis (temporal arteritis), Goodpasture's syndrome, Graves' disease, Hashimoto's disease, idiopathic thrombocytopenic purpura (ITP), IgA nephropathy, juvenile arthritis, diabetes, juvenile diabetes, Kawasaki syndrome, Lambert-Eaton syndrome, lupus (SLE), mixed connective tissue disease (MCTD), multiple sclerosis, myasthenia gravis, pemphigus, polyarteritis nodosa, type I, II, & III autoimmune polyglandular syndromes, polymyalgia rheumatica, polymyositis, psoriasis, psoriatic arthritis, Reiter's syndrome, relapsing polychondritis, rheumatoid arthritis, sarcoidosis, scleroderma, Sjogren's syndrome, sperm & testicular autoimmunity, stiff person syndrome, Takayasu's arteritis, temporal arteritis/giant cell arteritis, ulcerative colitis, uveitis, vasculitis, vitiligo, and Wegener's granulomatosis.

The disease or condition may be an inflammatory disease. Examples of inflammatory diseases include, but are not limited to, alveolitis, amyloidosis, angiitis, ankylosing spondylitis, avascular necrosis, Basedow's disease, Bell's palsy, bursitis, carpal tunnel syndrome, celiac disease, cholangitis, chondromalacia patella, chronic active hepatitis, chronic fatigue syndrome, Cogan's syndrome, congenital hip dysplasia, costochondritis, Crohn's Disease, cystic fibrosis, De Quervain's tendinitis, diabetes associated arthritis, diffuse idiopathic skeletal hyperostosis, discoid lupus, Ehlers-Danlos syndrome, familial mediterranean fever, fascitis, fibrositis/fibromyalgia, frozen shoulder, ganglion cysts, giant cell arteritis, gout, Graves' Disease, HIV-associated rheumatic disease syndromes, hyperparathyroid associated arthritis, infectious arthritis, inflammatory bowel syndrome/ irritable bowel syndrome, juvenile rheumatoid arthritis, lyme disease, Marfan's Syndrome, Mikulicz's Disease, mixed connective tissue disease, multiple sclerosis, myofascial pain syndrome, osteoarthritis, osteomalacia, osteoporosis and corticosteroid-induced osteoporosis, Paget's Disease, palindromic rheumatism, Parkinson's Disease, Plummer's Disease, polymyalgia rheumatica, polymyositis, pseudogout, psoriatic arthritis, Raynaud's Phenomenon/Syndrome, Reiter's Syndrome, rheumatic fever, rheumatoid arthritis, sarcoidosis, sciatica (lumbar radiculopathy), scleroderma, scurvy, sickle cell arthritis, Sjogren's Syndrome, spinal stenosis, spondyloisthesis, Still's Disease, systemic lupus erythematosis, Takayasu's (Pulseless) Disease, Tendinitis, tennis elbow/golf elbow, thyroid associated arthritis, trigger finger, ulcerative colitis, Wegener's Granulomatosis, and Whipple's Disease.

The methods may comprise titrating the T cell or population of T cells for a desired effect. Titrating the T cell or population of T cells may enable antigen density discrimination. For example, the fatal on-target, off-tumor reactivity for Her2 targeted CAR-T cells to low levels of Her2 expression in the lung has tempered the application of CAR-T cells to solid tumors in the clinic. In the clinic this may be used to titrate therapy to an appropriate therapeutic index.

In one embodiment, a pathogenic-antigen reactive T cell identified and enriched via a method described herein is expanded and administered to a patient to treat a pathogenic infection. In some examples, the pathogen is a virus, parasite, or bacteria. In some examples, the T cell is administered as a pharmaceutical composition.

In one embodiment, an auto-antigen reactive regulatory T cell identified and enriched via a method described herein is expanded and administered to a patient to treat an autoimmune disease or disorder. In one embodiment, the disease is Polymyositis. In some examples, the T cell is administered as a pharmaceutical composition.

### EXAMPLES

The following examples relate to our discovery that fucosyltransferase-modified dendritic cells could induce antigen specific biotinylation with CD8+ and CD4+ T cells and the magnitude of biotin tag is correlated to the binding affinities of antigens to T cell receptor (TCR). Based on these findings, we hypothesized that the isolated T cells are highly enriched for tumor-reactive T cells and that their reactivity is directed toward TSAs. This hypothesis is strongly supported by our data in mouse B16 melanoma model and E0771 triple negative breast cancer model. The enriched TSA-reactive T cells can be subjected to single-cell TCR sequencing to identify TSA-specific TCRs or directly expanded for patient specific immune cell therapy. This technique has several advantages: (1) avoid expensive and time-consuming approaches to identify TSAs; (2) relies on the interaction-mediated biotinylation rather than the expression of inhibitory receptors (e.g. PD-1)³¹⁻³² or activation markers (e.g. CD134 or CD137) which are also expressed on the cell surface of non-functional bystander CD8+ T cells found in human tumor infiltrates; (3) be capable of detecting TSA-reactive T cell candidates identifies CD8+ and CD4+ T cells with comparable accuracy. In summary, we expect this disclosure will significantly accelerate the pace for the discovery of TSA-reactive TCRs, which in turn will pave the way for lowering the cost and accessibility of personalized cancer treatment.

### EXAMPLE 1

### APPLICATION OF GLYCOSYLTRANSFERASE-MEDIATED CELL-SURFACE ENGINEERING TO CONJUGATE H. PYLORI ALPHA 1,3 FUCOSYLTRANSFERASE TO THE SURFACE OF CHO CELLS.

### Background:

In our previous study (PCT/US2018/016503, published as WO2018/144769), we discovered that H. pylori α1,3fucosyltransferase (FT) possesses remarkable donor substrate tolerance. It enables quantitative transfer of fluorescent probes, short strands of nucleic acids and even full length antibodies (> 100 kDa) conjugated to the GDP-Fucose (GDP-Fuc) donor to LacNAc (Galβ1,4GlcNAc), a common building block of glycocalyx, on the surface of live cells within a few minutes. Our group has developed a single-step method that efficiently modifies native substrates (e.g., LacNAc/sLacNAc-containing glycans) on the surfaces of both cultured cell lines and primary cells to introduce novel functionalities, including enhance T cell migration and guide NK cell to cancer cells *in vitro* and *in vivo.*¹⁵ Compared to sortagging¹⁶, the best known enzymatic covalent ligation reaction without the need for genetic manipulation of the target cell population, this FT-based method is significantly more efficient. Based on these results, we developed various strategies to efficiently introduce enzymes including FT on different cell surfaces to enable new functions to cells.

### General process:

Figure 1A shows an enzymatic method tagging a cell with a probe or label by glycoengineering the cell surface with H. pylori α1,3fucosyltransferase. Briefly, exogenous *H. pylori* α1,3fucosyltransferase enzyme is used to conjugate fucose acceptor glycans (e.g., LacNAc, sLacNAc) present on the surface of a cell to a GDP-fucose derivative that has been bound to a probe or tag (e.g., such as biotin) via a linker. Despite the presence of the probe or tag, the enzyme catalyzes the fucosylation of the cell surface fucose acceptor glycans due to the above-mentioned donor substrate tolerance of the fucosyltransferase. As one skilled in the art will readily appreciate, other fucosyltransferases may use in these reactions (*see, e.g.,* Example 7, which demonstrates similar donor substrate tolerance with human α1,3fucosyltransferase (FUT6) and also *H. pylori* α1,3/4fucosyltransferase).

Figure 1B shows an alternative means for performing similar cell-surface glycoengineering of a "prey cell" that comprises a cell-surface fucose acceptor glycan (e.g., LacNAc, sLacNAc). Briefly, *H. pylori* α1,3fucosyltransferase (FT) is conjugated to the surface of a "bate cell" and mixed with a prey cell in the presence of GDP-fucose derivative that has been bound to a probe or tag (e.g., biotin) via a linker. If the bait cell comes into contact with the prey cell in the presence of the labeled GDP-fucose, the FT on the surface of the bait cell catalyzes the fucosylation of the fucose acceptor glycan on the prey cell resulting in the conjugation of the labeled-fucose on the prey cell.

Due to its high *Kₘ* (1.3 mM) and high *k_{cat}* (442 min⁻¹) (Zheng et al., 2011, Angew. Chem. Int. Ed. 50, 4113-4118), the membrane anchored FT is ideal to enable proximity-dependent labeling of prey cells that interact with bait cells harboring the enzyme (FIG. 1B). In the absence of an interaction, the concentration of LacNAc (sLacNAc) acceptors in the proximity of FT is far below the FT-LacNAc *Kₘ.* Under such circumstances, the bimolecular reaction rate is governed by *k_{cat}*/*Kₘ.* By having a high *Kₘ,* the background labeling is minimized. When two cells interact, the local concentration of LacNAc (sLacNAc) in the vicinity of FT is high such that the pseudo-zero-order reaction rate is determined by *k_{cat}.* By having a high *k_{cat},* the labeling signal in the presence of a *bona fide* cell-cell interaction is maximized.

### Construction of an FT-conjugated cell.

As shown in Figure 2A, we used standard amine-coupling procedures to introduce the bioorthogonal handle trans-cyclooctene (TCO) with a PEG linker to FT (expressed as a His6-tagged recombinant protein). Subsequently, TCO-functionalized FT were reacted with tetrazine functionalized GDP-Fuc (GF-Az-Tz) to generate GDP-Fuc-conjugated FT (GDP-Fuc-FT). The catalytic activities of unmodified FT and GDP-Fuc-FT have no difference (data not shown). GDP-Fuc-FT serves as the self-catalyst to transfer Fuc-FT to LacNAc in the cell-surface glycocalyx. CHO cells were incubated with GDP-Fuc-FT (0.01 mg/mL) for 30 min at room temperature, and the cell-surface conjugated FT was probed with an anti-His-PE antibody (FT has a His-tag).

Figure 2B demonstrates the successful installation of FT on the cell surface of Chinese hamster ovary (CHO) cells, as confirmed by flow cytometry analysis which demonstrated strong hit-tag signal in the CHO + GDP-Fuc-FT treated experiment (FIG. 2B, right peak). Background his-tag signal was seen for untreated CHO cells (FIG. 2B, tallest left peak). Further, in a first control experiment, CHO cells were treated with free FT. No his-tag derived signal could be detected on the cell surface after the treatment (FIG. 2B, left cluster of peaks, lower peak). As a second control, Lec8 cell that do not express membrane LacNAc were treated with GDP-Fuc-FT, only resulting in background signal (FIG. 2B, left cluster of peaks, lower peak - overlays with the first control experiment peak). Right column chart shows the FT conjugation on CHO cell surface at various concentrations of GDP-Fuc-FT. 0.20 mg/mL was determined as the saturated concentration.

Using this method, we further demonstrated that FT can be robustly conjugated within 20 minutes on other cells including, e.g., activated CD8+ T cells (FIG. 2C, top, left panel), mouse bone marrow derived dendritic cells (DCs), (FIG. 2C, top, right panel), human lymphocytes (FIG. 2C, bottom, left panel), and DCs derived from human PBMCs (FIG. 2C, bottom, right panel). Importantly, the installed FT remained on the cell surface for approximately 10 hours (Fig. S3C) and did not affect the cell viability (Fig. S3D) and functions, e.g. DC-mediated CD8+ T cell priming (Fig. S4).

We used Fluorescent SDS-PAGE gel analysis of FT labeled cells to quantify the number of FT molecules that are conjugated to a cell surface via the above methods. FIG. 2D shows Fluorescent SDS-PAGE gel analysis of FT labeled cells. GDP-Fuc-FT molecules modified with Alexa Fluor 647 were used in the experiments for quantifying the number of FT molecules conjugated to one cell surface. At the GDP-Fuc-FT concentration of 0.20 mg/mL, approximately 6.6 x 10⁵ FT molecules were introduced to one CHO cell and approximately 4.0 × 10⁵ FT molecules were introduced to one DC cell. Importantly, FT remained detectable on the surface of CHO and OT-I conjugated cells for around 6 hours and on DC cells for around 10 hours (FIG. 3A). To determine whether the FT conjugation affected cell viability, activated CD8+ T cells and CD8+ T-FT conjugates were cultured separately in T cell medium at 37 °C in an incubator for 6 hours, then cell viabilities were measured by DAPI staining and forward-scattered light (FSC) signals in similar numbers of cells by flow cytometry. No differences in viability of CD8+ T-FT cells (live cell percentage 95.1%) as compared to unmodified CD8+ T cells (live cell percentage 95.6%) were observed (FIG. 3B).

Moreover, we confirmed that FT modification had no effect on DC antigen presentation abilities. FIG. 3C (left panel) shows the various treatment protocols utilized in this experiment. Briefly, iDCs derived from CD45.1+/+ C57BL/6 mice were either labelled with FT then pulsed with OVA257-264 or pulsed with OVA257-264 then labelled with FT. They were then co-cultured with splenocytes from CD45.1- OT-I mice at cell ratio 1:1. Untreated iDCs loaded with OVA257-264 or no antigen were used as a control. After 2 hours of co-culturing, cell mixtures were stained with anti-mCD45.1-FICT, anti-mCD8a-PB and anti-mCD69-PE for flow cytometry analysis. Representative flow cytometry figures from three replicates are shown.FIG. 3C (right panel). These data demonstrate that the FT functionalization of iDCs did not affect the iDC-mediated upregulation of CD69 in CD8+ T cells. Thus, the present invention provides for a versatile method of stably conjugating enzymes to the surface of a cell, without affecting cell viability and functions, e.g., DC-mediated CD8⁺ T cell priming.

### EXAMPLE 2

### FT FUNCTIONALIZED CHO CELLS ARE CAPABLE OF LABELLING THE CONTACT CELLS.

Having demonstrated stable conjugation of enzymes to the surface of cells (e.g., a bait cell as described in FIG. 1B), we sought to determine whether the conjugated enzymes retained functional activity and are able to facilitate contact-mediated labeling of a prey cell.

FIG. 4A show a schematic of the experimental method utilized to that end. Briefly, CHO cells (Cell A) were incubated in a glass chamber for 12 hours. Then FT modified CHO cells (CHO-FT) pre-stained with CellTracker^{™} Green (Cell B) were added to the glass chamber as a cell number ratio of A:B = 5:1. Cells were incubated at 37 °C for 2 hours, then GDP-Fuc-biotin (final concentration 20 µM) was added. After incubating for 20 min, cells were washed twice and stained with DAPI and Alexa Fluor 647-streptavidin for fluorescence microscopic imaging. FIG. 4B shows microscopy imaging of the cells, which clearly demonstrates that (1) all Cell B (green) were robustly labelled on the membrane (red); (2) Cell A not in contact with Cell B were not labelled (example 2), but the Cell A contacting with Cell B were selectively labelled at the cell-cell contacting regions (example 1). In contrast, the treatment of free FT resulted in the unselective labelling of all cells (picture not shown).

Thus, FT conjugated on the surface of a cell is enzymatically active, and FT-conjugated bait cells are capable of labelling prey cells that they interact with.

### EXAMPLE 3

### Ex Vivo USE OF "BAIT" FT-FUNCTIONALIZED DENDRITIC CELLS (DCS) TO DETECT DC INTERACTIONS WITH NAÏVE CD8+ T CELLS

Adaptive responses are initiated when naïve antigen-specific T cells encounter antigen-presenting DCs in lymph nodes and the spleen. This process is called T-cell priming. In this assay, we aim to use FT modified DCs ("bait" cells) to explore the details of DC mediated T-cell priming. FIG. 5A shows a cartoon illustrating one example of how FT modified DCs can be used in this manner to detect contact between antigen-primed DCs and T-cells with TCRs specific for the priming antigen. Briefly, FT modified DCs pulsed with SIINFEKL N4 peptide (OVA₂₅₇₋₂₆₄) (SEQ ID NO: 69) selectively interact with and label naive CD8+ T cells expressing a transgenic T cell receptor (TCR) specific for the SIINFEKL N4 (SEQ ID NO: 69) of ovalbumin presented on MHC I from OT-I transgenic mice. In contrast, FT modified DCs pulsed with GP₃₃₋₄₁ peptide (KAVYNFATM (SEQ ID NO: 75), derived from the lymphocytic choreomeningitis virus (LCMV) glycoprotein) are not recognized by OT-I CD8+ T cells.

FIG. 5B shows the experimental assay procedure used in this study example. Briefly, bone marrow cells from C57BL/6 mice (CD45.2+) were cultured with granulocyte-macrophage colony-stimulating factor (GM-CSF) to be differentiated to iDCs. iDCs were treated with GDP-Fuc-FT (0.20 mg/mL) (produced as described in Example 1) to introduce FT onto the surface as described in Example 1. FT-modified iDCs were pulsed with the cognate SIINFEKL N4 peptide (SEQ ID NO: 69) (10 nM or 100 nM) or with the LCMV peptide GP₃₃₋₄₁ (10 nM or 100 nM) before culturing with splenocytes of naive OT-I mice bearing the congenial CD45.1 marker (CD45.1+/-) as cell number ratio 1:1 for 2 hours. Then GDP-Fuc-biotin (50 µM) was added and incubated for another 30 min. After quenching the reaction, the cell mixture was stained with Alexa Fluor 647-streptavidin and analyzed using flow cytometry.

FIG. 5C shows flow cytometry analysis of this study, which revealed robust labeling on the interacting OT-I CD8+ cells with a signal-to-background ratio of 10:1 and 66:1 respectively (FIG. 5C, top and bottom, far right panels). Here, background is defined as the signal produced on OT-I CD8+ cells by incubating SIINFEKL (SEQ ID NO: 69) -pulsed DCs (without membrane-anchored FT) with naive OT-I splenocytes for the same period of time. In contrast, the iDC-FT loaded with the lymphocytic choriomeningitis virus (LCMV) GP₃₃₋₄₁ peptide only induced the background level labeling of OT-I CD8⁺ T cells, indicating the labeling is highly specific (FIG. 5C, top and bottom, middle panels). As the iDC-FT to T cell ratio increased, the intensity of the FT-mediated intercellular labelling increased accordingly, this was accompanied by the concomitant upregulation of the T-cell early activation marker CD69 (FIG. 5I).

Subsequently, we optimized the labeling condition using the iDC-OT-I splenocyte co-culturing system. To determine optimal GDP-Fuc-FT concentrations for iDC conjugation, iDCs (CD45.1 ^{+/+}) were treated with varying amounts of GDP-Fuc-FT as shown in FIG. 5J, then, iDC-FT loaded with antigen OVA₂₅₇₋₂₆₄ or LCMV GP₃₃₋₄₁ (100 nM) were co-cultured with CD45.1⁻ OT-I splenocytes at iDC/T ratio 1:1 for 2 hours followed the addition of GDP-Fuc-Biotin (50 µM) and another 30 minutes incubation. After quenching with LacNAc (2 mM), cells were washed and stained with anti-mCD45.1-FITC, anti-mCD8a-PB and streptavidin-APC for flow cytometry analysis. As shown in FIG. 5J, optimal labeling was achieved using iDC modified with with 0.2 mg/mL GDP-Fuc-FT.

To determine optimal T cell and iDCs co-culturing time, iDCs (CD45.1+/+) were treated with 0.2 mg/mL GDP-Fuc-FT. Then, iDC-FT loaded with antigen OVA257-264 or LCMV GP33-41 (100 nM) were co-cultured with CD45.1- OT-I splenocytes at iDC/T ratio 1:1 for the times indicated in FIG. 5K followed by the addition of GDP-Fuc-Biotin (50 µM) and another 30 minutes incubation. After quenching with LacNAc (2 mM), cells were washed and stained with anti-mCD45.1-FITC, anti-mCD8a-PB and streptavidin-APC for flow cytometry analysis. iDC/T ratio = 1:1. As shown in FIG. 5K, optimal labeling was achieved within 2 hours using iDC modified with 0.2 mg/mL GDP-Fuc-FT (FIG. 5J and 5K). Under this condition, the antigen specific labeling was positively correlated with the amount of OVA257-264 for iDC priming (FIG. 5L).

To explore if the labeling intensity achieved by FucoID reflects the strength of an interaction,, the above OT-I labeling study was repeated, but another two altered peptide ligands (APLs), SAINFEKL(A2) (SEQ ID NO: 70) and SIITFEKL(T4) (SEQ ID NO: 71) derived from the original OT-I ligand SIINFEKL N4 (SEQ ID NO: 69) were additionally utilized. These APLs bind equally well to MHC I H-2Kb as N4, but differ in their potency for interacting with TCR of OT-I CD8+ cells (binding strength: SIINFEKL(N4) (SEQ ID NO: 69) > SAINFEKL(A2) (SEQ ID NO: 70) > SIITFEKL(T4) (SEQ ID NO: 71)). FT modified iDCs were pulsed by these peptides and LCMV GP₃₃₋₄₁ individually with the final concentration of 100 nM for 30 min and the above pilot study experimental procedure was repeated. As shown in FIG. 5D (flow cytometry results) and FIG. 5E (quantification of the percentage of cells that were biotin + in the various experimental groups), the magnitudes of labeling are SIINFEKL(N4) (SEQ ID NO: 69) > SAINFEKL(A2) (SEQ ID NO: 70) > SIITFEKL(T4) (SEQ ID NO: 71) > LCMV GP₃₃₋₄₁ ≈ unprimed ≈ control (SIINFEKL N4 (SEQ ID NO: 69) pulsed DCs without membrane-anchored FT), which was exactly correlated with the binding strength of APLs. It is worth noting that the magnitudes of OT-I CD8+ cells activation also fitted this trend according to T cell activation marker CD69. Three independent repeats were included in this assay. In summary, the magnitude of biotin tag is correlated to the binding affinities of antigens to T cell receptor (TCR).

We repeated the experiment procedure as shown in FIG. 5B, except we used OT-II mice splenocytes in this case as opposed to the OT-I mice splenocytes that were used in FIG. 5B. FT-modified DCs were primed with 100 nM OVA323-339 peptide or LCMV GP61-80. The results of flow cytometry analysis of this experiment are shown in FIG. 5F, which revealed robust labeling on the interacting OT-II CD4+ cells. iDC-FT pulsed with OVA323-339 specifically biotinylated 11.3% of OT-II CD4+ T cells whose TCR was reactive with OVA323-339 under 2 hours of co-culturing. By contrast, only background labelling (1.68%) was observed in the group using LCMV GP61-80 pulsed DC-FT. Importantly, this demonstrates that FucoID can also be applied to probe iDC-CD4⁺ interactions despite significantly weaker binding between MHC-II bound peptides and CD4.

To further assess the sensitivity and specificity of FucoID in a more challenging situation, we mixed naive OT-I (CD45.1+/-) and P14 CD8+ T cells (Thy1.1+/-) that recognize LCMV GP33-41 presented by MHC-I and co-cultured the mixture with iDC-FT pulsed with OVA257-264 or LCMV GP33-41, respectively at the ratio of 1:1:1 (FIG. 5G, left panel). After co-culturing for 2 hours, GDP-Fuc-Biotin was added. As illustrated in FIG. 5G, right panel and FIG. 5H, OT-I and P14 CD8+ T cells were selectively labeled by OVA257-264 and LCMV GP33-41 primed iDCs-FT, respectively.

It is known that bi-directional transfer of plasma membrane fragments between presenting cells and lymphocytes, called trogocytosis, can occur when these cells are conjugated to one another.^{36, 37} Thus, we designed an experiment to rule out the possibility that the T-cell biotinylation observed in the previous examples was non-specifically generated via trogocytosis, rather than by interaction-dependent labeling. The Experimental design of the experiment is illustrated in FIG. 6A. Briefly, we labeled iDCs with FT (FT was expressed as a His6-tagged recombinant protein) or Fuc-Bio and primed the labeled DCs with OVA₂₅₇₋₂₆₄ or GP₃₃₋₄₁ and tested whether there were detectable differences between these groups and FT-conjugated iDC's treated with GDP-Fuc-Biotin. We then incubated the primed DCs with OT-I splenocytes. To the cell mixture, we either added GDP-Fuc-Bio to initiate the proximity-based labeling or directly stained cells with streptavidin-APC or an anti-His antibody for detecting if any Fuc-Bio or FT, respectively, was transferred via trogocytosis. FIG. 6B shows a histogram of T cells labeled via proximity-based biotinylation (left) or trogocytosis (transfer of Fuc-Bio, mid, or FT, right): OT-I CD8+ T cells were robustly labeled via the proximity-based biotinylation (left panel). By contrast, only background signals were detected for cells that were stained with streptavidin-647 or anti-His antibody directly (middle and right panels), indicating negligible amounts of Fuc-Bio or FT were transferred via trogocytosis.

Thus, taken together, the present example demonstrates that antigen-primed FT-conjugated bait DCs are capable of specifically labeling CD4+ and CD8+ prey T cells that express T cell receptors with binding affinity for the priming antigen.

### EXAMPLE 4

### IN VIVO USE OF "BAIT" FT-FUNCTIONALIZED DENDRITIC CELLS (DCS) TO DETECT DC INTERACTIONS WITH NAÏVE CD8+ T CELLS

With the validation of FucoID as a reliable technique for probing antigen-specific DC-T cell interactions *in vitro* / *ex vivo,* we assessed the feasibility of using this strategy to detect such interactions *in vivo.* Figure 7A shows the protocol of such an *in vivo* experiment p. Briefly, DCs were obtained as described in Example 3 and FT-conjugated DCs were prepared as described in FIG. 5B. Splenocytes from CD45.1+/- OT-I mice (donor mice) were transferred to C57BL/6 mice (CD45.2+) by intravenous (IV) injection (10 × 10⁶ cells per mouse). After 24 hours, unmodified DCs (control DC) or FT modified DC (CD45.2+) cells pulsed with SIITFEKL N4 (SEQ ID NO: 71) or LCMV GP₃₃₋₄₁ were transferred to mice by IV injection (2 × 10⁶ cells per mouse). After 4 hours, GDP-Fuc-Biotin was injected to mice by IV injection (0.4 µmol per mouse). Then inguinal lymph nodes and spleens were isolated, lysed and stained for flow cytometry analysis. FIG. 7B shows quantification of the flow cytometry results. SIITFEKL N4 (SEQ ID NO: 71) pulsed DC-FT could selectively interact with and label naïve CD8+ T cells from donor mice in both lymph nodes and spleens while this was not observed in control DC and LCMV GP₃₃₋₄₁ pulsed DC-FT. Interestingly, we found a small portion of CD8- cells from donor mice were labelled in spleens, which might be result from the interactions of DCs with other type of cells including CD4+ T cells, NK cells and macrophages. In sum, this labelling strategy can effectively monitor the details of cell-cell interaction *in vivo.*

### EXAMPLE 5

### DETECTION OF TUMOR REACTIVE T CELLS FROM INFILTRATING LYMPHOCYTES (TILS) IN B16 MELANOMA USING "BAIT" FT-FUNCTIONALIZED DCs

In tumor tissue, tumor infiltrating lymphocytes (TILs) only contain a minority of T cells bearing neoantigen specific TCRs, leading to the inefficiency of immune response.³³ The first stage of current patient-specific tumor immunotherapies involve complicated procedures to identify these specific T cells or the TCRs they express.³⁴⁻³⁵ An easier approach to identify neoantigen specific T cells in tumor tissue will significantly improve the efficiency of patient specific immunotherapy. FIG. 8A illustrates a method for utilizing FT-functionalized DCs to detect and sort neoantigen specific T cells in tumor tissue. Briefly, DC's are functionalized as in the preceding Examples, and FT-functionalized DCs are pretreated with tumor lysate and then mixed with tumor cell suspension, which includes TILs, in the presence of GDP-Fuc-Biotin. The FT-functionalized DCs present neoantigens from the tumor lysate, and T cells from the TIL, which bear TCRs specific for the presented neoantigens, bind the FT-functionalized DCs and are labeled by FT-mediated interaction-dependent labeling. By this way, the small portion of neoantigen specific T cells may be selected for further analysis.

FIG. 8B shows a more detailed experimental workflow for the direct detection of isolation of tumor-reactive T cells from TILs of B16 melanoma. B16 tumors were inoculated subcutaneously (s.c.) in C57BL/6 mice (0.5 million cells/mice). On day 13-15, tumor was collected and cross-cut into small pieces. Then the tumor pieces were minced and filtered through a cell strainer (100 µM) for the preparation of tumor lysates and single cell suspensions. For tumor lysate preparation, the tumor cells were resuspended in T cell medium (5 million cells/mL), then the cells were lysed by sonication for 1 min and then centrifuged at 2000 × g for 10 min. Then, supernatants were collected and stored at -80 °C for use. For single cell suspensions, the minced tumor cells were washed with PDB and then resuspended in 40% percoll solution (in 1 × DPBS, 25 mL). Then the cell suspension was slowly added on the top of 80% percoll (in 1 × DPBS, 25 mL) without disturbing the interface. After centrifuging at 800 × g for 20 min (acceleration: 5, deceleration: 5), the cells in the middle layer (around the interface of 40% percoll and 80% percoll) were collected. After washing with PBS, the collected cells were resuspended in T cell medium for use. If culture overnight was needed, 100 IU/mL IL2 was supplied in the medium.

iDCs prepared from CD45.1+/+ C57BL/6 mice were divided into four groups: (i) treated with no peptide); (ii) treated with OVA₂₅₇₋₂₆₄ (5 nM); (iii) treated with B16 specific human GP100 (5 nM); and (iv) treated with tumor lysate (prepared via sonication) overnight. Then the four groups of iDCs were functionalized with FT as in FIG. 5B, and tumor cells were incubated with each of the four groups of FT-functionalized DCs for 2 hours (Cell ratio: tumor derived single cells: iDCs = 10:1) at 37 °C. Then GDP-Fuc-biotin (50 µM) was added and incubated for another 30 min. Finally, after quenching the reaction with LacNAc (5 mM), the cell mixture was stained with Alexa Fluor 647-streptavidin and cell identity markers and analyzed using flow cytometry. PD-1+/CD8+/Alexa Fluor 647+ T cells were isolated as prospective TSA-specific T cells.

As shown in FIG. 8C, when tumor cell suspensions were incubated with OVA₂₅₇₋₂₆₄-primed DCs, negligible numbers of CD8+ TILs were biotinylated (FIG. 8C, upper right panel). By contrast, after incubating with DCs primed with the melanoma/melanocyte shared differentiation antigen GP100 or tumor lysates, TILs were robustly labeled (lower left and right panel, respectively). Among all CD8+ TILs in the tumor lysate treated group, ~70% are PD-1+, within which approximately 50% were biotinylated, and almost all biotinylated TILs were PD-1⁺, suggesting that these TILs have encountered their cognate antigens. The other half of PD-1+ TILs were not biotinylated by tumor-lysate primed DCs, suggesting that this fraction may be bystander cells (Fig. 8C, bottom right panel). Significantly, none of PD-1- T cells were biotinylated. Next, PD-1- (green, lower left quarter of lower right panel), biotin+ (red, upper right quarter of lower right panel) and biotin- (blue, upper left quarter of lower right panel) TILs as shown were sorted and rest in T cell medium (IL2: 100 IU/mL) for 72 hours, then the sorted cells were evaluated in an *ex vitro* tumor cell killing assay.

B16-OVA melanoma cells (stably transduced with firefly luciferase) were co-cultured with sorted CD8+ T cells for 20 hours as a ratio of 1:4. Then B16-OVA cell numbers were quantified through the luciferase activity (Bright-Glo, Promega). PD-1+/biotin+ T cells (right column) exhibited significant higher killing activities than PD-1+/biotin- (middle column) and PD-1- (left column) T cells, strongly suggesting that TSA-reactive T cells are enriched in the biotin+ population (FIG. 8D). Data was obtained from three independent experiments.

In a follow-up experiment, we assessed the tumor-reactivity of the expanded cells based on IFNγ secretion following co-culturing with DCs pulsed with tumor lysates or GP100₂₅₋₃₃ using an ELISpot assay. Data from this experiment is shown in FIG. 8E. The PD-1- (FIG. 8E, left column in each group) and PD-1+/biotin - (FIG. 8E, middle column in each group) cells only exhibited reactivity near the background level (background reactivity is defined as the spot number detected by incubating expanded cells with unprimed DCs or DCs primed with the OVA₂₅₇₋₂₆₄ peptide). By contrast, substantial tumor-reactivity was observed for the biotin+ cells (FIG. 8E, right column in each group). Data was obtained from three independent experiments. Unprimed DCs were used as a negative control and PMA Ionomycin was used as a positive control. In all figures, ns P > 0.05; ** P < 0.01; *** P < 0.001; **** P < 0.0001; one-way ANOVA followed by Tukey's multiple comparisons test.

### EXAMPLE 6

### DETECTION OF TUMOR REACTIVE T CELLS FROM INFILTRATING LYMPHOCYTES (TILS) IN TRIPLE NEGATIVE BREAST TUMOR E0771 USING "BAIT" FT-FUNCTIONALIZED DCs

After confirming FucoID as a highly effective approach to detect and enrich for TSA-reactive TILs from the B 16-OVA melanoma model and from B16 melanoma tumor, we sought to explore whether this method also worked with E0771 Triple Negative Breast Cancer (TNBC) tumors and MC38 colon cancer tumors. TNBC. is a complex and aggressive breast cancer lacking estrogen receptor, progesterone receptor, and HER2 amplifications, making its therapeutic strategies very limited. This example shows the potential of the present invention for enriching tumor reactive TILs from TNBC of E0771 breast tumor and MC38 colon cancer tumors. The E0771 cell line is a spontaneously developing medullary breast adenocarcinoma from C57BL/6 mice (Yang, Y.; Yang, H. H.; Hu, Y.; Watson, P. H.; Liu, H.; Geiger, T. R.; Anver, M. R.; Haines, D. C.; Martin, P.; Green, J. E.; Lee, M. P.; Hunter, K. W.; Wakefield, L. M. Oncotarget 2017, 8, 30621-30643.) The MC38 cell line is a validated model for hypermutated colorectal cancer (Efremova et al., Nat. Commun. 9, 32, 2018).

FIG. 9A shows the experimental workflow for the direct detection of isolation of tumor-reactive T cells from TILs of E0771 tumor, and the MC38 experimemts followed a similar protocol. Briefly, for E0771, 1 × 10⁶ E0771 tumor cells were inoculated (s.c.) in the mammary gland of C57BL/6 mice and for MC38, tumor cells were implanted subcutaneously into the flanks of male C57BL/6 mice (CD45.1- if no specific indication), in each case using the same method that is described in Example 5 above with respect to the B16 tumor cells for approximately 14 days before the isolation of tumor tissue. Tumor lysate and single cell suspensions were prepared using the same method that is described in Example 5 above. DCs prepared from CD45.1+/+ C57BL/6 mice were divided into four groups: (i) treated with no peptide; (ii) treated with OVA₂₅₇₋₂₆₄ (100 nM); (iii) treated with mammary gland lysate from healthy C57BL/6 mice; and (iv) treated with E0771 tumor lysate overnight. Then the four groups of DCs were functionalized with FT as in FIG. 5B, and tumor cells were incubated with each group of FT-functionalized DCs for 2 hours (Cell ratio: tumor derived single cells: iDCs = 10:1). Then GDP-Fuc-biotin (50 µM) was added and incubated for another 30 min. After quenching the reaction with LacNAc (5 mM), the cell mixture was stained with Alexa Fluor 647-streptavidin and cell identity markers and analysed using FACS. FIG. 9B and 9E show FACS results demonstrating that FT-functionalized DCs primed with E0771 tumor lysate or MC38 cell line lysate specifically labelled 17.3% and 24.9% of CD8+ TILs, respectively, and all of these cells are PD-1+ (far right panel, upper right quarter in FIGS. 9B and 9E). In contrast, FT-functionalized DCs treated with no antigen (second panel from the left in FIGS. 9B and 9E), OVA₂₅₇₋₂₆₄ (middle panel for E0771 in FIG. 9B) and healthy mammary gland lysate (second panel from the right in FIG. 9B) or healthy colon lysate (second panel from the right in FIG. 9E) showed minimal biotinylation to CD8+ TILs.

Sorted CD8+ T cells were rest in T cell medium (IL2: 100 IU/mL) for 72 hours. Then the sorted cells were evaluated in an *ex vitro* tumor cell killing assay. E0771 or MC38 cells (stably transduced with firefly luciferase) were co-cultured with sorted CD8+ T cells for 20 hours as a ratio of 1:4. Then E0771 or MC38 tumor cell numbers were quantified through the luciferase activity (Bright-Glo, Promega). For E0771, PD-1+/biotin+ T cells (FIG. 9C, right column) exhibited significant higher killing activities (>90%) than those of PD-1+/biotin-(~20%) (FIG. 9C, and PD-1- T cells (~5%), strongly suggesting that TSA-reactive T cells are enriched in the biotin+ population. Data was obtained from three independent experiments. Similar results were observed for MC38 tumor cells (FIG. 9F).

We then assessed the tumor-reactivity of the expanded cells based on IFNγ secretion in an ELISpot assay following co-culturing with DCs pulsed with (i) no antigens; (ii) OVA₂₅₇₋₂₆₄, (iii) tumor lysates; or (iv) tumor lysate + anti-mouse MHCI in the case of E0771 and pulsed with tumor lysate or tumor lysate + anti-mouse MHCL in the case of MC38. As shown in FIG. 9D with respect to E0771, the PD-1- and PD-1+/biotin- cells (left and middle columns, respectively, in each group) only exhibited reactivity near the background level. By contrast, substantial tumor-reactivity was observed for the Biotin+ cells (right column in each group). As shown in FIG. 9G, similar results were observed for MC38, with the PD-1- and PD-1+/biotin- cells (left two columns in each group) exhibiting near background levels, whereas substantial reactivity was seen in the PD-1+Bio+ cells exposed to tumor lysate exhibited substantial tumor-reactivity. It is worth noting that T cells treated with PMA/Ionomycin showed significantly stronger INFγ releasing in the Biotin+ population, and this of IFNγ release was completely blocked by the addition of MHCI antibody. Data was obtained from three independent experiments. In all figures, ns P > 0.05; ** P < 0.01; *** P < 0.001; **** P < 0.0001; one-way ANOVA followed by Tukey's multiple comparisons test.

### EXAMPLE 7

### CHARACTERIZATION OF TCR CLONOTYPIC REPERTOIRES OF TILs ISOLATED FROM E0771 AND MC38 TUMOR MODELS

The differences in TSA-reactivity of PD-1+Bio+ and PD-1+Bio- CD8+ TILs results from the differences of their TCR clonotypic repertories. We characterized TCR clonotypic repertoires of these two TIL subsets isolated from E0771 and MC38 tumor models directly after their FACS isolation without further in vitro expansion. TCRβ deep sequencing was employed for quantifying the frequency of individual T-cell clonotype in each subset. A productive CDR3 sequence that does not contain stop codons or frame shifts represents a unique TCR clonotype, and the total number of unique sequences determines the clonal diversity in each subset, providing each subset has comparable total CDR3 reads. We found that TCRβs in the PD-1+Bio+ population were significantly more oligoclonal than their counterparts in the PD-1+Bio- subset, suggesting the cells in the PD-1+Bio+ subset have undergone substantial TSA-driven clonal expansion (FIG. 12). Furthermore, there was no overlap of the 10 most abundant TCRβ CDR3s found between these two subsets in either tumor models (FIG. 12). The results from IFNγ ELISpot assay and TCRβ deep sequencing indicated that PD-1+Bio+ and PD-1+Bio- TILs represent two functionally and clonotypically distinct T cell subsets that co-exist in the same tumors and share a certain degree of phenotypical similarities (e.g. PD-1+).

Because PD-1+ TILs consisted of not only TSA-reactive T cells but also bystander T cells, upon in vitro expansion, anti-tumor cytotoxicity of the entire PD-1+ TIL population should be considerably weaker than that of the PD-1+Bio+ TIL subset providing that TSA-reactive and bystander T cells share similar expanding rates. To assess this hypothesis, PD-1+Bio+, PD-1+Bio- and total PD-1+ TILs isolated from the same tumors were subjected to the rapid expansion protocol. According to the recorded growth curve, PD-1+Bio- and total PD-1+ TILs exhibited very similar expansion rate, which was significantly faster than that of PD-1+Bio+ TILs during the entire course of expansion (FIG. 13). These observations suggest that at the end of expansion bystander PD-1+Bio- TILs become the dominant cell population within the expanded total PD-1+ TILs. On the expansion day 10, tumor killing capabilities of each subset were assessed. At different effector/target ratios PD-1+Bio+ TILs exhibited remarkably stronger tumor cell killing than the corresponding PD-1+Bio- and total PD-1+ TILs (FIGS. 8D, 9C, 9F, and FIG. 14). These results suggest that due to the faster proliferation of bystander T cells within the entire PD-1+ TIL population, anti-tumor activities of total PD-1+ TILs become significantly weaker than those of the expanded TSA-reactive PD-1+Bio+ TILs at the end point of rapid expansion.

### EXAMPLE 8

### THE EXPANDED PD-1+BIO+ TILs EXHIBIT SIGNIFICANTLY HIGHER ANTI-TUMOR ACTIVITIES THAN TOTAL PD-1+ TILs, IN VIVO

To compare anti-tumor activities of the expanded PD-1+Bio+ TILs and the total PD-1+ subset in vivo, we first explored the use of the TILs isolated from the B 16 melanoma model to control tumor growth in mice with established pulmonary micrometastases. Three days after the intravenous inoculation of B16 tumor cells stably transduced with firefly luciferase (B16-luc) to induce pulmonary metastasis, tumor-imbedded mice were treated with the expanded total PD-1+ TILs and PD-1+Bio+ TILs (3 × 10⁶ per mice), respectively, while the control group was injected with buffer only. Tumor proliferation was monitored by longitudinal, noninvasive bioluminescence imaging. As shown in FIG. 15A, the total PD-1+ TILs showed moderate therapeutic potency for preventing tumor proliferation with 60% lower bioluminescence than the HBSS control group on treatment day 8. In comparison, PD-1+Bio+ TILs significantly inhibited tumor growth, showing 98% lower bioluminescence signal than the HBSS control group. Remarkably, the survival of the tumor bearing mice was significantly elongated upon treatment with the PD-1+Bio+ TILs. All mice received buffer only and the total PD-1+ TILs died on treatment day 22 and 26, respectively. By contrast, all PD-1+Bio+ TIL recipient mice were alive until treatment day 27 and by the end of the experiment, still 20% mice remained alive (treatment day 40).

To assess capabilities of the expanded PD-1+Bio+ TILs of suppressing solid tumor growth we sought to use the TILs isolated from the subcutaneous MC38 tumors. Expanded total PD-1+ TILs and PD-1+Bio+ TILs (5 × 10⁶ per mice), respectively, were intravenously injected into mice with established subcutaneous MC38 tumors, followed by anti-PD-1 administration. In the control groups, mice were treated with anti-PD-1 and HBSS, respectively. Although anti-PD-1 and anti-PD-1 + total PD-1+ TIL treatments only showed modest tumor control, PD-1+Bio+ TILs combined with anti-PD-1 significantly slowed down tumor growth with median tumor size being only ¼ of that treated with anti-PD-1+ total PD-1+ TILs (treatment day 22, FIG. 15B). Moreover, whereas no mice in any control group survived up to day 28, 50% mice treated with PD-1+Bio+ TILs + anti-PD-1 were still alive by day 34. And one mouse was found to be tumor free by day 40 (FIG. 15B). These results indicated that the expanded PD-1+Bio+ TILs possess markedly higher activities to control tumor growth in vivo than the expanded total PD-1+ TILs that contain a large fraction of bystander T cells.

### EXAMPLE 9

### CONJUGATION OF OTHER ENZYMES TO THE SURFACE OF CELLS VIA FUCOSYLATION AND COMPARISON OF THE SAME TO CELLS CHEMICALLY CONJUGATED TO ENZYMES

In order to extend the versatility of the above-examples, we demonstrate in this example that various enzymes may be conjugated to the surface of a cell using our glycoconjugation method (described in the above Examples) or via a chemical conjugation method, and cells conjugated in either way are suitable for use in cell to cell proximity experiments. FIG. 10A illustrates these two schemes for the conjugation of any enzyme on cell surface of a cell via fucosylation (Method 1, left scheme) or via chemical conjugation (Method 2, right scheme).

Method 1 is described in the above Examples: first, enzymes were linked with a "clickable" group such as tetrazine, azide or alkyne by amine-coupling or site-specific modification (such as aldehyde tag or unnatural amino acid modification). The linked enzymes were then conjugated to GDP-Fucose derivatives bearing complementary "clickable" groups to form GDP-Fuc-Enzyme conjugates via click chemistry. The enzyme-functionalized GDP-fucose (GDP-Fuc-Enzyme) was then transferred onto the cell surface catalyzed by fucosyltransferase, (in this Example, *H. pylori* α1,3fucosyltransferase was used to transfer the enzymes to the cell surface, but other fucosyltransferases may be used to facilitate this step as can a sialyltransferase if enzyme-functionalized CMP-Neu5Ac analogue is used instead of the functionalized GDP-fucose), thereby linking the enzyme to the cell surface. In this experiment, we conjugated biotinylated human α2,6αsialyltransferase (ST6Gal1), His-tagged *H. pylori* α1,3fucosyltransferase (FT), His-tagged human α1,3fucosyltransferase (FUT6) and His-tagged Sortase A (5M) onto immature DC surfaces to form DC-enzyme conjugates *via* the Method 1 glycoconjugation technique.

In Method 2 (FIG. 10A, right scheme), the enzymes were linked with tetrazine by amine-coupling to form enzyme-tetrazine conjugates (Enzyme-Tz). Next, cells were treated with TCO-NHS Ester to introduce TCO moieties on the cell surfaces. Finally, the enzyme-Tz conjugates were reacted with the TCO-NHS moieties on the cell surfaces by biorthogonal reaction to form cell-enzyme surface conjugates. In this experiment, we conjugated His-tagged *H. pylori* α1,3fucosyltransferase (FT) and His-tagged *H. pylori* α1,3/4fucosyltransferase ((1,3/4)FT) on immature DC surfaces to form DC-enzyme conjugates *via* the Method 2 chemical conjugation technique.

Flow cytometry results using streptavidin-APC are presented in FIG. 10B confirming that human α2,6αsialyltransferase (ST6Gal1) was efficiently linked to the surface of immature Dendritic Cells using Method 1. The ST6Gal1 was pre-modified with biotin as a tag for detection purposes. The left peak shows the background streptavidin-APC signal in unconjugated DCs, and the right peak shows a marked increase in streptavidin-APC signal in cells that have been conjugated to the ST6Gal1 via the fucosylation method. Similarly, FIG. 10C shows flow cytometry results using examples of introducing His-tagged *H. pylori* α1,3fucosyltransferase (FT), His-tagged human α1,3fucosyltransferase (FUT6) and His-tagged Sortase A (5M) on immature DC surface to form DC-FT (as described in above examples) and DC-Sortase conjugates, respectively, using Method 1. The left peak shows the background anti-his tag-phycoerythrin (PE) signal in unconjugated DCs, and the other three peaks show, from left to right, increases in anti-his tag-phycoerythrin (PE) signals in cells that were conjugated to His-tagged Sortase A (5M), His-tagged human α1,3fucosyltransferase (FUT6), and His-tagged *H. pylori* α1,3fucosyltransferase (FT) via the fucosylation Method 1.

In FIG. 10D, results are shown from flow cytometry experiments demonstrating the successful formation of DC-NHS-FT and DC-NHS-(1,3/4)FT conjugates using the Method 2 chemical conjugation protocol. Specifically, FIG. 10D shows flow cytometry analysis with anti-his tag-phycoerythrin (PE) of immature DCs conjugated on their surface to His-tagged *H. pylori* α1,3fucosyltransferase (FT) and His-tagged *H. pylori* α1,3/4fucosyltransferase ((1,3/4)FT) by Method 2. The left peak shows the background anti-his tag-phycoerythrin (PE) signal in unconjugated DCs, and the other two peaks show, from left to right, increases in anti-his tag-phycoerythrin (PE) signals in cells that were conjugated to *H. pylori* α1,3fucosyltransferase (FT) and His-tagged *H. pylori* α1,3/4fucosyltransferase ((1,3/4)FT).

Having demonstrated that each of Methods 1 and 2 are capable of conjugating a wide variety of enzymes to the surface of cells, we sought to determine whether each of these conjugated cells were suitable for use in cell-to-cell interaction-dependent labeling reactions. FIG. 10E shows the experimental workflow of these experiments (top panel). Briefly, bone marrow cells from C57BL/6 mice bearing the congenial CD45.1 marker (CD45.1+/+) were cultured with granulocyte-macrophage colony-stimulating factor (GM-CSF) to be differentiated to immature DCs. Immature DCs were conjugated with enzymes using either Method 1 or Method 2 as described above. Then DC-Enzyme conjugates were primed with the cognate SIINFEKL (OVA) peptide (OVA₂₅₇₋₂₆₄)) (SEQ ID NO: 69) or LCMV GP₃₃₋₄₁ before culturing with naive CD8+ T cells from OT-I mice (CD45.1-/-) as cell number ratio 1:1 for 2 hours. The SIINFEKL (OVA) peptide (OVA₂₅₇₋₂₆₄) (SEQ ID NO: 69) selectively interacts with naive CD8+ T cells expressing a transgenic T cell receptor (TCR) specific for the SIINFEKL N4 (SEQ ID NO: 69) of ovalbumin presented on MHC I from OT-I transgenic mice. Next, corresponding substrate-biotin derivatives (100 µM CMP-Sialic acid-biotin for ST6Gal1; 50 µM GDP-Fuc-biotin for FT, FUT6 and 1,3/4FT; 500 µM biotin-LPETG (SEQ ID NO: 5) for DC-sortase) were added and incubated for another 30 min (2 hours for DC-Sortase). After quenching the reactions, the cell mixtures were stained with anti-CD8-Pacific Blue, anti-CD45.1-FITC and streptavidin-APC and analyzed using flow cytometry.

The results of these flow cytometry experiments are shown in FIG. 10E, lower panel. In all conditions (including DC-enzymes produced by either of Methods 1 and 2), OT-I CD8+ cells that were incubated with OVA peptide-primed DC-enzyme conjugates exhibited increased streptavidin-APC signals in comparison to similar OT-I CD8+ cells that were incubated with LCMV GP₃₃₋₄₁ peptide-primed DC-enzyme conjugates. Moreover, interestingly, FT-conjugated DCs produced via enzymatic coupling by Method 1 (DC-FT) exhibited vastly superior proximity transfer (77% streptavidin-APC + cells; signal/background ratio of 592) than the FT-conjugated DCs produced by Method 2 (DC-NHS-FT)(16% streptavidin-APC + cells; signal/background ratio of 9.2). Here background is defined as the ratio of biotinylated OT-I CD8+ T cells by DC-enzyme primed with LCMV GP₃₃₋₄₁.

Thus, these results demonstrate (i) that widely different enzymes may be used in the present method to catalyze interaction-dependent labeling of contacted cells; (ii) the interaction-dependent labeling catalyzed by the DC-enzyme conjugates is specific and occurs only upon binding to T cells expressing TCRs specific for their priming antigens; (iii) that chemically coupled DC-enzyme-conjugates are active; thus, chemical conjugation is another suitable means for attaching functional enzymes to the surface of a cell; and (iv) that, at least for FT, glycoconjugating the enzyme to the surface of DCs using Method 1 results in a superior DC-FT conjugates than chemical conjugating via Method 2.

Thus, in summary, the present invention provides a robust and versatile method for conjugating enzymes to the surface of cells to impart upon them new enzymatic functions, and cells engineered in this manner are suitable for monitoring cell to cell interactions *in vitro, ex vivo,* and *in vivo.*

### EXAMPLE 10

### INTERACTION-DEPENDENT LABELING OF T CELLS WITH DENDRITIC CELLS GENETIC MODIFIED TO EXPRESS ENZYMES ON THEIR CELL SURFACE

Having shown that enzymes chemically-conjugated or glyco-conjugated to the cell surface of a DC may be used to facilitate proximity-based labeling of T cells interacting with the DCs, we sought to determine whether enzymes, (e.g., FT) recombinantly expressed in DCs might also be suitable for this purpose.

Toward this end, iDCs are differentiated from PBMC of a patient with hematologic malignancies, such as AML; ALL; CLL, and subjected to lentiviral-transfection to express FUT6 on the cell-surface using the lentiviral expression vector map shown in Figure 11. The DNA sequence or this vector with the FUT6 insert is provided as SEQ ID NO: 3. This vector contains a FUT6 insert DNA sequence of SEQ ID NO: 2, which encodes for a FUT6 polypeptide having the amino acid sequence of SEQ ID NO: 1. In parallel, cancer cells are isolated from the same patient (bone marrow or blood) and lysed for priming iDCs. The primed iDCs or un-primed (control) iDCs are stained with CellTracker^{™} Green CMFDA, and and cultured with autologous PBMC of the same patient at different ratios for 1-2 hours. Then GDP-Fuc-biotin (50 µM) is added and incubated for another 30 min. After quenching the reaction with LacNAc, the cell mixture is stained with Alexa Fluor 647-streptavidin and cell identity markers, and subjected to flow cytometry analysis. CD4+ (FOXP3-) and or CD8+ T cells that are also Alexa Fluor 647+ will be isolated as prospective TSA-specific T cells.

### EXAMPLE 11

### DETECTION OF AUTO-REACTIVE CD4+ AND CD8+ T CELLS AND ANTIGEN SPECIFIC REGULATORY T CELLS IN AUTOIMMUNE DISEASE

Polymyositis is an inflammatory muscle disease that causes muscle tenderness, muscle weakness, and ultimately muscle atrophy and fibrosis. Although its cause is unknown, the presence of T cells, macrophages and dendritic cells in muscle tissue as well as the presence of disease specific autoantibodies suggest that autoimmune reactions likely play a role in the etiology and/or progression of this disease. Venalis P, Rheumatology (Oxford). 2014 Mar;53(3):397-405. However, known autoantibodies are all directed against ubiquitously expressed autoantigens and the specificity of the T cell reactivity is not known. *Id.*

Thus, in this example, we seek to identify auto-reactive CD4+ and CD8+ T Cells and antigen specific regulatory T cells in polymyositis using our interaction-dependent labeling method. In certain examples, this method is suitable for use in any autoimmune disease.

Tissue biopsy samples (one from the diseased tissue and one from neighboring normal tissue) are obtained from a polymyositis patient and are divided into two portions: one is used to prepare tissue lysates; one is used to prepare single cell suspension. Immature DCs are differentiated from PBMCs from the same patient and then the iDCs are primed with the lysates from the diseased tissue or control, normal tissue.

Subsequently, *H. pylori* α1,3fucosyltransferase (FT) is conjugated onto the cell surface of all groups of DCs using the Method 1 protocol described in Example 7, herein, and the modified DCs are incubated with single cell suspensions containing muscle cells, tissue infiltrating lymphocytes and other cell types before adding GDP-Fuc-Bio to initiate proximity-dependent labeling. After quenching the reaction with free LacNAc, cell mixtures are stained with antibodies for cell identity and activation markers, and analyzed and sorted by FACS. From this assay, biotin+, CD8+ T cells (prospective auto-reactive T cells) are isolated. Additionally, biotin+, CD4+, CD25+, FOXP3+ T cells (prospective antigen specific regulatory T cells) are isolated.

The enriched TSA-reactive T cells are subjected to signal single cell TCR sequencing to identify antigen-specific TCRs. Additionally, RNA-seq is performed to compare the biotin+ and biotin- populations.

### EXAMPLE 12

### PD-1+BIO+ TILs ARE DISTINCT TO PD-1+BIO- TILs AND DISPLAYS ACTIVATION/DYSFUNCTION GENE SIGNATURE

To gain an understanding of the genetic programs that underlie the phenotypical and functional features of the TSA-reactive and two different groups of bystander TILs, we characterized transcriptional profiles of PD-1+Bio+, PD-1+Bio- and PD-1- CD8+ T cells isolated from MC38 subcutaneous tumors. Principle component analysis (PCA) revealed that the transcript profiles of these three subsets of TILs shared substantial divergence (FIG. 16A). As identified by volcano plot messenger RNA (mRNA) comparisons between PD-1+Bio+ and PD-1+Bio- CD8+ TILs, 290 transcripts were significantly upregulated or downregulated (FIG. 16B). By contrast, a total of 3704 genes were differentially expressed between PD-1- and PD-1+Bio- TILs (FIG. 17).

We then focused on analyzing the less pronounced transcriptional difference of PD-1+Bio+ and PD-1+Bio- CD8+ TILs. An over-representation analysis was conducted to explore the enrichment of the 290 genes in biological processes annotated by the gene ontology database. Compared to PD-1+Bio- TILs, several up-regulated genes of PD-1+Bio+ TILs were significantly enriched in steroid biosynthesis and related metabolic pathways (FIG. 16C and FIG. 18), such as MSMO1 and DHCR7. This is consistent with the previously reported discovery that the cholesterol metabolism of T cells is fully reprogrammed upon cell activation to support cell proliferation(Bensinger et al., 2008; Tuosto and Xu, 2018). An increase in the plasma membrane cholesterol level of CD8+ T cells augments T-cell receptor clustering, signaling and the more efficient formation of the immunological synapse, which are essential for the effector function of CD8+ T cells (Yang et al., 2016). By contrast, downregulated genes are enriched in more diverse biological process networks, including those of lymphocyte differentiation, T cell migration and activation, viral response and calcium homeostasis (FIG. 16C and FIG. 18). These findings strongly suggest that PD-1+Bio- CD8+ TILs are bystander T cells with virus reactivity and have an altered spectrum of core cellular processes compared to PD-1+Bio+ TILs (Scheper et al., 2019; Simoni et al., 2018).

To further characterize genetic differences of these three subsets of TILs, we performed gene set enrichment analysis (GSEA) using the gene signatures and gene modules established in chronic virus infection induced T cell exhaustion and tumor associated T cell activation/dysfunction models. We initially compared these three subsets TILs for the enrichment of a previously reported naïve/memory-like T cell gene module and found this module is enriched in PD-1- T (FIG. 19A and Table 1) (Singer et al., 2016). Next, we assessed the three subsets TILs for the enrichment of the exhaustion signature derived from exhausted T cells isolated from chronic LCMV infection TILs (Wherry et al., 2007), finding a similar enrichment of this signature in both PD-1+Bio+ and PD-1+Bio- CD8+ subsets compared to PD-1- TILs (FIG. 18B and Table 2). We then compared the TSA-reactive PD-1+Bio+ TILs with the bystander PD-1+Bio- and PD-1- TILs for the enrichment of the gene modules shared by T cells infiltrating human or murine tumors. The T cell activation/dysfunction gene module established for B16F10 melanoma(Singer et al., 2016) was significantly enriched in PD-1+Bio+ vs. PD-1+Bio- TILs; notable genes in this module include genes encoding the cytokine IL2 receptor (IL2RA), the T cell activation related glycolysis enzyme GAPDH (GAPDH) and the plasma membrane transporter for monocarboxylates such as lactate and pyruvate (SLC16A3) (FIG. 16D, FIG. 19C and Table 3). Consistent with this finding, an enrichment of the upregulated cell cycle gene signature that was validated for human melanoma TILs was found in PD-1+Bio+ TILs in comparison to both PD-1+Bio- and PD-1- bystander TILs (FIG. 19D and Table 4). This finding, combined with the observed clonal expansion of PD-1+Bio+ TILs, provided strong evidence for ongoing proliferation within this dysfunctional but TSA-reactive T cell subset. This feature has also been observed previously for a subpopulation of CD8+ T cells infiltrating human and murine tumors that are believed to possess tumor reactivity. By comparing the transcript profiles of monoclonal CD8+ T cells specific for Tag epitope I (Tag-I; SAINNYAQKL (SEQ ID NO: 74)) infiltrating early and late stage murine tumors to that of D30 exhausted T cells isolated from chronic LCMV infection, Greenberg and coworkers discovered the unique gene signatures of dysfunctional, tumor-specific T cells that are not shared by dysfunctional T cells triggered by chronic viral infection(Schietinger et al., 2016). We compared our polyclonal TSA-reactive PD-1+Bio+ TILs with the bystander PD-1+Bio- and PD-1- TILs for the enrichment of this tumor-specific T cell gene set and found that it was significantly enriched in the PD-1+Bio+ subset (FIG. 16E, FIG. 19E and Table 5A and 5B).

Finally, we analyzed the transcript levels of the genes that were previously reported as tumor reactive TILs selection markers in PD-1+Bio+ and PD-1+Bio- TIL subsets isolated from the MC38 colon cancer model. To our surprise, in both TIL subsets similar transcript expression levels were detected for all of these selection markers, including PDCD1 (PD-1), HAVCR2 (TIM-3), LAG3 (LAG-3), ENTPD-1 (CD39), ITAGE (CD103), TNFRSF4 (OX-40) and TNFRSF9 (CD137) (FIG. 16F). We further analyzed the expression of several of these selection markers on the cell surface by flow cytometry (FIG. 16G and FIG. 20). Although the PD-1+Bio+ TIL subset was found to express higher levels of TIM-3 and CD137 than the PD-1+Bio- TILs, varying levels of LAG-3, CD39, and CD103 expressions were found in both subsets(Duhen et al., 2018; Gros et al., 2014a; Yossef et al., 2018). Taken together, we conclude that FucoID may be more generally applicable than these previously reported functional markers-based selection approaches to identify TSA-reactive TILs.

TILs within individual tumors consist of heterogeneous populations including not only the T cells specific for tumor antigens, but also those recognizing a wide range of epitopes unrelated to cancer (e.g. antigens from Epstein-Barr virus, human cytomegalovirus or influenza virus) (Scheper et al., 2019; Simoni et al., 2018). These bystander CD8⁺ TILs have diverse phenotypes that overlap with those of the tumor-specific T cells, but are not tumor reactive (Duhen et al., 2018; Yossef et al., 2018). Although several selection markers (e.g. PD-1, CD39, CD103) have been utilized to exclude bystander CD8⁺ TILs, they are empirical and may generate false positive selection. Moreover, TSA reactive TILs in less abundant or rare populations could be missed using such indirect selection methods because these TILs may not share the same exhaustion status or phenotypes with the most abundant TSA-reactive TILs. By contrast, the FucoID strategy developed here generates a selection maker, e.g., biotin, based on the direct TCR-pMHC interaction, thus providing an unbiased approach for TSA-reactive TIL identification. Through FucoID, T cell candidates that are TSA-reactive can be enriched directly for expansion and for rapid isolation of the corresponding TCRs to construct TCR engineered T cells for functional evaluation. As a consequence, research time and cost are dramatically reduced compared to the aforementioned reverse immunology based pMHC tetramer approach(Arnaud et al., 2020). Significantly, once a TSA-reactive TCR is confirmed, it is possible to use other recently developed methods to identify the corresponding antigen (Gee et al., 2018; Kula et al., 2019; Li et al., 2019).

TSA-reactive CD8⁺ T cells (i.e. PD-1⁺Bio⁺ T cells) isolated by FucoID from murine tumor models in this study exhibited a dysfunctional phenotype, but still possessed significant proliferative and tumor killing capacities. A subpopulation of these TSA-reactive T cells (~5%) harbored progenitor exhausted T cell characteristics (TCF1⁺TIM-3⁻) (FIG. 20B), which is in line with tetramer-sorted tumor-specific T cells from previous studies (Miller et al., 2019). It has been demonstrated that it is this subset of CD8⁺ T cells that provides the proliferative burst and effector function following anti-PD-1/PD-L1 therapy (Held et al., 2019; Im et al., 2016; Miller et al., 2019; Siddiqui et al., 2019). Therefore, future efforts should be devoted to approaches for enlarging this subset during rapid expansion to boost the therapeutic potential of TIL-based adoptive cell transfer.

We demonstrated here that FT modified mouse DCs could induce antigen specific fucosyl-biotinylation of not only CD8⁺ but also CD4⁺ T cells, and the labeling strength was correlated to the binding affinities of pMHC to TCR. Thus, FucoID opens a new door to study primary antigen specific CD4⁺ T cells not only in tumors but also in autoimmune diseases. CD4⁺ T cells are challenging targets to study using conventional approaches partially due to the diversity and length variation (11 to 30 amino acids) of MHC-II binding epitopes and their weak interactions with MHC-II (Editorial, 2017; Racle et al., 2019). Likewise, FucoID may be appliced to separate T cells possessing high affinity TCRs from those with weaker ones for studying their functions in tumor and related infection models.

As the first glycosyltransferase-mediated tagging approach for probing cell-cell interactions, FucoID does not rely on genetic manipulations such that it is readily applicable to probe primary cell interactions. Importantly, installing FT onto human DCs is as easy and straightforward as what has been shown here for human DC functionalization. Therefore, FucoID has a high potential to be translated to a clinical setting for the detection and isolation of TSA-reactive TILs from human patients. Through popularizing FucoID, we expect the pace for the discovery of TSA-reactive TILs and their TCRs would be significantly accelerated, which in turn would pave the way for lowering the cost and accessibility of personalized cancer treatment (Arnaud et al., 2020; Yamamoto et al., 2019).

### EXAMPLE 13

### DETECTION OF CD8+ AND CD4+ TUMOR REACTIVE T CELLS FROM INFILTRATING LYMPHOCYTES (TILS) IN PANCREATIC TUMOR CELLS USING "BAIT" FT-FUNCTIONALIZED DCs

To further demonstrate the versatility of the Fuco-ID proximity labeling method described herein we explored whether we could label pancreatic tumor specific CD8+ and CD4+ T cells isolated from a mouse model of pancreatic cancer and whether such TILs could effect specific killing of tumor cells. Pancreatic cancer is one of the deadliest cancers, with the a 5-year survival rate of just 10% (NCI statistics, available at the world wide web address cancer.gov/types/pancreatic). And, pancreatic ductal adenocarcinoma (PDAC) is the most common and also the most aggressive form of exocrine pancreatic cancer causing ~90% of cases (Adamska et al., Int J Mol Sci. 2017 Jul; 18(7): 1338). Pan02 is a well-established mouse model for Grade III ductal adenocarcinoma of the pancreas. In this model, pancreatic cancer was induced chemically in male C57BL/6 mice using 3-MCA (3-methylcholanthrene) (Corbett, et al., Cancer Res. 1984, 44:717-726). This example shows the potential of the present invention for enriching tumor reactive TILs from Pan02 pancreatic tumors.

The experimental workflow for this experiment was similar to that which is shown in FIG. 9A, except Pan02 cells were utilized in lieu of the E0771 tumor cells shown in the figure. Briefly, [1 × 10⁶] Pan02 tumor cells were inoculated (s.c.) in C57BL/6 mice using the same method that is described in Example 5 above and then tumor tissue was isolated approximately 21 days after inoculation. Tumor lysate and single cell suspensions were prepared using the same method that is described in Example 5 above. iDCs prepared from CD45.1+/+ C57BL/6 mice were divided into two groups to be treated with healthy pancreatic (healthy Pan) lysates and Pan02 tumor lysates, respectively, overnight. Then the two groups of iDCs were functionalized with FT as in FIG. 5B to form iDC-FT.

In parallel, TILs from Pan02 tumors were isolated by mechanically dissociating tumor tissue prior to centrifugation on a discontinuous Percoll gradient (GE Healthcare). The resulting TILs were washed with PBS and incubated with the 2 groups of iDC-FT, respectively, for 2 hours in the case of the CD8+ TILs and for 4 hours in the case of the CD4+ TILs (TIL: DC = 10:1). Then GDP-Fuc-biotin (50 µM) was added and incubated for another 30 min. After quenching the reaction with LacNAc (5 mM), the cell mixture was stained with APC-streptavidin and cell identity markers (CD8/PD-1 for CD8 T cells and CD3/CD4 for CD4 T cells) and analysed using flow cytometry. FIGS. 21A and 21B show the FACS results demonstrating that FT-functionalized iDCs primed with Pan02 tumor lysate specifically labelled 8.39% and 12.2%% of CD8+ and CD4+ TILs, respectively (far right panel, upper right quarter in FIG. 21A and right panel, right box in FIG. 21B). In contrast, FT-functionalized iDCs treated with healthy pancreas lysate (second panel from the right in FIG. 21A and left panel in FIG. 21B showed minimal biotinylation to CD8+ and CD4+ TILs.

To determine whether the CD8+ biotin-labelled TILs were indeed tumor specific cytotoxic T cells, Biotin+ (red) and biotin - (blue) CD8 + T cells were sorted by FACS, cultured with IL-2 in T cell medium (IL2: 100 IU/mL) for 12hours, then B16 melanoma cells (stably transduced with firefly luciferase) were co-cultured with sorted CD8+ T cells for 20 hours as a ratio of 1:4. Then the killing of B16 tumor were quantified through the luciferase activity (Bright-Glo, Promega). as described in the previous examples. Briefly, CD8+ T cells were co-cultured with Pan02 tumor cells at effector-to-target ratios of 10:1, 20:1, and 40:1 for 20 hours followed by LDH assay to evaluate cell viability. The comparison of tumor killing abilities of Biotin⁻ and Biotin⁺ CD8+ T cells are showed in the bar chart of FIG. 21A. Biotin+ cells exhibited significant higher killing activities than those that were biotin- (FIG. 21A), and there was a clear dose dependent effect, with the greatest level of killing occurring at the highest tested effector to target ratio (40:1). Like the data presented in the prior examples, these data strongly suggest that TSA-reactive T cells are enriched in the biotin+ population. Data was obtained from three independent experiments. In all figures, ns P > 0.05; ** P < 0.01; *** P < 0.001; **** P < 0.0001; one-way ANOVA followed by Tukey's multiple comparisons test.

### REFERENCES

(1) Roozendaal, R.; Mebius, R. E. Annual review of immunology 2011, 29, 23-43.
(2) Kim, H.; Cruz, M.; Bourdeau, A.; Dumont, D. J. PloS one 2013, 8, e52197.
(3) Chao, D. L.; Ma, L.; Shen, K. Nature reviews. Neuroscience 2009, 10, 262-71.
(4) Cahalan, M. D.; Parker, I. Annual review of immunology 2008, 26, 585-626.
(5) Pasqual, G.; Chudnovskiy, A.; Tas, J. M. J.; Agudelo, M.; Schweitzer, L. D.; Cui, A.; Hacohen, N.; Victora, G. D. Nature 2018, 553, 496.
(6) Liu, D. S.; Loh, K. H.; Lam, S. S.; White, K. A.; Ting, A. Y. PloS one 2013, 8, e52823.
(7) Miller, J. F.; Sadelain, M. Cancer Cell 2015, 27, 439-49.
(8) Cornetta, K.; Pollok, K. E.; Miller, A. D. Cold Spring Harbor Protocols 2008, 2008, pdb.prot4884.
(9) Dupré, L.; Trifari, S.; Follenzi, A.; Marangoni, F.; Lain de Lera, T.; Bernad, A.; Martino, S.; Tsuchiya, S.; Bordignon, C.; Naldini, L.; Aiuti, A.; Roncarolo, M.-G. Mol. Ther. 2004, 10, 903-915.
(10) Shearer, R. F.; Saunders, D. N. Genes Cells 2015, 20, 1-10.
(11) Stephan, M. T.; Irvine, D. J. Nano Today 2011, 6, 309-325.
(12) Swartz, M. A.; Hirosue, S.; Hubbell, J. A. Sci. Transl. Med. 2012, 4, 148rv9-148rv9.
(13) Parmar, S.; Liu, X.; Najjar, A.; Shah, N.; Yang, H.; Yvon, E.; Rezvani, K.; McNiece, I.; Zweidler-McKay, P.; Miller, L.; Wolpe, S.; Blazar, B. R.; Shpall, E. J. Blood 2015, 125, 1502-1506.
(14) Sackstein, R.; Merzaban, J. S.; Cain, D. W.; Dagia, N. M.; Spencer, J. A.; Lin, C. P.; Wohlgemuth, R. Nat. Med. 2008, 14, 181.
(15) Li, J.; Chen, M.; Liu, Z.; Zhang, L.; Felding, B. H.; Moremen, K. W.; Lauvau, G.; Abadier, M.; Ley, K.; Wu, P. ACS Central Science 2018, 4, 1633-1641.
(16) Popp, M. W.; Antos, J. M.; Grotenbreg, G. M.; Spooner, E.; Ploegh, H. L. Nature Chemical Biology 2007, 3, 707.
(17) Sharma, P.; Allison, J. P. Science 2015, 348, 56-61.
(18) Rosenberg, S. A.; Restifo, N. P. Science 2015, 348, 62-68.
(19) Guedan, S.; Ruella, M.; June, C. H. Annual review of immunology 2018.
(20) Riaz, N.; Morris, L.; Havel, J. J.; Makarov, V.; Desrichard, A.; Chan, T. A. International immunology 2016, 28, 411-9.
(21) Le, D. T.; Uram, J. N.; Wang, H.; Bartlett, B. R.; Kemberling, H.; Eyring, A. D.; Skora, A. D.; Luber, B. S.; Azad, N. S.; Laheru, D.; Biedrzycki, B.; Donehower, R. C.; Zaheer, A.; Fisher, G. A.; Crocenzi, T. S.; Lee, J. J.; Duffy, S. M.; Goldberg, R. M.; de la Chapelle, A.; Koshiji, M.; Bhaijee, F.; Huebner, T.; Hruban, R. H.; Wood, L. D.; Cuka, N.; Pardoll, D. M.; Papadopoulos, N.; Kinzler, K. W.; Zhou, S.; Cornish, T. C.; Taube, J. M.; Anders, R. A.; Eshleman, J. R.; Vogelstein, B.; Diaz, L. A., Jr. The New England journal of medicine 2015, 372, 2509-20.
(22) Rizvi, N. A.; Hellmann, M. D.; Snyder, A.; Kvistborg, P.; Makarov, V.; Havel, J. J.; Lee, W.; Yuan, J.; Wong, P.; Ho, T. S.; Miller, M. L.; Rekhtman, N.; Moreira, A. L.; Ibrahim, F.; Bruggeman, C.; Gasmi, B.; Zappasodi, R.; Maeda, Y.; Sander, C.; Garon, E. B.; Merghoub, T.; Wolchok, J. D.; Schumacher, T. N.; Chan, T. A. Science 2015, 348, 124-8.
(23) Snyder, A.; Makarov, V.; Merghoub, T.; Yuan, J.; Zaretsky, J. M.; Desrichard, A.; Walsh, L. A.; Postow, M. A.; Wong, P.; Ho, T. S.; Hollmann, T. J.; Bruggeman, C.; Kannan, K.; Li, Y.; Elipenahli, C.; Liu, C.; Harbison, C. T.; Wang, L.; Ribas, A.; Wolchok, J. D.; Chan, T. A. The New England journal of medicine 2014, 371, 2189-2199.
(24) McGranahan, N.; Furness, A. J.; Rosenthal, R.; Ramskov, S.; Lyngaa, R.; Saini, S. K.; Jamal-Hanjani, M.; Wilson, G. A.; Birkbak, N. J.; Hiley, C. T.; Watkins, T. B.; Shafi, S.; Murugaesu, N.; Mitter, R.; Akarca, A. U.; Linares, J.; Marafioti, T.; Henry, J. Y.; Van Allen, E. M.; Miao, D.; Schilling, B.; Schadendorf, D.; Garraway, L. A.; Makarov, V.; Rizvi, N. A.; Snyder, A.; Hellmann, M. D.; Merghoub, T.; Wolchok, J. D.; Shukla, S. A.; Wu, C. J.; Peggs, K. S.; Chan, T. A.; Hadrup, S. R.; Quezada, S. A.; Swanton, C. Science 2016, 351, 1463-9.
(25) Yarchoan, M.; Johnson, B. A., 3rd; Lutz, E. R.; Laheru, D. A.; Jaffee, E. M. Nature reviews. Cancer 2017, 17, 209-222.
(26) Tran, E.; Ahmadzadeh, M.; Lu, Y. C.; Gros, A.; Turcotte, S.; Robbins, P. F.; Gartner, J. J.; Zheng, Z.; Li, Y. F.; Ray, S.; Wunderlich, J. R.; Somerville, R. P.; Rosenberg, S. A. Science 2015, 350, 1387-90.
(27) Klebanoff, C. A.; Rosenberg, S. A.; Restifo, N. P. Nat Med 2016, 22, 26-36.
(28) Schumacher, T. N.; Schreiber, R. D. Science 2015, 348, 69-74.
(29) Linnemann, C.; van Buuren, M. M.; Bies, L.; Verdegaal, E. M. E.; Schotte, R.; Calis, J. J. A.; Behjati, S.; Velds, A.; Hilkmann, H.; Atmioui, D. e.; Visser, M.; Stratton, M. R.; Haanen, J. B. A. G.; Spits, H.; van der Burg, S. H.; Schumacher, T. N. M. Nature Medicine 2014, 21, 81.
(30) Simoni, Y.; Becht, E.; Fehlings, M.; Loh, C. Y.; Koo, S.-L.; Teng, K. W. W.; Yeong, J. P. S.; Nahar, R.; Zhang, T.; Kared, H.; Duan, K.; Ang, N.; Poidinger, M.; Lee, Y. Y.; Larbi, A.; Khng, A. J.; Tan, E.; Fu, C.; Mathew, R.; Teo, M.; Lim, W. T.; Toh, C. K.; Ong, B.-H.; Koh, T.; Hillmer, A. M.; Takano, A.; Lim, T. K. H.; Tan, E. H.; Zhai, W.; Tan, D. S. W.; Tan, I. B.; Newell, E. W., Bystander CD8+ T cells are abundant and phenotypically distinct in human tumour infiltrates. Nature 2018, 557 (7706), 575-579.
(31) Stevanović, S.; Pasetto, A.; Helman, S. R.; Gartner, J. J.; Prickett, T. D.; Howie, B.; Robins, H. S.; Robbins, P. F.; Klebanoff, C. A.; Rosenberg, S. A.; Hinrichs, C. S., Landscape of immunogenic tumor antigens in successful immunotherapy of virally induced epithelial cancer. Science 2017, 356 (6334), 200.
(32) Gros, A.; Robbins, P. F.; Yao, X.; Li, Y. F.; Turcotte, S.; Tran, E.; Wunderlich, J. R.; Mixon, A.; Farid, S.; Dudley, M. E.; Hanada, K.; Almeida, J. R.; Darko, S.; Douek, D. C.; Yang, J. C.; Rosenberg, S. A., PD-1 identifies the patient-specific CD8(+) tumor-reactive repertoire infiltrating human tumors. The Journal of clinical investigation 2014, 124 (5), 2246-59.
(33) Yarchoan, M.; Johnson, B. A., III; Lutz, E. R.; Laheru, D. A.; Jaffee, E. M., Targeting neoantigens to augment antitumour immunity. Nat. Rev. Cancer 2017, 17 (9), 569.
(34) Stronen, E.; Toebes, M.; Kelderman, S.; van Buuren, M. M.; Yang, W.; van Rooij, N.; Donia, M.; Boeschen, M.-L.; Lund-Johansen, F.; Olweus, J.; Schumacher, T. N., Targeting of cancer neoantigens with donor-derived T cell receptor repertoires. Science 2016, 352 (6291), 1337-1341.
(35) Ott, P. A.; Hu, Z.; Keskin, D. B.; Shukla, S. A.; Sun, J.; Bozym, D. J.; Zhang, W.; Luoma, A.; Giobbie-Hurder, A.; Peter, L.; Chen, C.; Olive, O.; Carter, T. A.; Li, S.; Lieb, D. J.; Eisenhaure, T.; Gjini, E.; Stevens, J.; Lane, W. J.; Javeri, I.; Nellaiappan, K.; Salazar, A. M.; Daley, H.; Seaman, M.; Buchbinder, E. I.; Yoon, C. H.; Harden, M.; Lennon, N.; Gabriel, S.; Rodig, S. J.; Barouch, D. H.; Aster, J. C.; Getz, G.; Wucherpfennig, K.; Neuberg, D.; Ritz, J.; Lander, E. S.; Fritsch, E. F.; Hacohen, N.; Wu, C. J., An immunogenic personal neoantigen vaccine for patients with melanoma. Nature 2017, 547 (7662), 217-221.
(36) Li, G.; Bethune, M. T.; Wong, S.; Joglekar, A. V.; Leonard, M. T.; Wang, J. K.; Kim, J. T.; Cheng, D.; Peng, S.; Zaretsky, J. M.; Su, Y.; Luo, Y.; Heath, J. R.; Ribas, A.; Witte, O. N.; Baltimore, D. T cell antigen discovery via trogocytosis. Nature methods 2019, 16, 183-190.
(37) Gary, R.; Voelkl, S.; Palmisano, R.; Ullrich, E.; Bosch, J. J.; Mackensen, A. Antigen-specific transfer of functional programmed death ligand 1 from human APCs onto CD8+ T cells via trogocytosis. Journal of immunology 2012, 188, 744-752.

## Claims

1. A method for identifying antigen-reactive T cells present in a population of tissue infiltrating lymphocytes (TiILs) or circulating T cells, the method comprising:
a. providing a dendritic cell;
b. incubating the dendritic cell with one or more antigens or a source of one or more antigens in order to prime the dendritic cell with one or more of the antigens;
c. conjugating the dendritic cell on its cell surface with a suitable enzyme for catalyzing an interaction-dependent labeling reaction on a prey cell; wherein the conjugating occurs before or after incubating step (b);
d. contacting the dendritic cell after conjugating step (C) with a population of TiILs or circulating T cells, wherein the population comprises at least one antigen-reactive T cell and wherein the contacting occurs in the presence of a substrate for the enzyme, said substrate comprising a detectable tag;
e. detecting any cells comprising the tag; thereby identifying the antigen-reactive T cells;
wherein the enzyme is a glycosyltransferase and the substrate is a labeled donor sugar nucleotide.

2. The method of claim 1, comprising enriching for the cells comprising the tag.

3. The method of claim 2, comprising isolating single cells comprising the tag.

4. The method of claim 2 or 3, comprising expanding cells comprising the tag.

5. The method of claim 1, wherein the glycosyltransferase is a fucosyltransferase and the substrate is tagged GDP-fucose.

6. The method of claim 5, wherein the fucosyltransferase is *H. pylori* α1,3fucosyltransferase.

7. The method of claim 1, wherein the glycotransferase is a sialyltransferase and the substrate is tagged CMP-Neu5Ac.

8. The method of any one of claims 1-7, further comprising determining whether the cells comprising the tag further comprise other markers indicative of TSA reactivity.

9. The method of any one of claims 1-8, further comprising enriching for cells comprising the other markers, if present, that are indicative of TSA reactivity via FACS.

10. The method of claim 8 or 9, wherein the other markers include one or more of PD-1 expression; CD134 expression, CD137 expression, CXCR5 expression, and TIM3 expression.

11. The method of any one of claims 8-10, wherein the method comprises enriching for cells comprising both the tag and PD-1 expression.

12. The method of any one of claims 1-11, wherein the method further comprises enriching for cells comprising CD8+ and/or CD4+ expression.

13. The method of any one of claims 1-12, wherein the tag is selected from biotin, a fluorescent molecule, a dye, a FAM dye, a cyanine dye, Cy2, Cy3, Cy3B, Cy3.5, Cy5, Cy5.5, Cy7, an Alexa Fluor dye, and a Janelia Fluor dye.

## Patentansprüche

1. Verfahren zum Identifizieren Antigen-reaktiver T-Zellen, die in einer Population von Gewebe infiltrierenden Lymphozyten (TiILs) oder zirkulierenden T-Zellen vorhanden sind, wobei das Verfahren umfasst:
a. Bereitstellen einer dendritischen Zelle;
b. Inkubieren der dendritischen Zelle mit einem oder mehreren Antigenen oder einer Quelle eines oder mehrerer Antigene, um die dendritische Zelle mit einem oder mehreren der Antigene zu immunisieren;
c. Konjugieren der dendritischen Zelle auf ihrer Zelloberfläche mit einem geeigneten Enzym zum Katalysieren einer interaktionsabhängigen Markierungsreaktion auf einer Beutezelle; wobei das Konjugieren vor oder nach Inkubationsschritt (b) erfolgt;
d. Kontaktieren der dendritischen Zelle nach Konjugationsschritt (C) mit einer Population von TiILs oder zirkulierenden T-Zellen, wobei die Population mindestens eine Antigen-reaktive T-Zelle umfasst und wobei das Kontaktieren in Gegenwart eines Substrats für das Enzym erfolgt, wobei das Substrat ein nachweisbares Tag umfasst;
e. Nachweisen aller Zellen, die das Tag umfassen; dadurch Identifizieren der Antigenreaktiven T-Zellen;
wobei das Enzym eine Glycosyltransferase und das Substrat ein markiertes Spender-Zuckernukleotid ist.

2. Verfahren nach Anspruch 1, das das Anreichern der das Tag umfassenden Zellen umfasst.

3. Verfahren nach Anspruch 2, das das Isolieren einzelner Zellen umfasst, die das Tag umfassen.

4. Verfahren nach Anspruch 2 oder 3, das das Erweitern von Zellen umfasst, die das Tag umfassen.

5. Verfahren nach Anspruch 1, wobei die Glycosyltransferase eine Fucosyltransferase ist und das Substrat getaggte GDP-Fucose ist.

6. Verfahren nach Anspruch 5, wobei die Fucosyltransferase *H. pylori* α1,3-Fucosyltransferase ist.

7. Verfahren nach Anspruch 1, wobei die Glycotransferase eine Sialyltransferase ist und das Substrat getaggtes CMP-Neu5Ac ist.

8. Verfahren nach einem der Ansprüche 1 bis 7, das ferner das Bestimmen umfasst, ob die Zellen, die das Tag umfassen, ferner andere Marker umfassen, die auf TSA-Reaktivität hinweisen.

9. Verfahren nach einem der Ansprüche 1 bis 8, das ferner das Anreichern von Zellen umfasst, die die anderen Marker, sofern vorhanden, umfassen, die über FACS auf eine TSA-Reaktivität hinweisen.

10. Verfahren nach Anspruch 8 oder 9, wobei die anderen Marker einen oder mehrere von PD-1-Expression; CD134-Expression, CD137-Expression, CXCR5-Expression und TIM3-Expression beinhalten.

11. Verfahren nach einem der Ansprüche 8 bis 10, wobei das Verfahren das Anreichern von Zellen umfasst, die sowohl das Tag als auch die PD-1-Expression umfassen.

12. Verfahren nach einem der Ansprüche 1 bis 11, wobei das Verfahren ferner das Anreichern von Zellen umfasst, die CD8+- und/oder CD4+-Expression umfassen.

13. Verfahren nach einem der Ansprüche 1 bis 12, wobei das Tag ausgewählt ist aus Biotin, einem fluoreszierenden Molekül, einem Farbstoff, einem FAM-Farbstoff, einem Cyaninfarbstoff, Cy2, Cy3, Cy3B, Cy3.5, Cy5, Cy5.5, Cy7, einem Alexa-Fluor-Farbstoff und einem Janelia-Fluor-Farbstoff.

## Revendications

1. Procédé d'identification de lymphocytes T réactifs à un antigène présents dans une population de lymphocytes infiltrant les tissus (TiIL) ou de lymphocytes T circulants, le procédé comprenant :
a. la fourniture d'une cellule dendritique ;
b. l'incubation de la cellule dendritique avec un ou plusieurs antigènes ou une source d'un ou plusieurs antigènes afin d'amorcer la cellule dendritique avec un ou plusieurs des antigènes ;
c. la conjugaison de la cellule dendritique sur sa surface cellulaire avec une enzyme appropriée pour catalyser une réaction de marquage dépendante de l'interaction sur une cellule proie ; ladite conjugaison se produisant avant ou après l'étape d'incubation (b) ;
d. la mise en contact de la cellule dendritique après l'étape de conjugaison (c) avec une population de TiIL ou de lymphocytes T circulants, ladite population comprenant au moins un lymphocyte T réactif à un antigène et ladite mise en contact se produisant en présence d'un substrat pour l'enzyme, ledit substrat comprenant une étiquette détectable ;
e. la détection de toutes les cellules comprenant l'étiquette ; identifiant ainsi les lymphocytes T réactifs à un antigène ;
dans lequel l'enzyme est une glycosyltransférase et le substrat est un nucléotide de sucre donneur marqué.

2. Procédé de la revendication 1, comprenant l'enrichissement des cellules comprenant l'étiquette.

3. Procédé de la revendication 2, comprenant l'isolement de cellules individuelles comprenant l'étiquette.

4. Procédé de l'une des revendications 2 ou 3, comprenant l'expansion de cellules comprenant l'étiquette.

5. Procédé de la revendication 1, dans lequel la glycosyltransférase est une fucosyltransférase et le substrat est du GDP-fucose étiqueté.

6. Procédé de la revendication 5, dans lequel la fucosyltransférase est la *H. pylori* α1,3fucosyltransférase.

7. Procédé de la revendication 1, dans lequel la glycotransférase est une sialyltransférase et le substrat est du CMP-Neu5Ac étiqueté.

8. Procédé de l'une quelconque des revendications 1 à 7, comprenant en outre le fait de déterminer si les cellules comprenant l'étiquette comprennent en outre d'autres marqueurs indiquant une réactivité TSA.

9. Procédé de l'une quelconque des revendications 1 à 8, comprenant en outre l'enrichissement de cellules comprenant les autres marqueurs, s'ils sont présents, qui indiquent la réactivité TSA par FACS.

10. Procédé de l'une des revendications 8 ou 9, dans lequel les autres marqueurs comprennent une ou plusieurs parmi l'expression de PD-1 ; l'expression de CD134, l'expression de CD137, l'expression de CXCR5 et l'expression de TIM3.

11. Procédé de l'une quelconque des revendications 8 à 10, dans lequel le procédé comprend l'enrichissement de cellules comprenant à la fois l'étiquette et l'expression de PD-1.

12. Procédé de l'une quelconque des revendications 1 à 11, dans lequel le procédé comprend en outre l'enrichissement de cellules comprenant l'expression de CD8+ et/ou CD4+.

13. Procédé de l'une quelconque des revendications 1 à 12, dans lequel l'étiquette est choisie parmi la biotine, une molécule fluorescente, un colorant, un colorant FAM, un colorant cyanine, Cy2, Cy3, Cy3B, Cy3.5, Cy5, Cy5.5, Cy7, un colorant Alexa Fluor et un colorant Janelia Fluor.
